(19)

**Europäisches Patentamt**
**European Patent Office**
**Office européen des brevets**

(11) **EP 4 295 765 A1**

(12) **EUROPEAN PATENT APPLICATION**

(43) Date of publication:
**27.12.2023 Bulletin 2023/52**

(51) International Patent Classification (IPC):
**A61B 5/113** (2006.01) **A61B 5/16** (2006.01)

(21) Application number: **22305903.1**

(22) Date of filing: **22.06.2022**

(52) Cooperative Patent Classification (CPC):
**A61B 3/113; A61B 5/163; A61B 5/167**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Orasis Eye Analytics and Rehabilitation**
**75015 PARIS (FR)**

(72) Inventor: **KAPOULA, Zoi**
**75015 Paris (FR)**

(74) Representative: **Regimbeau**
**20, rue de Chazelles**
**75847 Paris Cedex 17 (FR)**

(54) **METHOD TO APPRECIATE CREATIVITY VIA MEASURE AND ANALYSIS OF BINOCULAR EYE MOVEMENTS WHILE EXPLORING PAINTINGS**

(57) The present invention relates to a method for measuring binocular motricity parameters in an individual based on the measure of binocular motricity parameters during free exploration of one or several pictorial artwork(s). It further relates to a method for detecting features of a learning disorder (in particular dyslexia) or an autistic spectrum disorder (in particular autism) in an individual based on binocular motricity parameters calculated from previously obtained recordings of eyes movements during free exploration of one or several pictorial artwork(s). It also refers to a method for assessing the level of creativity of an individual based on binocular motricity parameters calculated from previously obtained recordings of eyes movements during free exploration of one or several pictorial artwork(s). Those methods are applicable to any individual, very rapid and playful.

Figure 10

EP 4 295 765 A1

**Description**

**TECHNICAL FIELD OF THE INVENTION**

The present invention is in the field of tests useful to assess creativity and presence or absence of learning disorders and autism spectrum disorders (ASD). It relates to a method for measuring binocular motricity parameters in an individual based on the measure of binocular motricity parameters during free exploration of one or several pictorial artwork(s). It further relates to a method for detecting features of a learning disorder (in particular dyslexia) or an autism spectrum disorder (in particular autism) in an individual based on binocular motricity parameters calculated from previously obtained recordings of eyes movements during free exploration of one or several pictorial artwork(s). It also refers to a method for assessing the level of creativity of an individual based on binocular motricity parameters calculated from previously obtained recordings of eyes movements during free exploration of one or several pictorial artwork(s).

**BACKGROUND ART**

Creativity can be hard to define. Indeed, though it seems almost counter-intuitive to try to quantify or measure creativity, there have been many different attempts at studying and evaluating an individual's creativity. One such method is the Torrance Test of Creative Thinking (TTCT), which was first developed in 1966 and has been used widely across the world, having been re-developed four times (1974, 1984, 1990, and 1998) and translated into 35 different languages (Torrance, E. P. Torrance tests of creative thinking. Norms-technical manual. Research edition. Verbal tests, forms A and B. Figural tests, forms A and B; Personnel Press, 1966). The test has two components, verbal and figural. The figural test measures four key domains of creativity: fluidity, flexibility, originality, and elaboration. Fluidity is a measurement of the ability to produce a high number of relevant ideas as measured by the number of figures the participant is able to produce. Flexibility is a measurement of the ability to produce a wide range of variable ideas. Originality is a measurement of the number of statistically non-frequent ideas, or unique ideas. Elaboration is a measurement of the development and amount of detail that is produced around one idea (Kapoula, Z.; Ruiz, S.; Spector, L.; Mocorovi, M.; Gaertner, C.; Quilici, C.; Vernet, M. Education Influences Creativity in Dyslexic and Non-Dyslexic Children and Teenagers. PLoS One 2016, 11 (3), e0150421. DOI: 10.1371/journal.pone.0150421).

However, such tests are relatively long to perform and not easy to interpret; they require training and the quotation of a test can take up to one hour. Conceptually creativity is limited to 4 dimensions which is also somehow reductionist

There is thus a need for new tests able to measure creativity of an individual, that would be more rapid and easier to interpret. Most important, test that more physiologic, and beyond the voluntary conscious control of the person.

There has been some data that show that dyslexics are more creative than their peers, largely in the figural categories, though it does not appear that dyslexics demonstrate higher creativity consistently in any particular domain (Everatt, J.; Steffert, B.; Smythe, I. An eye for the unusual: Creative thinking in dyslexics. Dyslexia 1999, 5 (1), 28-46; Rack, L. Developmental dyslexia and literary creativity: creativity in the area of deficit. J Learn Disabil 1981, 14 (5), 262-263. DOI: 10.1177/002221948101400506). For example, one study reported that dyslexics were more able to connect original thoughts to produce different types of conclusions (Cancer, A.; Manzoli, S.; Antonietti, A. The alleged link between creativity and dyslexia: Identifying the specific process in which dyslexic students excel. Cogent Psychology 2016, 3 (1), 1190309. DOI: 10.1080/23311908.2016.1190309). One study reported that students with dyslexia were more creative in the originality category than their peers (Tafti, M. A.; Hameedy, M. A.; Baghal, N. M. Dyslexia, a deficit or a difference: comparing the creativity and memory skills of dyslexic and nondyslexic students in Iran. Social Behavior and Personality: An International Journal 2009, 37, 1009+, Report. (acccessed 2022/5/4/). From Gale Gale OneFile: Health and Medicine). Our own lab has discovered that children and teenagers with dyslexia demonstrate higher creativity scores as compared to their peers, which was amplified in those who were enrolled in schools that place emphasis on visual arts (Kapoula, Z.; Ruiz, S.; Spector, L.; Mocorovi, M.; Gaertner, C.; Quilici, C.; Vernet, M. Education Influences Creativity in Dyslexic and Non-Dyslexic Children and Teenagers. PLoS One 2016, 11 (3), e0150421. DOI: 10.1371/journal.pone.0150421). Other studies have confirmed that dyslexics are more prevalent in artistic schools, perhaps demonstrating an increased aptitude for producing creative visual interpretations of the world around them (Wolff, U.; Lundberg, I. The prevalence of dyslexia among art students. Dyslexia 2002, 8 (1), 34-42. DOI: 10.1002/dys.211).

Parallelly, prior research analyzing eye movements during REMOBI® testing of saccades and vergence revealed abnormalities of eye movements in dyslexic. The major abnormalities were abnormal velocity profiles for both saccades and vergence namely slower deceleration. Second increased discoordination of the eyes during the saccades called disconjugacy that are believed to be due to lack of fast vergence correcting mechanism (Ward, L. M.; Kapoula, Z. Differential diagnosis of vergence and saccade disorders in dyslexia. Sci Rep 2020, 10 (1), 22116. DOI: 10.1038/s41598-020-79089-1; WO2022/074130A1).

Studies on reading itself indicated same problems for saccades (abnormal velocity profiles and discoordination).

Moreover, this discoordination is increased when the text to read does not have any sense (Ward, L. M.; Kapoula, Z. Dyslexics' Fragile Oculomotor Control Is Further Destabilized by Increased Text Difficulty. Brain Sci 2021, 11 (8). DOI: 10.3390/brainsci11080990; WO2022/074130A1).

Machine learning application using these parameters all together of eye movement abnormality succeeds to predict dyslexia and reading speed at 83% i.e. similar rates as the phonologic tests (El Hmimdi AE, Ward LM, Palpanas T, Kapoula Z. Predicting Dyslexia and Reading Speed in Adolescents from Eye Movements in Reading and Non-Reading Tasks: A Machine Learning Approach).

However, while such tests are useful to diagnose dyslexia or to detect intrinsic ocular motor abnormalities in dyslexics, they are not particularly playful (in particular reading tests) and they focus only on dyslexics' disabilities. There is thus also a need for improved tests for detecting features of dyslexia, that would be more playful and not only focus on dyslexics's abnormalities but also highlight their increased creativity, thus improving the self-esteem of dyslexics.

In addition, no correlation between dyslexic increased creativity and particular abnormal eyes movements has been described in the prior art.

## SUMMARY OF THE INVENTION

In the context of the present invention, the inventors surprisingly found that creativity of an individual may be measured rapidly and easily by recording and analyzing eyes' movements during free exploration by the individual of one or several pictorial artwork(s). Indeed, some binocular motricity parameters were found to be significantly different during pictorial artwork free exploration between dyslexic (known to be more creative) and normal (less creative) individuals. These binocular motricity parameters may thus be used for measuring creativity in any individual, but also as a surrogate marker for detecting features of pathologies known to be associated to increased creativity, such as learning disorders (in particular dyslexia, see background section above) and also autism spectrum disorders. Indeed, some studies show higher originality and elaboration scores in the autistic individuals (Pennisi P, Giallongo L, Milintenda G, Cannarozzo M. Autism, autistic traits and creativity: a systematic review and meta-analysis. Cogn Process. 2021 Feb;22(1):1-36). In addition, there is evidence for artistic talents in autism and theories about a genetic basis although the role of epigenetics is also emphasized (Sotiropoulos MG, Anagnostouli M. Genes, brain dynamics and art: the genetic underpinnings of creativity in dancing, musicality and visual arts. J Integr Neurosci. 2021 Dec 30;20(4):1095-1104. doi: 10.31083/j.jin2004110. PMID: 34997732). Finally, a recent study also highlights a unique creative cognitive profile among children with autistic syndrome (Kasirer A, Adi-Japha E, Mashal N. Verbal and Figural Creativity in Children With Autism Spectrum Disorder and Typical Development. Front Psychol. 2020 Oct 27;11:559238. doi: 10.3389/fpsyg.2020.559238. PMID: 33192819; PMCID: PMC7652732). Moreover, problems of binocular motricity in persons with autism spectrum disorders are likely to be more frequent than in healthy, as many epidemiologic studies suggest vergence-accommodation disorders in persons with autism spectrum disorders (Coulter RA, Bade A, Jenewein EC, Tea YC, Mitchell GL. Near-point Findings in Children with Autism Spectrum Disorder and in Typical Peers. Optom Vis Sci. 2021 Apr 1;98(4):384-393. doi: 10.1097/OPX.0000000000001679. PMID: 33852554; PMCID: PMC8051934; Svend Erik Mouridsen, Bente Rich & Torben Isager (2017) Eye Disorders among Adult People Diagnosed with Infantile Autism in Childhood: A Longitudinal Case Control Study, Ophthalmic Epidemiology, 24:5, 332-335; Jolie R. Keemink et al. Eye Movements and Behavioural Responses to Gaze-Contingent Expressive Faces in Typically Developing Infants and Infant Siblings. Autism Res 2021, 14: 973-983).

Methods based on the measure of binocular motricity parameters during free exploration by the individual of one or several pictorial artwork(s) are advantageous as:

- When used for detecting features of a learning disorder in an individual, they are more playful than tests based on reading (in particular for dyslexic individuals) or even using minimalistic ocular motor tests such as with the binocular motor stimulation device configured to specifically and physically stimulate vergences and saccades, comprising visual or audiovisual targets placed in real space at eccentricities and depths of a surface (such as REMOBI® or REMOBI-Neurocog device). In addition, the pictorial method does not focus only on disabilities of individuals suffering from a learning disorder (in particular dyslexia) but also highlights their increased creativity.
- When used for assessing the level of creativity of an individual, the pictorial eye movement exploration methods are rapid (only a short - at most 1 minute- free exploration of one or a few - at most 10- pictorial artwork(s) are necessary) and easy to implement and the analysis is automatic, contrary to conventional creativity tests.

In a first aspect, the present invention thus relates to a method for measuring binocular motricity parameters in an individual, comprising:

a1) inviting the individual to free exploration of one or several pictorial artwork(s);
b1) recording the eye movements of the left eye and the right eye of the individual during the free exploration of the one or more pictorial artwork(s); and

c1) calculating, using a digital processing device, the value of one or more binocular motricity parameter(s) from the records obtained in step b1);

wherein when several pictorial artworks are explored, they are explored one by one, and

wherein each of the one or more pictorial artwork(s) is selected from pictorial artworks creating an illusion of visual motion in depth and pictorial artworks with spatial or sense ambiguity.

In a second aspect, the present invention thus relates to a method for detecting features of a learning disorder (in particular dyslexia) or an autistic spectrum disorder (in particular autism) in an individual, comprising:

a2) providing previously obtained recordings of the eye movements of the left eye and the right eye of the individual during free exploration of one or several pictorial artwork(s);

b2) calculating, using a digital processing device, the value(s) of at least one binocular motricity parameter from the records provided in step a2); and

c2) comparing the value(s) calculated in step b2) to one or more reference value(s) obtained in one or more reference individual(s); and

d2) concluding to the presence or absence of features of a learning disorder (in particular dyslexia) or an autistic spectrum disorder (in particular autism) in the individual based on said comparison.

In a third aspect, the present invention thus relates to a method for assessing the level of creativity of an individual, comprising:

a3) providing previously obtained recordings of the eye movements of the left eye and the right eye of the individual during free exploration of one or several pictorial artwork(s);

b3) calculating, using a digital processing device, the value(s) of at least one binocular motricity parameter from the records provided in step a3);

c3) comparing the value(s) calculated in step b3) to one or more reference value(s) obtained in one or more reference individual(s); and

d3) concluding to the level of creativity of the individual based on said comparison.

## DESCRIPTION OF THE FIGURES

**Figure 1A.** Painting 1: Bridget Riley, Blaze 1, 1962. The original pictorial art is in shades of grey and measures 53 $\times$ 52.1 cm.

**Figure 1B.** Painting 2: Bridget Riley, Movements in square,1961. The original pictorial art is in black and white and measures 122 $\times$ 122 cm.

**Figure 1C.** Presentation in shades of grey of Painting 3: Bridget Riley, Cataract 3, 1967. The original pictorial art is in color (mainly alternating waves of grey, red and white) and measures 221.9 $\times$ 222.9 cm.

**Figure 1D.** Painting 4: M. C. Escher, Relativité, 1953. The original pictorial art is in shades of grey and measures 27.9 $\times$ 28.9 cm.

**Figure 1E.** Presentation in shades of grey of Painting 7: René Magritte, La trahison des images, 1973. The original pictorial art is in shades of grey and measures 60 $\times$ 81 cm.

**Figure 1F.** Presentation in shades of grey of Painting 5: René Magritte, La condition humaine I, 1935. The original pictorial art is in shades of grey and measures 100 $\times$ 81 cm.

**Figure 1G.** Presentation in shades of grey of Painting 6: René Magritte, The Art of Conversation-Color Variation, 1950. The original pictorial art is in shades of grey and measures 50 $\times$ 65 cm.

**Figure 2.** Example of one participant's eye tracing over the 30 second viewing period.

**Figure 3A.** Postural parameters in the dyslexic and non-dyslexic population while viewing Painting 1: Bridget Riley, Blaze 1, 1962.

**Figure 3B.** Postural parameters in the dyslexic and non-dyslexic population while viewing Painting 2: Bridget Riley, Movement in Squares, 1961.

**Figure 3C.** Postural parameters in the dyslexic and non-dyslexic population while viewing Painting 3: Bridget Riley, Cataract 3, 1967.

**Figure 4.** Subjective feelings of appreciation and destabilization after viewing each painting in the dyslexic and non-dyslexic populations.

**Figure 5A.** Example of a dyslexic response with high fluidity (score of 10) and high flexibility (score of 10) to Part 2 of the figural portion of the Torrance Test for Creative Thinking.

**Figure 5B.** Example of a non-dyslexic response with low fluidity (score of 3) and high flexibility (score of 3) to Part 2 of the figural portion of the Torrance Test for Creative Thinking.

**Figure 6A.** Example of one dyslexic participant's eye tracing over the 30 second viewing period of René Magritte, La trahison des images, 1973.

**Figure 6B.** Example of one dyslexic participant's eye tracing over the 30 second viewing period of René Magritte, La condition humaine I, 1935.

**Figure 6C.** Example of one dyslexic participant's eye tracing over the 30 second viewing period of René Magritte, The Art of Conversation-Color Variation, 1950.

**Figure 7.** Subjective reports of appreciation, perception of the bizarre, and contradiction in each population.

**Figure 8.** Number of spaces perceived in Painting #2 by population.

**Figure 9.** Number of words perceived in Painting #3 by population.

**Figure 10.** Illustration of the selected segment by our algorithm and the different steps for the DGI algorithm.

**Figure 11.** Pairplot of the accuracy of the Support vector machine and the logistic regression when using Aideal based feature on the analysis 1,2 and 3.

**Figure 12.** Combined painting - histogram of the amplitude segments.

**Figure 13A.** Histogram of the amplitude segments for the Painting 1 dataset.

**Figure 13B.** Histogram of the amplitude segments for the Painting 2 dataset.

**Figure 13C.** Histogram of the amplitude segments for the Painting 3 dataset.

**Figure 13D.** Histogram of the amplitude segments for the Painting 4 dataset.

**Figure 13E.** Histogram of the amplitude segments for the Painting 5 dataset.

**Figure 13F.** Histogram of the amplitude segments for the Painting 6 dataset.

**Figure 13G.** Histogram of the amplitude segments for the Painting 7 dataset.

**Figure 14A.** Distributions of the DGI (frequency), mean duration and mean amplitude of disconjugate segments for the Painting 1 dataset.

**Figure 14B.** Distributions of the DGI (frequency), mean duration and mean amplitude of disconjugate segments for the Painting 2 dataset.

**Figure 14C.** Distributions of the DGI (frequency), mean duration and mean amplitude of disconjugate segments for the Painting 3 dataset.

Figure 14D. Distributions of the DGI (frequency), mean duration and mean amplitude of disconjugate segments for the Painting 4 dataset.

**Figure 14E.** Distributions of the DGI (frequency), mean duration and mean amplitude of disconjugate segments for the Painting 5 dataset.

**Figure 14F.** Distributions of the DGI (frequency), mean duration and mean amplitude of disconjugate segments for the Painting 6 dataset.

**Figure 14G.** Distributions of the DGI (frequency), mean duration and mean amplitude of disconjugate segments for the Painting 7 dataset.

**Figure 15.** Pair plot of the accuracy of the Support vector machine and the logistic regression when using DGI based feature on the analysis 1,2 and 3.

**Figure 16.** Method for saccade analysis, illustrated for saccades of 20° in an individual with vergence disorders (TC). All trials are superimposed at t = 0, the instant of target appearance. The bold lines show an individual trial of saccade to the left (A, B): Onset (I) and offset (P) of eye movements were defined according to velocity threshold (V), traces of velocity being illustrated at the bottom (C). Note that coordination of saccades may be evaluated from the disconjugate amplitude (B).

**Figure 17.** Method for vergence analysis: convergence and divergence were obtained by subtraction of right eye (RE) position from the left eye (LE) position (LE - RE). The corresponding velocity trace is shown *below.* Mark of onset and offset of vergence is based on velocity criteria: the time point when the eye velocity respectively exceeded or dropped below 5°/s. The *horizontal dotted lines* indicate the targets' (T for divergence, T' for convergence) position.

## DETAILED DESCRIPTION OF THE INVENTION

### Method for measuring binocular motricity parameters in an individual

The present invention first relates to a method for measuring binocular motricity parameters in an individual, comprising:

a1) inviting the individual to free exploration of one or several pictorial artwork(s);

b1) recording the eye movements of the left eye and the right eye of the individual during the free exploration of the one or more pictorial artwork(s); and

c1) calculating, using a digital processing device, the value of one or more binocular motricity parameter(s) from the records obtained in step b1);

wherein when several pictorial artworks are explored, they are explored one by one, and

wherein each of the one or more pictorial artwork(s) is selected from pictorial artworks creating an illusion of visual motion in depth and pictorial artworks with spatial or sense ambiguity.

***Step a1): free exploration of one or several pictorial artwork(s)***

The method for measuring binocular motricity parameters according to the invention relies on free exploration of one or several pictorial artwork(s) selected from pictorial artworks creating an illusion of visual motion in depth and pictorial artworks with spatial or sense ambiguity.

Pictorial artwork(s)

The pictorial artwork(s) is(are) preferably two-dimensional pictorial artwork(s), i.e. any form of visual art that exists in two dimensions, positioned in a vertical plane. Any type of two-dimensional pictorial artwork may be used, including paintings, drawings, lithographies, prints, and photographs, or any reproduction thereof on a two-dimensional support (sheet, screen...). The pictorial artwork(s) (preferably two-dimensional pictorial artwork(s) positioned in a vertical plane) may use black and white, shades of grey or color.

The pictorial artwork(s) (preferably two-dimensional pictorial artwork(s) positioned in a vertical plane) that the individual is invited to freely explore is(are) selected from pictorial artworks creating an illusion of visual motion in depth and pictorial artworks with spatial or sense ambiguity. The selection (of pictorial artwork(s) (preferably two-dimensional pictorial artwork(s) positioned in a vertical plane) creating an illusion of visual motion in depth is based on the fact that more creative dyslexic individuals are perceptually more sensitive than less creative non-dyslexic individuals to the illusion of movement in depth. The selection of pictorial artwork(s) (preferably two-dimensional pictorial artwork(s) positioned in a vertical plane) with spatial or sense ambiguity is based on the fact that more creative dyslexic individuals have a different capacity in analyzing complex space compositions, or contradictory images than less creative non-dyslexic individuals.

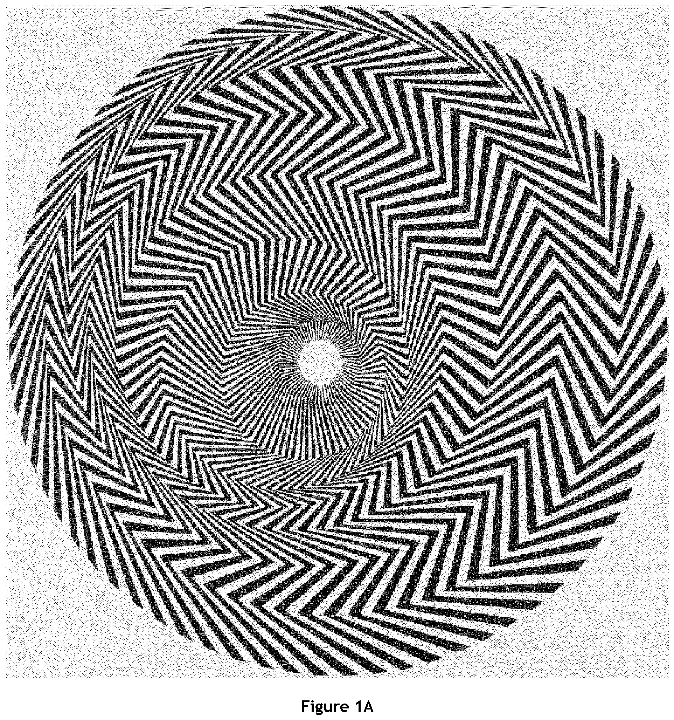

As used herein "**pictorial artwork creating an illusion of visual motion in depth**" refers to any pictorial artwork able to manipulate depth perception and cause an illusion of the pictorial artwork moving either into an anterior or posterior plane. Many examples of such pictorial artworks are available in the art. As an illustration, Op-art paintings from Bridget Riley may be used, such as *Blaze 1* (1962), *Movements in square* (1961), and *Cataract 3* (1967) (see **Figures 1A to 1C**). But other pictorial artworks creating an illusion of visual motion in depth may be used instead.

As used herein "**pictorial artwork with spatial ambiguity**" refers to any pictorial artwork stimulating bistable perception (i.e. containing visual patterns that are too ambiguous for the human visual system to definitively and uniquely interpret them, leading to an alternation between two mutually exclusive perceptual states) or containing hidden words or objects (i.e. words or object that are not immediately apparent). Here also, many examples of such pictorial artworks are available in the art. As an illustration, many of Escher's pictorial artworks (including the lithography *Relativité*, 1953) stimulate bistable perception through the presence of Schroder's stairs (i.e. stairs which may be perceived either as a drawing of a staircase leading from left to right downwards or the same staircase only turned upside down), and René Magritte painting *The Art of Conversation-Color Variation*, 1950, contains a French word ("REVE", translated as "dream") dissimulated into a rock formation. But other pictorial artworks with spatial ambiguity may be used instead.

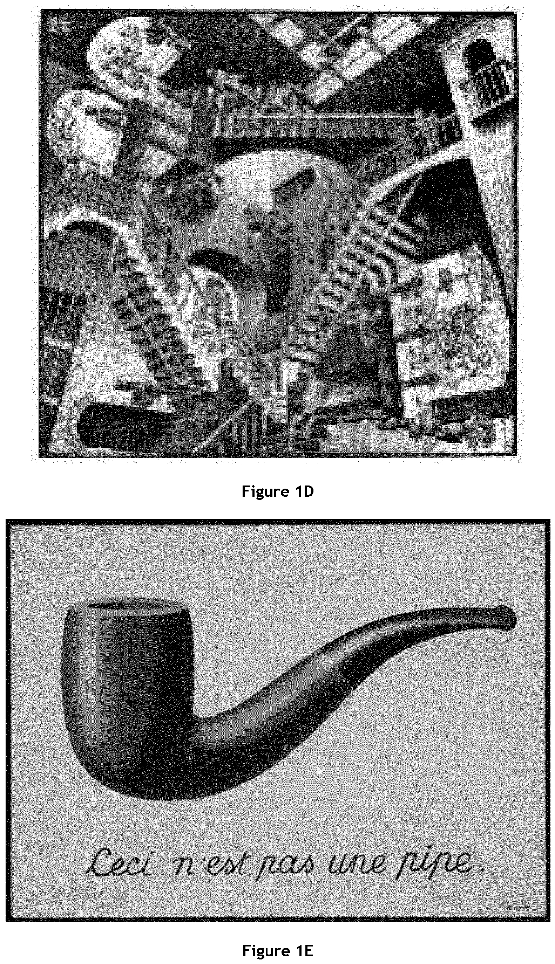

As used herein "**pictorial artwork with sense ambiguity**" refers to any pictorial artwork containing contradictory parts. For instance, the pictorial artwork may contain contradiction between image and text present on the pictorial artwork, it is then referred to "**image/text ambiguity**". Various examples of such pictorial artworks are available in the art. As an illustration, René Magritte painting *La trahison des images,* 1973, appears to demonstrate a direct contradiction between image and text message: while the painting depicts a pipe, it also depicts a sentence underneath the pipe that reads "Ceci n'est pas une pipe [This is not a pipe]" (see **Figure 1E**), and is thus an example of a pictorial artwork with image/text ambiguity. But other pictorial artworks with sense ambiguity (such as image/text ambiguity) may be used instead.

Pictorial artworks with spatial or sense ambiguity may thus be selected from Escher and Magritte pictorial artworks, preferably from:

- Escher, Relativité, 1953,
- René Magritte, La trahison des images, 1973,

- René Magritte, La condition humaine I, 1935, and
- René Magritte, The Art of Conversation-Color Variation, 1950.

Pictorial artworks with spatial or sense ambiguity may also be selected from Magritte pictorial artworks, preferably from:

- René Magritte, La trahison des images, 1973,
- René Magritte, La condition humaine I, 1935, and
- René Magritte, The Art of Conversation-Color Variation, 1950.

But other pictorial artworks with spatial or sense ambiguity may be used instead.

In the method for measuring binocular motricity parameters according to the invention, the individual is preferably submitted to free exploration of several pictorial artwork(s) in step a1. In particular, the individual may be submitted to free exploration of:

i) several pictorial artworks creating an illusion of visual motion in depth,
ii) several pictorial artworks with spatial or sense ambiguity (in particular text/image ambiguity), or
iii) one or more pictorial artwork(s) creating an illusion of visual motion in depth and one or more pictorial artwork(s) with spatial or sense ambiguity (in particular one or more pictorial artwork(s) creating an illusion of visual motion in depth and one or more pictorial artwork(s) with text/image ambiguity).

When the individual is submitted to free exploration of i) several pictorial artworks creating an illusion of visual motion in depth, two or more pictorial artworks creating an illusion of visual motion in depth may be used, such as 2 to 5, 2 to 4, in particular 2 or 3. Notably, 2 or 3 Op-art paintings from Bridget Riley may be used, selected from Blaze 1 (1962), Movements in square (1961), and Cataract 3 (1967). For instance, Blaze 1 (1962) and Movements in square (1961), Blaze 1 (1962) and Cataract 3 (1967), Movements in square (1961) and Cataract 3 (1967), or Blaze 1 (1962), Movements in square (1961), and Cataract 3 (1967) may be used.

When the individual is submitted to free exploration of ii) several pictorial artworks with spatial or sense ambiguity, two or more pictorial artworks with spatial or sense ambiguity may be used, such as 2 to 6, 3 to 5, in particular 3 or 4. Preferably, the two or more pictorial artworks with spatial or sense ambiguity include one or more (such as 1 to 4, notably 1, 2, 3, or 4) pictorial artwork(s) with spatial ambiguity (for instance selected from those mentioned above for illustration purposes) and one or more (such as 1 or 2) pictorial artwork(s) with sense ambiguity (for instance René Magritte's painting La trahison des images, 1973). Notably, a combination of René Magritte paintings La trahison des images (1973), La condition humaine I (1935) and The Art of Conversation-Color Variation (1950) may be used, optionally with also Escher's lithography, Relativité, 1953.

When the individual is submitted to free exploration of iii) one or more pictorial artwork(s) creating an illusion of visual motion in depth and one or more pictorial artwork(s) with spatial or sense ambiguity, the following combinations may be used:

- 2 to 5 pictorial artworks creating an illusion of visual motion in depth and 2 to 6 pictorial artworks with spatial or sense ambiguity;
- 2 to 5 pictorial artworks creating an illusion of visual motion in depth, 1 to 4 pictorial artwork(s) with spatial ambiguity and 1 or 2 pictorial artwork(s) with sense ambiguity;
- 2 or 3 pictorial artworks creating an illusion of visual motion in depth and 3 or 4 pictorial artworks with spatial or sense ambiguity;
- 2 or 3 pictorial artworks creating an illusion of visual motion in depth, 2 or 3 pictorial artworks with spatial ambiguity, and 1 or 2 pictorial artwork(s) with sense ambiguity;
- 2 or 3 Op-art paintings from Bridget Riley and 3 or 4 pictorial artworks with spatial or sense ambiguity from René Magritte and Escher or from René Magritte only;
- 2 or 3 of Bridget Riley's Blaze 1 (1962), Movements in square (1961), and Cataract 3 (1967), 2 or 3 of René Magritte's paintings La trahison des images (1973), La condition humaine I (1935) and The Art of Conversation-Color Variation (1950), and optionally Escher's lithography, Relativité, 1953; and
- All of Bridget Riley's Blaze 1 (1962), Movements in square (1961), and *Cataract 3,* René Magritte's paintings La trahison des images (1973), La condition humaine I (1935) and The Art of Conversation-Color Variation (1950), and optionally Escher's lithography, Relativité, 1953.

Procedure of free exploration

The individual is invited to free exploration of one or several pictorial artwork(s) (preferably two-dimensional pictorial artwork(s) positioned in a vertical plane).

By "**free exploration**", it is meant the individual is invited to explore the pictorial artwork(s) (preferably two-dimensional pictorial artwork(s) positioned in a vertical plane) as he/she wishes for a given period of time, generally 15 seconds to 1 minute, such as 20 to 40 seconds, in particular 30 seconds.

When using a two-dimensional pictorial artwork, the two-dimensional pictorial artwork (or any support, such as a screen, reproducing the pictorial artwork) is positioned in a vertical plane, at the eyes level of the individual, who may be standing or sitting.

The individual is preferably instructed to keep his/her head still and not to move his/her body during free exploration.

When several pictorial artworks (preferably several two-dimensional pictorial artworks positioned in a vertical plane) are explored in step a1), a rest period of 15 seconds to 1 minute, such as 20 to 40 seconds, in particular 30 seconds, is preferably inserted between the free explorations of two distinct pictorial artworks.

***Step b1): recording the eye movements of the left eye and the right eye of the individual***

In step b1), which occurs simultaneously with step a1), the eye movements of the left eye and the right eye of the individual during the free exploration of the pictorial artwork(s) of step a1) are recorded.

This may be performed using any appropriate eye tracker (also referred to as an eye movement acquisition and recording system). The eye movement acquisition and recording system works advantageously with a video-oculography system, i.e. micro-cameras that focus their lenses on both eyes and record their movements when the person is looking at the device to stimulate binocular motricity (vergence and saccade tests) or when he or she is reading (reading test).

Such a system is for example an eyetracker from the company Pupil Labs™, such as the head mounted device Pupil Core, enabling binocular recording at 200 Hz per eye (https://pupil-labs.com/products/core/)

Another type of eye movement acquisition system is the Powref III from the company PlusOptix™. Such a type of system is placed at a distance from the person, for example in front of the person on support containing the explored pictorial artwork. This system allows to measure the eye movement but also, at a distance, the change of eye accommodation for and to plot eye positions but also the plot of the change of eye accommodation. In this regard, one can refer to the document (https://www.medicus.ua/eng/product/ophthalmic-equipment/plusoptiX-R09-PowerRef-3/manufacturer:, 8 "HSOA Journal of Clinical Studies and Medical Case Reports", Kapoula Z, et al, J Clin Stud Med Case Rep 2019, 6: 74).

Other examples of suitable eye trackers include the EyeSeeCam system (University of Munich Hospital, Clinical Neuroscience, Munich, Germany, available in the public domain at http://eyeseecam.com/,) or other remote devices e.g. Tobbii (https://www.tobii.com/), Powref (https://plusoptix.com/home).

***Step c1): calculating, using a digital processing device, the value of one or more binocular motricity parameter(s)***

In step c1), one or more binocular motricity parameter is calculated from the records obtained in step b1) using a digital processing device.

Said one or more binocular motricity parameter(s) calculated in step c1) may be selected from:

i) binocular motricity parameters of the horizontal and/or vertical component(s) of saccades, and/or
ii) frequency, mean duration and mean amplitude of disconjugacy segments during the whole exploration of each pictorial artwork.

Classical binocular motricity parameters during saccades

In the method for measuring binocular motricity parameters according to the invention, one or more binocular motricity parameter(s) calculated in step c1) may be selected from binocular motricity parameters of the horizontal and/or vertical component(s) of saccades.

A "**saccade**" is a quick, simultaneous movement of both eyes between two or more phases of fixation in the same direction, horizontally, vertically or in oblique, i.e. horizontally and vertically at the same time e.g. looking up and right, down and left etc.

A saccade may be horizontal (it is then referred to a "horizontal saccade"), when both eyes move from one location to another location at the same height on the right (it is then referred to a "right saccade" or "rightward saccade") or on the left (it is then referred to a "left saccade" or "leftward saccade"). Horizontal saccades are thus a first type of saccade, left and right saccades being each a subtype of horizontal saccade. Horizontal saccades can be made with the eyes at different level of elevation: position of the eyes at the straight ahead and saccading left or right, eyes elevated and saccading left or right, eyes down and saccading left or right

A saccade may also be vertical (it is then referred to a "vertical saccade"), when both eyes move from one location to another location at the same lateral position but at a distinct height, either above (it is then referred to an "upward saccade") or below (it is then referred to a "downward saccade") the initial position. Vertical saccades are thus a second type of saccade, upward and downward saccades being each a subtype of vertical saccade. Vertical saccades can also start from different gaze positions e.g. eyes at the straight head position saccade up or down, or from eye fixating at a lateral left or right position and saccading up or down.

A saccade may also be oblique (it is then referred to an "oblique saccade"), when both eyes move from one location to another location at another height on the right or on the left. Depending on the specific initial and final locations, it may be referred to an "upward right saccade", an "upward left saccade", a "downward right saccade", or a "downward left saccade". Oblique saccades are thus a second type of saccade, upward right, upward left, downward right and downward left saccades being each a subtype of oblique saccade.

While the movements of horizontal and vertical saccades are included in only 1 dimension (horizontal and vertical, respectively), the movement of oblique saccades involves two dimensions: horizontal and vertical.

Therefore, as used herein, the "**horizontal component of saccades**" refers to the horizontal movements made by the left and right eyes during horizonal and oblique saccades, and the "**vertical component of saccades**" refers to the vertical movements made by the left and right eyes during vertical and oblique saccades.

The inventors have shown that various binocular motricity parameters of horizontal components of saccades significantly differ between more creative dyslexic individuals and less creative non-dyslexic individuals (see Examples 1 and 2), but the same finding may be expected for the vertical component of saccades.

Among binocular motricity parameters of horizontal and/or vertical components (preferably the horizontal component) of saccades, the one or more binocular motricity parameter(s) calculated in step c1) may be selected from:

- velocity parameters of the horizontal and/or vertical component(s) (preferably the horizontal component) of saccades, in particular peak velocity or average velocity of the initial phasic component (more preferably average velocity of the initial phasic component) of horizontal and/or vertical components (preferably the horizontal component) of saccades,
- amplitude parameters of the horizontal and/or vertical component(s) (preferably the horizontal component) of saccades, preferably amplitude of the initial phasic component of the horizontal and/or vertical component(s) (preferably the horizontal component) of saccades,
- duration parameters of the horizontal and/or vertical component(s) (preferably the horizontal component) of saccades, preferably initial phasic duration of the horizontal and/or vertical component(s) (preferably the horizontal component) of saccades, and
- horizontal and/or vertical disconjugacy during saccades.

Based on the recording of eye movements made by the eye tracker, the effective trajectory corresponding to a saccade is obtained by calculating a conjugate signal defined by the average of the position of the left eye with the position of the right eye (i.e. left+right eyes positions/2).

Each saccade effective trajectory is calculated between the time point when a visual or audiovisual stimulation inducing saccade movement starts and the time point when a next visual or audiovisual stimulation inducing saccade movement starts; during free exploration of pictorial artworks, the stimulation is triggered internally by the observer who decides on a moment to moment basis where to saccade next, where to look next.

A representative saccade trajectory corresponding to a mean trajectory of all effective trajectories is calculated, and effective trajectory at too much variance with the representative trajectory may be discarded for further analysis.

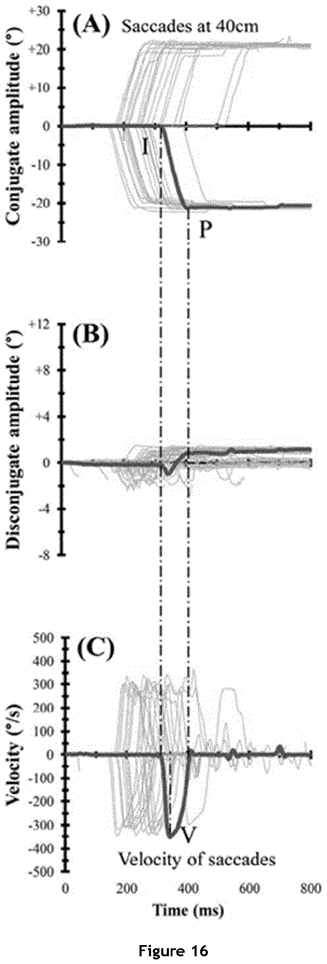

For each saccade effective trajectory, a corresponding effective velocity trajectory is further calculated by conventional calculation (see **Figure 16**, illustrating this for an individual with a vergence disorder tested for saccades of 40° on a REMOBI device).

Each effective velocity trajectory first permits to calculate the peak velocity of saccade (right or left), which is defined as the highest velocity value of the effective velocity trajectory (see **Figure 16**).

For each saccade effective trajectory and corresponding velocity trajectory, several parts of the saccade effective trajectory and specific time points may then be calculated (see **Figure 16**):

- Onset of saccade (right or left):
  This parameter is defined as the time point when the saccade velocity, initially close to zero has increased to reach 8-12%, preferably 10%, of the peak velocity (highest velocity in the effective trajectory), or when the saccade velocity

initially close to zero has increased to reach a predefined velocity. For instance, when using a saccade test involving a right or left saccade of about 20°, onset of saccade may be defined as the time point when the saccade velocity reaches 40-50°/s, preferably 45°/s.

- Offset of saccade (right or left):
This parameter is defined as the time point when the saccade velocity, after decreasing from the peak velocity reaches only 8-12%, preferably 10%, of the peak velocity, or when the saccade velocity after decreasing from the peak velocity reaches a predefined velocity. For instance, when using a saccade test involving a right or left saccade of about 20°, offset of saccade may be defined as the time point when the saccade velocity once more reaches 40-50°/s , preferably 45°/s after decreasing from peak velocity.
- Latency trajectory part (right or left):
This part of the effective saccade trajectory corresponds to the part between the time point when the visual or audiovisual stimulation starts (beginning of the trajectory) and right or left saccade onset.
- Initial phasic trajectory part of saccade or "initial phasic component":
This part of the effective saccade trajectory corresponds to the part between right or left saccade onset and offset.
- 80 ms drift trajectory part of saccade or "80 ms component":
This part of the effective saccade trajectory corresponds to the 80 ms after right or left saccade offset.
- 160 ms drift trajectory part of saccade or "160 ms component":
This part of the effective saccade trajectory corresponds to the 160 ms after right or left saccade offset.

Based on the above-defined time points and trajectory parts, saccade parameters may be calculated. Saccade parameters include:

- Duration parameters:

  ◦ Latency duration of saccade (right or left):
  This parameter is defined as the duration between the time point when the visual or audiovisual stimulation starts and right or left saccade onset.
  ◦ Initial phasic duration of saccade (right or left):
  This parameter is defined as the duration of the initial phasic trajectory part of saccade, i.e. the duration between right or left saccade onset and offset.
  ◦ Total duration:
  This parameter is defined as the sum of the initial phasic duration of saccade and the following 160 ms.

- Amplitude parameters:

  o Amplitude of the initial phasic component: This parameter is defined as (conjugated signal at offset - conjugated at signal onset).
  ◦ Amplitude of the 80 ms component: This parameter is defined as (conjugated signal 80 ms after offset - conjugated signal at offset).
  ◦ Amplitude of the 160 ms component: This parameter is defined as (conjugated signal 160 ms after offset - conjugated signal at offset).
  ◦ Total amplitude: This parameter is defined as the sum of the amplitude of the initial phasic component and the amplitude of the 160 ms component.

- Velocity parameters:

  o Peak velocity:
  As mentioned above, peak velocity is the highest velocity value of the effective velocity trajectory.
  Peak velocity of specific parts of each vergence effective trajectory may also be calculated, including the peak velocity of the initial phasic component. However, it should be noted that peak velocity is clearly found during the initial phasic component and is thus equal to the peak velocity of the initial phasic component
  ◦ Average velocity of the initial phasic component:
  This parameter is defined as the ratio between the amplitude and the duration of the initial phasic component.
  ◦ Total average velocity:
  This parameter is defined as the ratio between the total amplitude and the total duration, as defined above.

For saccades, to evaluate binocular motricity of saccades, a disconjugate signal (i.e., left eye - right eye, see **Figure 16**) may also be first calculated and a disconjugacy parameter may then be calculated, defined as the difference in saccade amplitude between the left and right eye signal may be calculated in the initial phasic component (referred to as "disconjugacy during saccade" parameter), or after the initial phasic component (referred to as "disconjugacy after saccade" or "post-saccadic drift disconjugacy"), in particular in the 80 ms component (referred to as "disconjugacy 80 ms after saccade" parameter), in the 160 ms component (referred to as "disconjugacy 160 ms after saccade" parameter).

When disconjugacy during saccade is calculated using the horizontal component of saccades, it is referred to horizontal disconjugacy during saccades, and when disconjugacy during saccade is calculated using the vertical component of saccades, it is referred to vertical disconjugacy during saccades.

The above saccade parameters are well-known in the art, and may be calculated by any suitable software able to compute saccade effective trajectories from the recordings made by the eye tracker and calculate saccade parameters. Such software may preferably be the AIDEAL® software disclosed in WO2021/228724, the content of which is herein incorporated by reference.

Preferably, the one or more binocular motricity parameter(s) calculated in step c1) may be selected from:

- velocity parameters of the horizontal and/or vertical component(s) (preferably the horizontal component) of saccades, in particular peak velocity or average velocity of the initial phasic component (preferably average velocity of the initial phasic component) of the horizontal and/or vertical component(s) of saccades, and

- horizontal and/or vertical (preferably horizontal) disconjugacy during saccades.

A particularly preferred binocular motricity parameter that may be calculated in step c1) is the average velocity of the initial phasic component of the horizontal and/or vertical component(s) of saccades, in particular the average velocity of the initial phasic component of the horizontal component of saccades.

New binocular motricity parameters

Based on the recordings of step b1), the inventors further defined new binocular motricity parameters, based on the detection of all disconjugacy segments present during the whole exploration of each pictorial artwork.

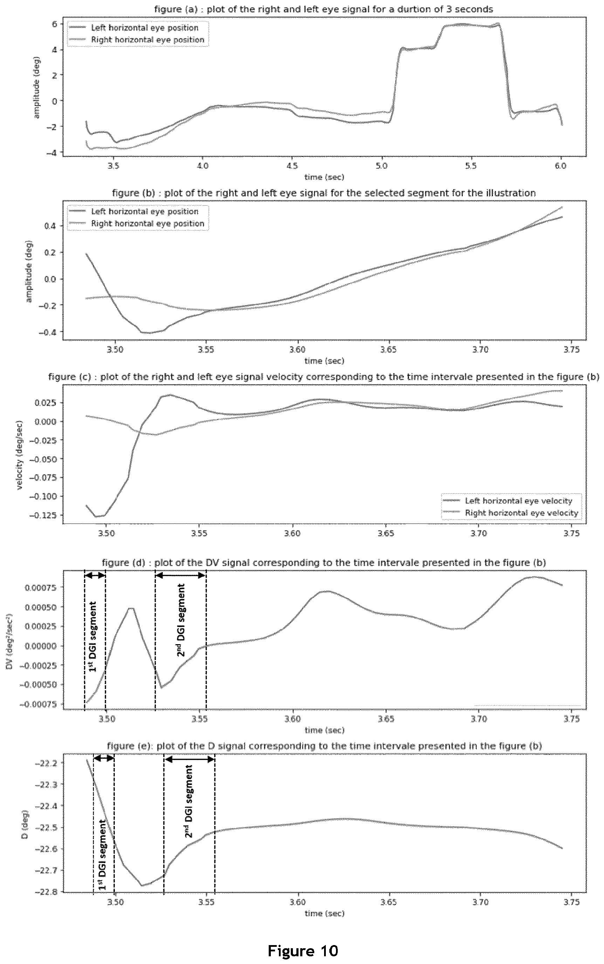

**Figure 10** illustrates the nature of the selected disconjugacy segments. The light grey curve represents the right eye movement coordinate over the disconjugacy axis, while the dark grey curve represents the left eye movement coordinate over the same axis.

A disconjugacy segment is defined as a time interval of at least 12 ms where two eyes are moving laterally in opposite directions (i.e. converging or diverging, as is done when changing depth to look at near vs far objects).

In the new parameters defined by the inventors, disconjugacy segments are detected based on the velocity and position of eye movement signals of the two eyes continuously, i.e. during the whole exploration of each pictorial artwork, as opposed to the approach relying on binocular motricity parameters of saccades defined above, which only take into account eyes movement during the saccades and the periods immediately before and after the movement.

The disconjugacy segments extracted from eyes movement recordings are visible in the position trace (Figure 10(d) and (e)). The disconjugacy segments are correspond to segments where the product of the right and left eye velocity signals is negative. For example, if the instantaneous velocities of the left eye and right eye are +10 degree/sec and -10 degree/sec respectively, then the product is -100 degree$^2$/sec$^2$ and this identifies an instant of a disconjugacy segment. In contrast, if the instantaneous velocities are +10 degree/sec and +20degree/sec for the left and right eye, respectively, then the product is 200 degree$^2$/sec$^2$ and therefore this instant is not part of a disconjugacy segment.

Based on the identification of disconjugacy segments during the whole exploration of each pictorial artwork, three new parameters may be calculated:

- the frequency of disconjugacy segments during the whole exploration of each pictorial artwork, also referred to in the present description as the "DGI index" or "DGI score".
- the mean duration of disconjugacy segments during the whole exploration of each pictorial artwork, and
- the mean amplitude of disconjugacy segments during the whole exploration of each pictorial artwork.

To evaluate the DGI index, the total number of disconjugacy segments during the whole exploration of each pictorial artwork is counted, and then divided by the total duration of each pictorial artwork free exploration. To count the total number of disconjugacy segments present in the recording of each pictorial artwork free exploration, the position of the left eye on the horizontal axis over time and the position of the right eye on the horizontal axis over time are retrieved (1). Then, the disconjugate signal, denoted by D and defined as the difference of the left horizontal signal minus the right horizontal signal (2) is computed. Also, the derivative of the left signal and the right signal and the product of the two derivatives, denoted by Dv (3) are computed. Then, the Dv signal is used to retrieve all the segments where the sign of the Dv signal is negative (4). Those segments are the moments where the two eyes were moving in the opposite direction over the X, horizontal axis, in other words, these are the moments where the two eyes were moving oppositely converging or diverging.

Each Dv segment is then split into sub-segments whenever the D corresponding signal changes its sign (5). It is important to consider both the Disconjugate position signal (D) and the product of the velocities of the two eyes (Dv) to be able to identify the periods during which the eyes move in opposite directions; the D signal alone does not give this information, as there may be instances where the eyes travel in the same direction but by different amounts.

This new analysis is more focused on identifying continuously all the instantaneous segments during which the eyes are traveling in opposite directions. Once each micro-diverging or micro-converging segment (i.e. each disconjugacy segment) is identified, all those segments that last for a period of at least 12 ms of time. This duration is based on a prior analysis testing different segment minimal periods and seems physiologically plausible and not just a noise in the eye-tracking signals.

Finally, the total number of the disconjugacy segments is counted, and divided by the total duration of the recording of the free exploration of a pictorial art (6).

The different steps to compute the DGI score are formally presented in the equations below, to which the numbers between parentheses above refer:

$$X_l = [l_1, l_2, ..., l_n], \ X_r = [r_1, r_2, ..., l_n], \tag{1}$$

$$D = \left[ X_l^{(i)} - X_r^{(i)} \right]_{i=1}^{n} \tag{2}$$

$$DV = \left[ \frac{X_l^{(i+1)} - X_l^{(i)}}{T^{(i+1)} - T^{(i)}} \times \frac{X_r^{(i+1)} - X_r^{(i)}}{T^{(i+1)} - T^{(i)}} \right]_{i=1}^{n} \tag{3}$$

$$E = \left\{ (s,l) \in [2,n] \times [s+3, n-1] \mid DV^{(s-1)} > 0 \ \& \ DV^{(l+1)} > 0 \ \& \ \forall i \in [s,l], DV^{(i)} < 0 \right\} \tag{4}$$

$$Count = \sum_{(s,l)}^{E} card(\left\{ (a,b) \in [s,n] \times ]a,l] \mid D^{(a-1)} \neq D^{(a)} \ \& \ D^{(b+1)} \neq D^{(b)} \ \& \ \forall i \in [a,b], D^{(i+1)} = D^{(i)} \right\}) \tag{5}$$

$$DGI = \frac{Count}{T^{(n)} - T^{(i)}} \tag{6}$$

In addition to the DGI score, two other binocular motricity parameters of the disconjugacy segments during the whole exploration of each pictorial artwork may be calculated in step c1): the mean amplitude and the mean duration of the different retrieved disconjugacy segments.

In the method for measuring binocular motricity parameters according to the invention, one or more binocular motricity parameter(s) calculated in step c1) may thus be selected from binocular motricity parameters of disconjugacy segments during the whole exploration of each pictorial artwork, in particular from frequency (DGI index), mean duration and mean amplitude of disconjugacy segments during the whole exploration of each pictorial artwork.

Preferably one or more binocular motricity parameter(s) calculated in step c1) is(are) selected from frequency (DGI index) and mean duration of disconjugacy segments during whole exploration of each pictorial artwork, as these two parameters are the more significantly different between more creative dyslexic and less creative non-dyslexic individuals (see Example 3).

Most preferably, one binocular motricity parameter calculated in step c1) is the frequency of disconjugacy segments during the whole exploration of each pictorial artwork (DGI index).

Combinations of parameters of saccades and parameters of disconjugacy segments

While binocular motricity parameters of horizontal and/or vertical components of saccades alone and binocular motricity parameters of disconjugacy segments during whole exploration of each pictorial artwork alone may be calculated in step c1), the inventors found that combining binocular motricity parameters of horizontal and/or vertical components of saccades and binocular motricity parameters of disconjugacy segments during whole exploration of each pictorial artwork could be useful to improve proper classification of individuals are suffering or not suffering from a learning disorder (see Example 3). As a result, binocular motricity parameter(s) calculated in step c1) may comprise:

a) one or more binocular motricity parameter(s) of the horizontal and/or vertical component(s) (preferably the horizontal component) of saccades as defined above, and
b) one or more binocular motricity parameters of disconjugacy segments during whole exploration of each pictorial artwork as defined above (preferably from frequency (DGI index) and mean duration of disconjugacy segments during whole exploration of each pictorial artwork).

In particular, binocular motricity parameter(s) calculated in step c1) may comprise:

a) binocular motricity parameters of the horizontal and/or vertical component(s) (preferably the horizontal component) of saccades, preferably the average velocity of the initial phasic component of the horizontal and/or vertical component(s) of saccades (in particular the average velocity of the initial phasic component of the horizontal component of saccades) and/or the horizontal and/or vertical disconjugacy during saccades (preferably the horizontal disconjugacy during saccades), and
b) the frequency of disconjugacy segments during the whole exploration of each pictorial artwork (DGI index).

In a particularly preferred embodiment, binocular motricity parameter(s) calculated in step c1) comprise:

a) the average velocity of the initial phasic component of the horizontal component of saccades and/or the horizontal disconjugacy during saccades, and
b) the frequency of disconjugacy segments during the whole exploration of each pictorial artwork (DGI index).

### *Optional steps d1), e1) and f1)*

While the method for measuring binocular motricity parameters according to the invention may be used alone, it may further comprise other tests of binocular motricity known to be useful in individuals.
In particular, the method for measuring binocular motricity parameters according to the invention may be completed by the method for measuring binocular motricity parameters described in WO2022/074130, which is herein entirely incorporated by reference.
The method for measuring binocular motricity parameters according to the invention may thus further comprise:

d1) submitting the individual to one or more ocular motor test(s) selected from a reading test, a vergence test and a horizontal saccade test;
e1) recording the eye movements of the left eye and the right eye of the individual during each of the ocular motor tests; and
f1) calculating, using a digital processing device, the value of at least one binocular motricity parameter from the records obtained in step e1);

wherein the vergence test and the saccade test are conducted using a binocular motor stimulation device configured to specifically and physically stimulate vergences and saccades, comprising visual or audiovisual targets placed in real space at eccentricities and depths of a surface;
wherein the vergence test comprises 30 to 50 trials of:

- visually and/or audiovisually stimulating the individual at a point located at the eyes level, in the central axis between the left eye and the right eye, at a first distance, and
- visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and

wherein the horizontal saccade test comprises 30 to 50 trials of:

- visually and/or audiovisually stimulating the individual at a point located at the eyes level, in the central axis between the left eye and the right eye, and

- visually and/or audiovisually stimulating the individual at another point located at the eyes level, on the left or on the right of the previous point, half of the trials being on the left, the other half on the right, the trials on the left and on the right being interleaved.

Optional step d1)

In step d1), the individual is submitted to ocular motor tests comprising at least a reading test, a vergence test and a saccade test.

*Reading test(s)*

A reading test is preferably performed using a text of 10 to 25 lines, preferably 10 to 20 lines, or 12 to 18 lines, preferably comprising 200 to 300 words, preferably 220 to 280 words. The text may or not make sense. Standardized reading tests (using texts that do or not make sense) are available in the art.

For instance, a well-known example of a French text of 265 words that does not make sense and that is routinely used in dyslexia diagnosis by orthophonist is called "L'Alouette", which is reproduced in **Figure 16A** of WO2022/074130.

Non sense texts testing of reading speed exists in all other languages used by speech therapists.

An example of a French text that does make sense and that may be used in methods according to the invention is an excerpt of 15 lines in black text on a white background from a children's book (35 Kilos D'Espoir, Anna Gavalda, Bayard Jeunesse), which is reproduced in **Figure 16B** of WO2022/074130. Other similar texts (10 to 20 lines, preferably 15 lines $\pm$ 1 or 2 lines) in black text on a white background, taken from a children's book, may be used instead. In addition to its length and presentation as defined in preceding sentence, the selected text that does make sense should preferably engage emotionally the reader.

In some cases, both a text that makes sense and a text that does not make sense may be used.

If only one type of text is used for reading testing, it is preferably a text that does not make sense (for example "L'Alouette" for French-speaking individuals), as the inventors have shown that increased text difficulty due to absence of sense further destabilizes the fragile ocular control of individuals suffering from a learning disorder (see Example 8 of WO2022/074130).

*Vergence and horizontal saccade tests*

A "**vergence**" is the simultaneous movement of both eyes in opposite directions to increase or decrease the angle of the optic axes (the vergence angle) to obtain or maintain single binocular vision of objects located at different depths. To look at an object closer by, the eyes rotate towards each other (convergence), while for an object farther away they rotate away from each other (divergence). Vergence is always connected with some vertical movements, upward saccade for diverging and downward saccade for converging. This vertical component is however minimized when using the device according to the invention in horizontal position but intrinsically the physiologic synergy is always present.

The vergence and saccade tests to which the individual is submitted in the invention are conducted using a binocular motor stimulation device configured to specifically and physically stimulate vergences and saccades, comprising visual or audiovisual targets placed in real space at eccentricities and depths of a surface. In this case, detected vergence abnormalities may not be due to language understanding difficulties. These tests thus permit to detect intrinsic vergence and/or saccade abnormalities.

Any binocular motor stimulation device configured to specifically and physically stimulate vergences and saccades and comprising visual or audiovisual targets placed in real space at eccentricities and depths of a surface may be used for the vergence test(s). A device for stimulating binocular motricity by sensory stimulation means such as diodes (visual stimulation) and optionally sound diffusion means (audio stimulation), which are arranged along different arcs of iso-vergence (an arc of iso-vergence is such that the vergence angle of both eyes required to fix each point on this arch is the same) in front of a person may notably be used. This device advantageously comprises four arcs of iso-vergence. Preferably, the sensory stimulation means are arranged on a flat support. The means for sensory stimulation are arranged on arcs at a distance of between 20 and 150 cm from an individual and between each means of stimulation placed on the same arc there is an angle of 10 degrees. The support is advantageously mounted on a height-adjustable stand allowing the device to be properly positioned at the level of the face of an individual. The support for the sensory stimulation means is preferably trapezoidal and has suitable dimensions to test the entire natural range of movements of the eyes (saccades at different depths of iso-vergence arcs, vergences along the median plane, movements combining saccades and vergences) and rehabilitate binocular motricity in direction (saccades), in depth (vergences) or combining them. The stimulation offered by this type of device is flexible, it can be audiovisual (thanks to light diodes and sound means), only audio or only visual. The means for visual and audio stimulation constitute targets for the person.

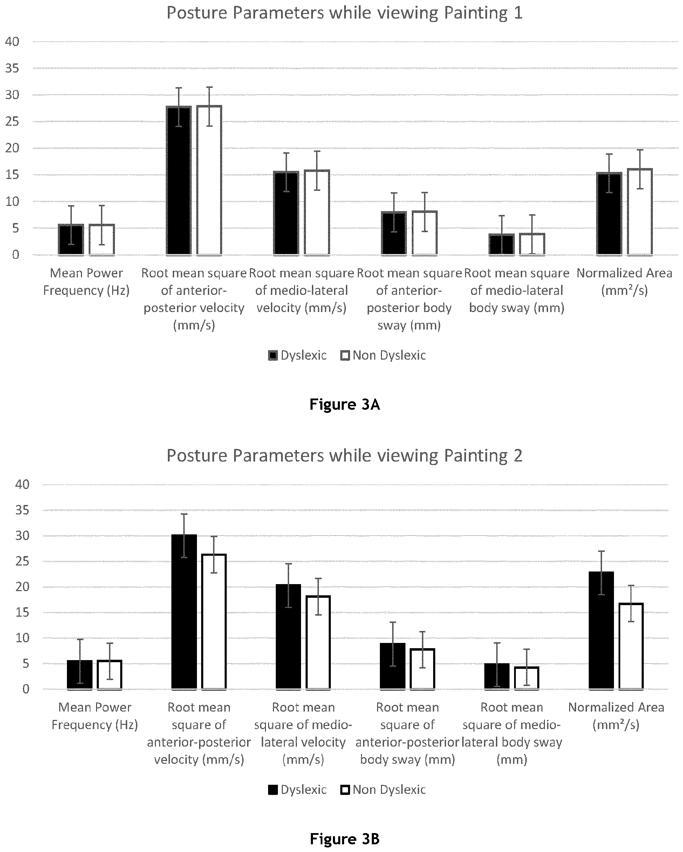

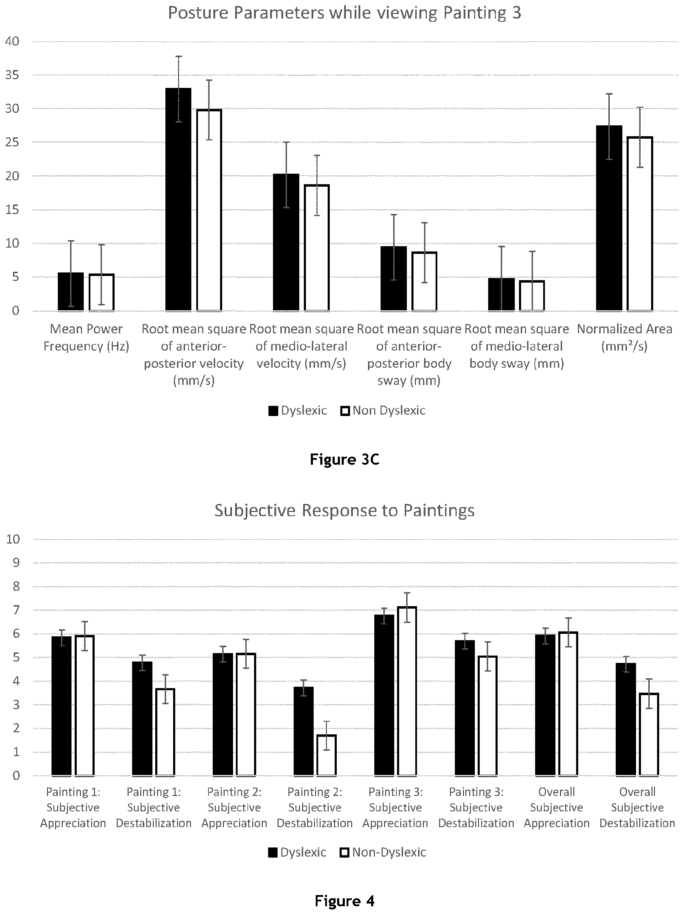

A preferred such device is the REMOBI® device, which structure is notably described in US8851669 and WO2011073288A1, the content of which are herein incorporated by reference. In particular, Figure 3 of US8851669 and WO2011073288A1 illustrate a bottom view of a device for the rehabilitation and/or training device for the binocular motivity of a patient according to the invention (display means for visual stimuli and means of generating auditory stimuli).

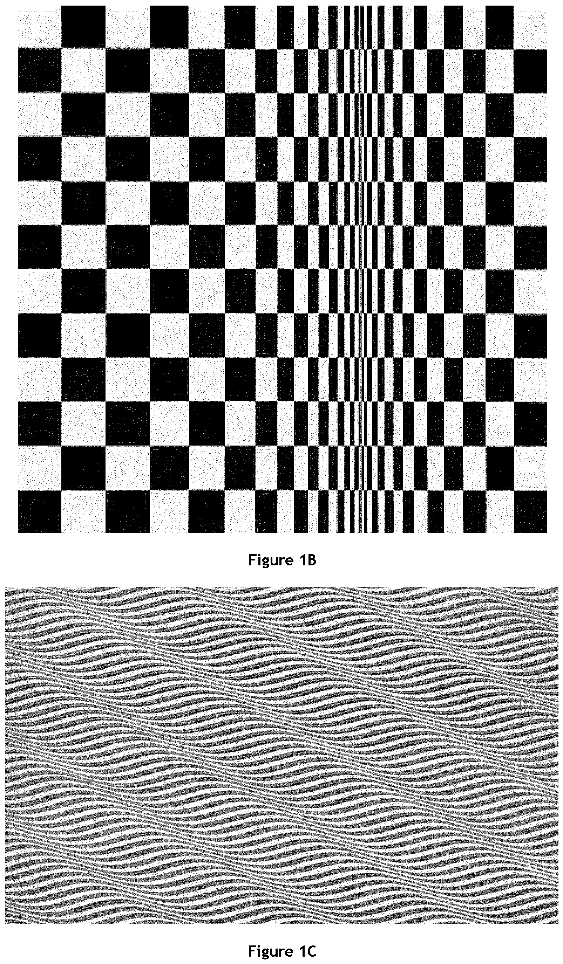

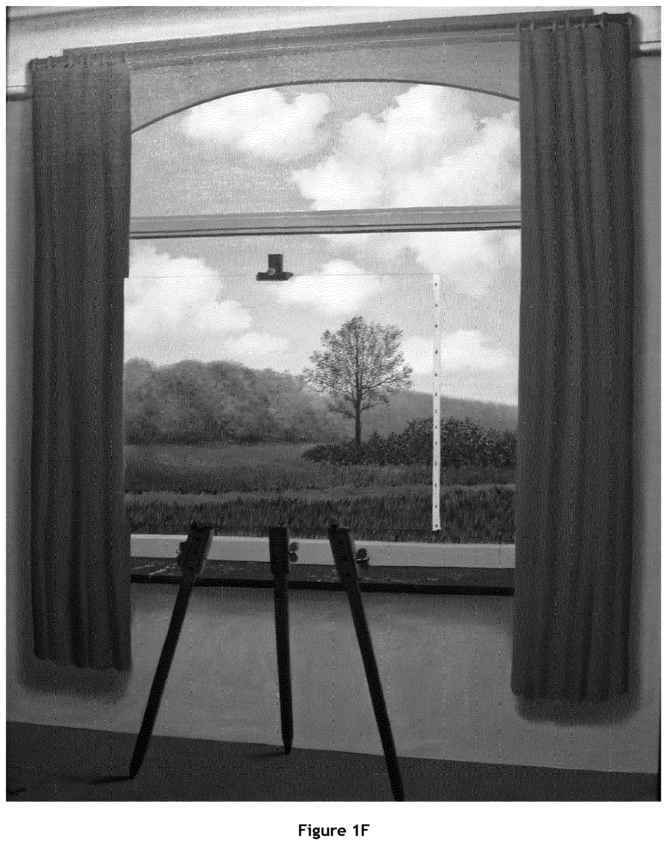

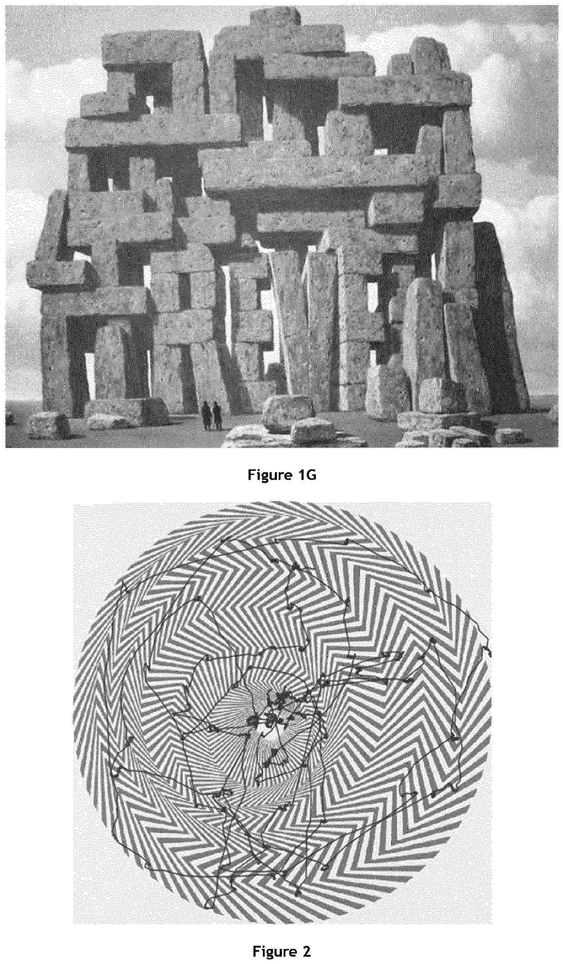

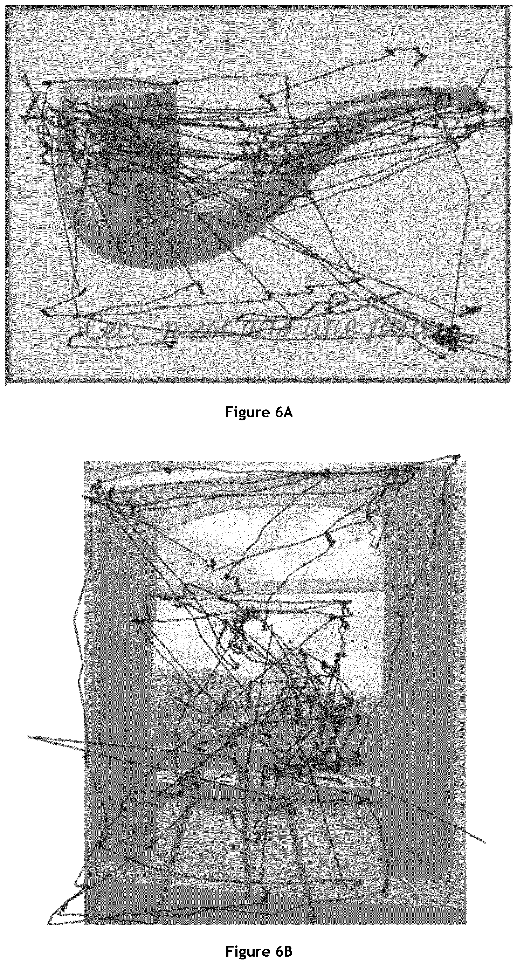

Another preferred device in the REMOBI-Neurocog device disclosed in European patent application filed on January 20, 2022 under number EP22305061.8, which is herein entirely incorporated by reference. In particular, Figures 1-9 of EP22305061.8 illustrate various views of the REMOBI-Neurocog device and Figures 10-12 how vergence and saccade tests may be performed using this device.

Due to the presence in the REMOBI® device and in the REMOBI-Neurocog device at quasi the same locations of both visual and audio targets (it is then referred to audiovisual targets), it is possible to perform vergence and saccade tests either using audiovisual stimulation (in this case, each stimulation involves both a visual signal and an audio signal) or using visual stimulation only (in this case, each stimulation involves only a visual signal, but no audio signal).

In the case of audiovisual stimulation, the visual signal and the audio signal start preferably simultaneously or very near, typically the audio signal precedes the visual signal by 50 msec and its duration is shorter (about 50-200 ms, preferably about 100 ms) than the visual signal (generally more than 1000 ms).

The vergence and/or the saccade test(s) may be performed twice, once using audiovisual stimulation and once using visual stimulation only. The vergence/saccade test using audiovisual stimulation and the vergence/saccade test using visual stimulation only may be performed in any order.

A vergence test comprises 30 to 50 trials, preferably an even number of trials between 30 and 50, more preferably between 36 and 44 trials, such as 36, 38, 40, 42 or 44 trials, in particular 40 trials.

Similarly, a horizontal saccade test comprises 30 to 50 trials, preferably an even number of trials between 30 and 50, more preferably between 36 and 44 trials, such as 36, 38, 40, 42 or 44 trials, in particular 40 trials.

When using the REMOBI® device, preferred protocol for the vergence test and the horizontal saccade test are disclosed in WO2022/074130.

When using the REMOBI-Neurocog device, preferred protocol for the vergence test and the horizontal saccade test are disclosed in EP22305061.8.

Step d1) may further comprise submitting the individual to one or more image analysis test(s). In such test, an image is given to the individual and his/her eyes movements are recorded during his/her analysis of the image. The individual may then be questioned about what is represented in the image and what story the image discloses. The sections of the image fixated by the eyes may then be analyzed (the eyes trajectories may be reported on the image to see what parts of the image have been fixated and in which order) and compared to the verbal reports and understanding of the image provided by the individual. This type of test is used typically by neurologists and neuropsychologists for functional exploration of the attention and cognitive executive process. Comparison of verbal reports of the patient with the eye movement exploration enables better understanding on the deployment of the image analysis by the patient (see Examples 7 and 10 of WO2022/074130). Other tests, including Stroop test, Test of Visual Perceptual Skills, EVADYS evaluation of visuo-attentional span, and DEM development eye movement test, all well known in the art, may also or alternatively be used. For instance, Daniel & Kapoula have shown the utility of the use of the Stroop test combined with eye movement recording and analysis for evaluating the interplay between quality of binocular motricity and cognition (see e.g., François Daniel, Zoï Kapoula Sci Rep. 2019; 9: 1247 ; François Daniel et al. Front Integr Neurosci. 2016; 10: 33. Published online 2016 Oct 20).

Optional step e1)

In step e1), which occurs simultaneously with step d1), the eye movements of the left eye and the right eye of the individual during the ocular motor tests of step a) are recorded. This may be done as described above for non-optional step b1).

Optional step f1)

In step f1), at least one binocular coordination parameter is calculated using a digital processing device from the records obtained in step e1). When present, calculation step f1) may or not be simultaneously performed with calculation step c1).

*Calculation of saccade parameters during horizontal saccade test or reading test*

Saccade parameters during a reading test or a horizontal saccade test may be calculated as described above for calculation of classical binocular motricity parameters during saccades in step c1).

*Calculating vergence parameters during vergence tests*

Based on the recording of eye movements made by the eye tracker, the effective trajectory corresponding to each vergence is obtained by calculating an unconjugated signal defined by the difference of the position of the left eye with the position of the right eye (i.e., left eye - right eye).

Each vergence effective trajectory is calculated between the time point when a visual or audiovisual stimulation inducing vergence movement starts and the time point when a next visual or audiovisual stimulation inducing vergence movement starts.

In any vergence test, a representative vergence trajectory corresponding to a mean trajectory of all effective trajectories is calculated, and effective trajectory at too much variance with the representative trajectory may be discarded for further analysis.

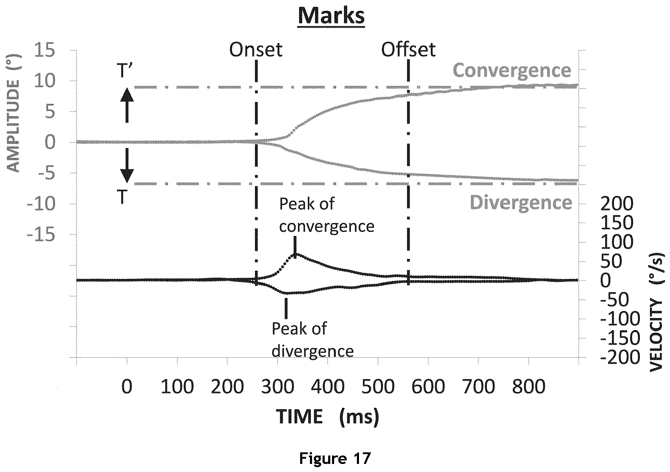

For each vergence effective trajectory, a corresponding effective velocity trajectory is further calculated by conventional calculation (see **Figure 17**).

Each effective velocity trajectory first permits to calculate the peak velocity of vergence (divergence or convergence), which is defined as the highest velocity value of the effective velocity trajectory (see **Figure 17**).

For each vergence effective trajectory and corresponding velocity trajectory, several parts of the vergence effective trajectory and specific time points may then be calculated (see **Figure 17**):

- Onset of vergence (convergence or divergence):
  This parameter is defined as the time point when the vergence velocity, initially close to zero has increased to reach 8-12%, preferably 10%, of the peak velocity (highest velocity in the effective trajectory), or when the vergence velocity initially close to zero has increased to reach a predefined velocity. For instance, when using a vergence test involving a convergence or divergence of about 7-9°, onset of vergence may be defined as the time point when the vergence velocity reaches 4-6°/s, preferably 5°/s.
- Offset of vergence (convergence or divergence):
  This parameter is defined as the time point when the vergence velocity, after decreasing from the peak velocity reaches only 8-12%, preferably 10%, of the peak velocity, or when the vergence velocity after decreasing from the peak velocity reaches a predefined velocity. For instance, when using a vergence test involving a convergence or divergence of about 7-9°, offset of vergence may be defined as the time point when the vergence velocity once more reaches 4-6°/s, preferably 5°/s after decreasing from peak velocity.
- Latency trajectory part (convergence or divergence):
  This part of the effective vergence trajectory corresponds to the part between the time point when the visual or audiovisual stimulation starts (beginning of the trajectory) and convergence or divergence onset.
- Initial phasic trajectory part of vergence or "initial phasic component":
  This part of the effective vergence trajectory corresponds to the part between convergence or divergence onset and offset.
- 80 ms slower trajectory part of vergence or "80 ms component":
  This part of the effective vergence trajectory corresponds to the 80 ms after convergence or divergence offset.
- 160 ms slower trajectory part of vergence or "160 ms component":
  This part of the effective vergence trajectory corresponds to the 160 ms after convergence or divergence offset.

Based on the above-defined time points and trajectory parts, vergence parameters may be calculated. Vergence parameters include:

- Duration parameters:

  o Latency duration of vergence (convergence or divergence):
  This parameter is defined as the duration between the time point when the visual or audiovisual stimulation starts and convergence or divergence onset.
  ∘ Initial phasic duration of vergence (convergence or divergence):
  This parameter is defined as the duration of the initial phasic trajectory part of vergence, i.e. the duration between convergence or divergence onset and offset.
  ∘ Total duration:
  This parameter is defined as the sum of the initial phasic duration of vergence and the following 160 ms.

- Amplitude parameters:

◦ Amplitude of the initial phasic component:

This parameter is defined as (unconjugated signal at offset - unconjugated at signal onset).

◦ Amplitude of the 80 ms component:

This parameter is defined as (unconjugated signal 80 ms after offset-unconjugated signal at offset).

◦ Amplitude of the 160 ms component:

This parameter is defined as (unconjugated signal 160 ms after offset-unconjugated signal at offset).

◦ Total amplitude:

This parameter is defined as the sum of the amplitude of the initial phasic component and the amplitude of the 160 ms component.

- Velocity parameters:

o Peak velocity:

As mentioned above, peak velocity is the highest velocity value of the effective velocity trajectory. Peak velocity of specific parts of each vergence effective trajectory may also be calculated, including the peak velocity of the initial phasic component. However, it should be noted that peak velocity is clearly found during the initial phasic component and is thus equal to the peak velocity of the initial phasic component.

◦ Average velocity of the initial phasic component:

This parameter is defined as the ratio between the amplitude and the duration of the initial phasic component.

◦ Total average velocity:

This parameter is defined as the ratio between the total amplitude and the total duration, as defined above.

Vergence parameters are preferably calculated separately for divergence and convergence trajectories.

No matter which vergence parameter(s) is(are) calculated, the calculation may be made using any suitable software able to compute vergence effective trajectories from the recordings made by the eye tracker and calculate vergence parameters. Such software may preferably be the AIDEAL® software disclosed in WO2021/228724, the content of which is herein incorporated by reference.

### Individual

The method for measuring binocular motricity parameters according to the invention may be used for any individual, either healthy or suffering or suspected to suffer from any disorder, in particular learning disorders and autism spectrum disorders (ASD).

As exemplified below, it is particularly useful in individuals suffering or suspected to suffer from a learning disorder, as the binocular motricity parameters calculated in step c1) permit to classify an individual as suffering or not suffering from a learning disorder (see also below the method for detection of features of a learning disorder according to the invention).

The individual suffering or suspected to suffer from a learning disorder may be selected from individuals suffering or suspected to suffer from dyslexia, dyspraxia, dysphasia, dyscalculia, dysgraphia, and attention-deficit/hyperactivity disorder (ADHD), in particular from individuals suffering or suspected to suffer from dyslexia (see Examples 1-3 below).

In addition, there is evidence for artistic talents in autism and theories about a genetic basis although the role of epigenetics is also emphasized (Sotiropoulos MG, Anagnostouli M. Genes, brain dynamics and art: the genetic underpinnings of creativity in dancing, musicality and visual arts. J Integr Neurosci. 2021 Dec 30;20(4):1095-1104. doi: 10.31083/j.jin2004110. PMID: 34997732). Another study (Pennisi P, Giallongo L, Milintenda G, Cannarozzo M. Autism, autistic traits and creativity: a systematic review and meta-analysis. Cogn Process. 2021 Feb;22(1):1-36) highlights that autistic individuals show higher scores in elaboration and in original in creativity tests. Finally, a recent study also highlights a unique creative cognitive profile among children with autistic syndrome (Kasirer A, Adi-Japha E, Mashal N. Verbal and Figural Creativity in Children With Autism Spectrum Disorder and Typical Development. Front Psychol. 2020 Oct 27;11:559238. doi: 10.3389/fpsyg.2020.559238. PMID: 33192819; PMCID: PMC7652732). Moreover, problems of binocular motricity in persons with autism spectrum disorders are likely to be more frequent than in healthy, as many epidemiologic studies suggest vergence-accommodation disorders in persons with autism spectrum disorders (Coulter RA, Bade A, Jenewein EC, Tea YC, Mitchell GL. Near-point Findings in Children with Autism Spectrum Disorder and in Typical Peers. Optom Vis Sci. 2021 Apr 1;98(4):384-393. doi: 10.1097/OPX.0000000000001679. PMID: 33852554; PMCID: PMC8051934; Svend Erik Mouridsen, Bente Rich & Torben Isager (2017) Eye Disorders among Adult People Diagnosed with Infantile Autism in Childhood: A Longitudinal Case Control Study, Ophthalmic Epidemiology, 24:5, 332-335; Jolie R. Keemink et al. Eye Movements and Behavioural Responses to Gaze-Contingent Expressive Faces in Typically Developing Infants and Infant Siblings. Autism Res 2021, 14: 973-983). As a result, the use of the method for measuring binocular motricity parameters according to the invention is also expected to be useful for detecting features of an ASD in individuals suffering or suspected to suffer from an ASD.

Moreover, dyslexic individuals are known to be more creative than non-dyslexic individuals, and data presented in Examples 1-3 below comparing dyslexic and non-dyslexic populations also represents an example of data comparing more creative and less creative individuals. On this basis, the method for measuring binocular motricity parameters according to the invention is thus also expected to be useful for assessing the level of creativity of an individual, and may thus also be used in healthy individuals, in order to assess their level of creativity.

## Method for detecting features of a learning disorder or an autistic spectrum disorder in an individual

The present invention also relates to a method for detecting features of a learning disorder (in particular dyslexia) or an autistic spectrum disorder (in particular autism) in an individual, comprising:

a2) providing previously obtained recordings of the eye movements of the left eye and the right eye of the individual during free exploration of one or several pictorial artwork(s);
b2) calculating, using a digital processing device, the value(s) of at least one binocular motricity parameter from the records provided in step a2);
c2) comparing the value(s) calculated in step b2) to one or more reference value(s) obtained in one or more reference individual(s); and
d2) concluding to the presence or absence of features of a learning disorder (in particular dyslexia) or an autistic spectrum disorder (in particular autism) in the individual based on said comparison.

### Step a2): providing previously obtained recordings of the eye movements of the left eye and the right eye of the individual during free exploration of one or several pictorial artwork(s)

The method for detecting features of a learning disorder or an ASD in an individual according to the invention is based on previously obtained recordings of the eye movements of the left eye and the right eye of the individual during free exploration of one or several pictorial artwork(s) (preferably two-dimensional pictorial artwork(s) positioned in a vertical plane).

Such recordings have been made using an eye tracker as defined above for the method for measuring binocular motricity parameters according to the invention.

The free exploration of one or several pictorial artwork(s) (preferably two-dimensional pictorial artwork(s) positioned in a vertical plane) selected from pictorial artworks creating an illusion of visual motion in depth and pictorial artworks with spatial or sense ambiguity to which the individual has been submitted for obtaining the recordings provided in step a1) have the same general and preferred features and combinations of features as those disclosed above for the method for measuring binocular motricity parameters according to the invention.

### Step b2): calculating, using a digital processing device, the value(s) of at least one binocular motricity parameter from the records provided in step a2)

The binocular motricity parameter(s) calculated in step b2) of the method for detecting features of a learning disorder or an ASD according to the invention also have the same general and preferred features and combinations of features as those disclosed above for step c1) of the method for measuring binocular motricity parameters according to the invention.

### Step c2): comparing the value(s) calculated in step b2) to one or more reference value(s) obtained in one or more reference individual(s)

In order to detect features of a learning disorder (in particular dyslexia) or an ASD (in particular autism) in the individual, the value(s) of binocular motricity parameter(s) calculated in step b2) are compared with corresponding reference values obtained when submitting one or more individual(s) not suffering from a learning disorder nor from an ASD (preferably healthy individuals) to the same free exploration of one or several pictorial artwork(s) (preferably two-dimensional pictorial artwork(s) positioned in a vertical plane) and/or with reference values obtained when submitting one or more individual(s) suffering from a learning disorder or an ASD to the same free exploration of one or several pictorial artwork(s) (preferably two-dimensional pictorial artwork(s) positioned in a vertical plane).

Exemplary reference values of healthy individuals not suffering from a learning disorder nor from an ASD and of individuals suffering from a learning disorder (dyslexia) are provided in Examples 1-3 below for various binocular motricity parameters that may be calculated in the method of detecting features of a learning disorder (in particular dyslexia) according to the invention.

Exemplary reference values of individuals suffering from another learning disorder or from an ASD may be obtained by submitting a population of such individuals to the method for measuring binocular motricity parameters according to the invention.

***Step d2): concluding to the presence or absence of features of a learning disorder or ASD***

Based on the comparison made in step c2), it may be concluded to the presence or absence of features of a learning disorder (in particular dyslexia) or an ASD (in particular autism) in the individual.

In particular, it may be concluded to the presence of a features of a learning disorder (in particular dyslexia) in the individual if at least one binocular motricity parameter calculated in step b2) is significantly different from the corresponding reference value(s) obtained in one or more individual(s) not suffering from a learning disorder (preferably healthy individuals). Alternatively, it may be concluded to the presence of a features of a learning disorder (in particular dyslexia) in the individual if the binocular motricity parameter(s) calculated in step b2) are not significantly different from the corresponding reference value(s) obtained in one or more individual(s) suffering from a learning disorder (in particular dyslexia).

In contrast, it may be concluded to the absence of features of a learning disorder (in particular dyslexia) in the individual if the binocular motricity parameter(s) calculated in step b2) are not significantly different from the corresponding reference value(s) obtained in one or more individual(s) not suffering from a learning disorder (in particular healthy individuals).

Notably, reference normal ranges or average values of binocular motricity parameter(s) may be used in step d2). In this case, it may be concluded to:

- absence of features of a learning disorder (in particular dyslexia) in the individual if all binocular motricity parameters calculated in step b2) are less than 20% above or below the corresponding reference average value in healthy individuals, and

- presence of features of a learning disorder (in particular dyslexia) in the individual if at least one binocular motricity parameter calculated in step b2) is at least 20% above or below the corresponding reference average value in healthy individuals, and/or if the binocular motricity parameter(s) calculated in step b2) are not significantly different from the corresponding reference value(s) obtained in one or more individual(s) suffering from a learning disorder (in particular dyslexia).

Similarly, it is concluded to the presence of a features of an ASD (in particular autism) in the individual if at least one binocular motricity parameter calculated in step b2) is significantly different from the corresponding reference value(s) obtained in one or more individual(s) not suffering from an ASD (preferably healthy individuals). Alternatively, it may be concluded to the presence of a features of an ASD (in particular autism) in the individual if the binocular motricity parameter(s) calculated in step b2) are not significantly different from the corresponding reference value(s) obtained in one or more individual(s) suffering from an ASD (in particular autism).

In contrast, it is concluded to the absence of features of an ASD (in particular autism) in the individual if the binocular motricity parameter(s) calculated in step b2) are not significantly different from the corresponding reference value(s) obtained in one or more individual(s) not suffering from an ASD (in particular healthy individuals).

Notably, reference normal ranges or average values of binocular motricity parameter(s) may be used in step d2). In this case, it may be concluded to:

- absence of features of an ASD (in particular autism) in the individual if all binocular motricity parameters calculated in step b2) are less than 20% above or below the corresponding reference average value in healthy individuals, and
- presence of features of an ASD (in particular autism) in the individual if at least one binocular motricity parameter calculated in step b2) is at least 20% above or below the corresponding reference average value in healthy individuals, and/or if the binocular motricity parameter(s) calculated in step b2) are not significantly different from the corresponding reference value(s) obtained in one or more individual(s) suffering from an ASD (in particular autism).

While a conclusion may be drawn based on direct comparison, one by one, of the value(s) of binocular motricity parameter(s) calculated in step b2) with corresponding reference values obtained when submitting one or more individual(s) not suffering from a learning disorder or ASD (preferably healthy individuals) to the same free exploration of one or several pictorial artwork(s) (preferably two-dimensional pictorial artwork(s) positioned in a vertical plane) or with reference values obtained when submitting one or more individual(s) suffering from a learning disorder or an ASD to the same free exploration of one or several pictorial artwork(s) (preferably two-dimensional pictorial artwork(s) positioned in a vertical plane), as disclosed above, it is also possible to use an algorithm (also referred to as a classifier) that will classify the individual as presenting features or not presenting features of a learning disorder or an ASD based on the value(s) of binocular motricity parameter(s) calculated in step b2).

The classifier has been previously trained based on the value(s) of binocular motricity parameter(s) calculated in step b2) obtained from a cohort comprising both individuals suffering from a learning disorder (or an ASD) and individuals not suffering from a learning disorder (preferably healthy individuals, see Example 3 below for the detection of features of a learning disorder).

Any suitable classifier may be used, including both linear and non-linear classifiers. Examples of classifiers that maybe used include logistic regression, Support Vector Machine (SVM), Ensembing, GaussianProcessClassifier, random-forest, KNeighborsClassifier, AdaBoost, BernoulliNB, Linear discriminant analysis, GaussianNB, Decision Tree, Quadratic discriminant analysis and Multilayer perceptron.

Preferred classifiers include logistic regression and Support Vector Machine (SVM) (as they were found able to classify individuals suffering and not suffering from a learning disorder in Example 3 below).

***Optional steps e2 to g2)***

The method for detecting features of a learning disorder (preferably dyslexia) or an ASD (preferably autism) according to the invention may further comprise:

e2) providing previously obtained recordings of the eye movements of the left eye and the right eye of the individual during one or more other ocular motor test(s), preferably selected from a reading test, a vergence test and a saccade test;

f2) calculating, using a digital processing device, the value(s) of at least one binocular motricity parameter from the records provided in step e2); and

g2) comparing the value(s) calculated in step f2) to one or more reference value(s) obtained in one or more reference individual(s);

wherein steps e2) to g2) are performed before step d2), and step d2) consists in concluding to the presence or absence of features of a learning disorder (in particular dyslexia) or an ASD (in particular autism) in the individual based on the comparisons of steps c2) and g2).

Step e2)

Step e2) is based on previously obtained recordings of the eye movements of the left eye and the right eye of the individual during one or more other ocular motor test(s). Said other ocular motor test(s) is(are) preferably selected from a reading test, a vergence test and a saccade test.

For detection of features of a learning disorder (in particular dyslexia), the other ocular motor tests preferably comprise a reading test, a vergence test and a saccade test, as the combination of these tests has been shown to provide particularly useful binocular coordination parameters for functional exploration of ocular motor abnormalities in such individuals (WO2022/074130).

For detection of features of an ASD (in particular autism), the other ocular motor tests are preferably selected from a vergence test and a saccade test, and may notably comprise both a vergence test and a saccade test. Indeed, many epidemiologic studies suggest vergence-accommodation disorders in persons with autism spectrum disorders (Coulter RA, Bade A, Jenewein EC, Tea YC, Mitchell GL. Near-point Findings in Children with Autism Spectrum Disorder and in Typical Peers. Optom Vis Sci. 2021 Apr 1;98(4):384-393. doi: 10.1097/OPX.0000000000001679. PMID: 33852554; PMCID: PMC8051934; Svend Erik Mouridsen, Bente Rich & Torben Isager (2017) Eye Disorders among Adult People Diagnosed with Infantile Autism in Childhood: A Longitudinal Case Control Study, Ophthalmic Epidemiology, 24:5, 332-335; Jolie R. Keemink et al. Eye Movements and Behavioural Responses to Gaze-Contingent Expressive Faces in Typically Developing Infants and Infant Siblings. Autism Res 2021, 14: 973-983).

The recordings have been made using an eye tracker as defined above for the method for measuring binocular motricity parameters according to the invention.

The reading test(s), vergence test(s), saccade test(s) and optional further test(s) to which the individual suffering from a learning disorder has been submitted for obtaining the recordings provided in step e2) have the same general and preferred features and combinations of features as those disclosed above for optional step d1) of the method for measuring binocular motricity parameters in an individual according to the invention.

Step f2)

The binocular motricity parameter(s) calculated in step f2) also have the same general and preferred features and combinations of features as those disclosed above for optional step e1) of the method for measuring binocular motricity parameters in an individual according to the invention.

When present, calculation step f2) may or not be simultaneously performed with calculation step b2).

Step g2)

In order to detect features of a learning disorder (in particular dyslexia) in the individual, the value(s) of binocular motricity parameter(s) calculated in step f2) are compared with corresponding reference values obtained when submitting one or more individual(s) not suffering from a learning disorder (preferably healthy individuals) to the same one or more other ocular motor test(s) (preferably reading, vergence and saccade tests) and/or with reference values obtained when submitting one or more individual(s) suffering from a learning disorder to the same one or more other ocular motor test(s) (preferably reading, vergence and saccade tests).

In particular, reference values of healthy individuals not suffering from a learning disorder and of individuals suffering from a learning disorder (dyslexia) for vergence parameters obtained in a specific vergence test are provided in Table 1 of WO2022/074130, and corresponding reference values for another vergence test may be obtained by submitting a population of individuals suffering from a learning disorder and a population of healthy individuals not suffering from a learning disorder to the vergence test of interest and calculating binocular motricity parameters of interest in the two populations.

Similarly, reference values of healthy individuals not suffering from a learning disorder and of individuals suffering from a learning disorder (dyslexia) for saccade parameters obtained in a specific saccade test are provided in **Table** 2 of WO2022/074130, and corresponding reference values for another saccade test may be obtained by submitting a population of individuals suffering from a learning disorder and a population of healthy individuals not suffering from a learning disorder to the saccade test of interest and calculating binocular motricity parameters of interest in the two populations.

Similarly, reference values of healthy individuals not suffering from a learning disorder and of individuals suffering from a learning disorder (dyslexia) for saccade parameters obtained in a reading test may be obtained by submitting a population of individuals suffering from a learning disorder and a population of healthy individuals not suffering from a learning disorder to the reading test of interest and calculating binocular motricity parameters of interest in the two populations.

In order to detect features of an ASD (in particular autism) in the individual, the value(s) of binocular motricity parameter(s) calculated in step f2) are compared with corresponding reference values obtained when submitting one or more individual(s) not suffering from an ASD (preferably healthy individuals) to the same one or more other ocular motor test(s) (preferably vergence and saccade tests) and/or with reference values obtained when submitting one or more individual(s) suffering from an ASD to the same one or more other ocular motor test(s) (preferably vergence and saccade tests).

Reference values of healthy individuals not suffering from an ASD and of individuals suffering from an ASD (in particular autism) for binocular motricity parameters in a reading, vergence or saccade test or in any other ocular motor test may be obtained by submitting each population to the test of interest and calculating binocular motricity parameters of interest in the two populations.

Modified step d2) when optional steps e2) to g2) are present

When steps e2) to g2) are present, steps e2) to g2) are performed before step d2), and step d2) is modified and consists in concluding to the presence or absence of features of a learning disorder (in particular dyslexia) or an ASD (in particular autism) in the individual based on the comparisons of steps c2) and g2).

Based on the comparisons made in steps c2) and g2), it may be concluded to the presence or absence of features of a learning disorder (in particular dyslexia) or an ASD (in particular autism) in the individual.

In particular, it may be concluded to the presence of features of a learning disorder (in particular dyslexia) in the individual if at least one binocular motricity parameter calculated in steps b2) and f2) is significantly different from the corresponding reference value(s) obtained in one or more individual(s) not suffering from a learning disorder (preferably healthy individuals). Alternatively, it may be concluded to the presence of a features of a learning disorder (in particular dyslexia) in the individual if the binocular motricity parameter(s) calculated in steps b2) and f2) are not significantly different from the corresponding reference value(s) obtained in one or more individual(s) suffering from a learning disorder (in particular dyslexia).

In contrast, it may be concluded to the absence of features of a learning disorder (in particular dyslexia) in the individual if the binocular motricity parameter(s) calculated in step b2) and f2) are not significantly different from the corresponding reference value(s) obtained in one or more individual(s) not suffering from a learning disorder (preferably healthy individuals).

Notably, reference normal ranges or average values of binocular motricity parameter(s) may be used in step d2). In this case, it may be concluded to:

- absence of features of a learning disorder (in particular dyslexia) in the individual if all binocular motricity parameters calculated in steps b2) and f2) are less than 20% above or below the corresponding reference average value in healthy individuals, and
- presence of features of a learning disorder (in particular dyslexia) in the individual if at least one binocular motricity parameter calculated in steps b2) and f2) is at least 20% above or below the corresponding reference average value in healthy individuals, and/or if the binocular motricity parameter(s) calculated in steps b2) and f2) are not significantly different from the corresponding reference value(s) obtained in one or more individual(s) suffering from a learning disorder (in particular dyslexia).

Similarly, it may be concluded to the presence of features of an ASD (in particular autism) in the individual if at least one binocular motricity parameter calculated in steps b2) and f2) is significantly different from the corresponding reference value(s) obtained in one or more individual(s) not suffering from an ASD (preferably healthy individuals). Alternatively, it may be concluded to the presence of a features of an ASD (in particular autism) in the individual if the binocular motricity parameter(s) calculated in steps b2) and f2) are not significantly different from the corresponding reference value(s) obtained in one or more individual(s) suffering from an ASD (in particular autism).

In contrast, it may be concluded to the absence of features of an ASD (in particular autism) in the individual if the binocular motricity parameter(s) calculated in step b2) and f2) are not significantly different from the corresponding reference value(s) obtained in one or more individual(s) not suffering from an ASD (preferably healthy individuals).

Notably, reference normal ranges or average values of binocular motricity parameter(s) may be used in step d2). In this case, it may be concluded to:

- absence of features of an ASD (in particular autism) in the individual if all binocular motricity parameters calculated in steps b2) and f2) are less than 20% above or below the corresponding reference average value in healthy individuals, and

- presence of features of an ASD (in particular autism) in the individual if at least one binocular motricity parameter calculated in steps b2) and f2) is at least 20% above or below the corresponding reference average value in healthy individuals, and/or if the binocular motricity parameter(s) calculated in steps b2) and f2) are not significantly different from the corresponding reference value(s) obtained in one or more individual(s) suffering from an ASD (in particular autism).

While a conclusion may be drawn based on direct comparison, one by one, of the value(s) of binocular motricity parameter(s) calculated in steps b2) and f2) with corresponding reference values obtained when submitting one or more individual(s) not suffering from a learning disorder or an ASD (preferably healthy individuals) to the same free exploration of one or several pictorial artwork(s) (preferably two-dimensional pictorial artwork(s) positioned in a vertical plane) or with reference values obtained when submitting one or more individual(s) suffering from a learning disorder or an ASD to the same free exploration of one or several pictorial artwork(s) (preferably two-dimensional pictorial artwork(s) positioned in a vertical plane), as disclosed above, it is also possible to use an algorithm (also referred to as a classifier) that will classify the individual as presenting or not presenting features of a learning disorder or an ASD based on the value(s) of binocular motricity parameter(s) calculated in steps b2) and f2).

The classifier has been previously trained based on the value(s) of binocular motricity parameter(s) calculated in steps b2) and f2) obtained from a cohort comprising both individuals suffering from a learning disorder (or from an ASD) and individuals not suffering from a learning disorder (preferably healthy individuals, see Example 3 below in the case of dyslexia).

Any suitable classifier may be used, including both linear and non-linear classifiers. Examples of classifiers that maybe used include logistic regression, Support Vector Machine (SVM), Ensembing, GaussianProcessClassifier, random-forest, KNeighborsClassifier, AdaBoost, BernoulliNB, Linear discriminant analysis, GaussianNB, Decision Tree, Quadratic discriminant analysis and Multilayer perceptron.

Preferred classifiers include logistic regression and Support Vector Machine (SVM).

*Individual*

The method for detecting features or a learning disorder (preferably dyslexia) or an ASD (preferably autism) according to the invention is preferably performed for an individual suspected of suffering from a learning disorder (preferably dyslexia) or an ASD (preferably autism), including all those mentioned above in the context of the method for measuring binocular motricity parameters according to the invention.

Depending on the specific learning disorder or ASD, suspicion may have arisen from various symptoms. For instance, in the case of dyslexia, an individual with difficulties when reading may be considered as suspected of suffering from dyslexia. In the case of autism, individuals with difficulties to fixate the eyes of other individuals may be considered as suspected of suffering from autism.

The individual suspected of suffering from a learning disorder (preferably dyslexia) or an ASD (preferably autism) may notably be a child or an adolescent.

**Method for assessing the level of creativity of an individual**

The present invention also relates to a method for assessing the level of creativity of an individual, comprising:

a3) providing previously obtained recordings of the eye movements of the left eye and the right eye of the individual during free exploration of one or several pictorial artwork(s) (preferably two-dimensional pictorial artwork(s) positioned in a vertical plane);
b3) calculating, using a digital processing device, the value(s) of at least one binocular motricity parameter from the records provided in step a3);
c3) comparing the value(s) calculated in step b3) to one or more reference value(s) obtained in one or more reference individual(s); and
d3) concluding to the level of creativity of the individual based on said comparison.

*Steps a3) and b3)*

Step a3) of the method for assessing the level of creativity of an individual according to the invention may be performed using any embodiment described above for step a2) of the method for detecting features of a learning disorder or an ASD according to the invention.

Similarly, step b3) of the method for assessing the level of creativity of an individual according to the invention may be performed using any embodiment described above for step b2) of the method for detecting features of a learning disorder or an ASD according to the invention.

*Step c3)*

In order to assess the level of creativity in the individual, the value(s) of binocular motricity parameter(s) calculated in step b3) are compared with corresponding reference values obtained when submitting one or more individual(s) with high creativity to the same free exploration of one or several pictorial artwork(s) (preferably two-dimensional pictorial artwork(s) positioned in a vertical plane) and/or with reference values obtained when submitting one or more individual(s) with low creativity to the same free exploration of one or several pictorial artwork(s) (preferably two-dimensional pictorial artwork(s) positioned in a vertical plane).

As dyslexic individuals are known to be more creative that the general healthy population, reference values from a dyslexic population (see Examples below) may be used as reference values obtained when submitting one or more individual(s) with high creativity to the same free exploration of one or several pictorial artwork(s) (preferably two-dimensional pictorial artwork(s) positioned in a vertical plane). Alternatively, as autistic individuals are also known to show increased creativity, reference values from an autistic population may be used as reference values obtained when submitting one or more individual(s) with high creativity to the same free exploration of one or several pictorial artwork(s) (preferably two-dimensional pictorial artwork(s) positioned in a vertical plane).

Conversely, a general healthy population, without any selection for highly creative individuals (see Examples below) may be used as reference values obtained when submitting one or more individual(s) with low creativity to the same free exploration of one or several pictorial artwork(s) (preferably two-dimensional pictorial artwork(s) positioned in a vertical plane).

*Step d3)*

Based on the comparison made in step c3), it may be concluded to a high or low level of creativity in the individual.

In particular, it is concluded to a low level of creativity in the individual if at least one binocular motricity parameter calculated in step b3) is significantly different from the corresponding reference value(s) obtained in one or more individual(s) with high creativity.

In contrast, it is concluded to a high level of creativity in the individual if the binocular motricity parameter(s) calculated in step b3) are not significantly different from the corresponding reference value(s) obtained in one or more individual(s) with high creativity.

Notably, reference normal ranges or average values of binocular motricity parameter(s) may be used in step d3). In this case, it may be concluded to:

- a high level of creativity in the individual if all binocular motricity parameters calculated in step b3) are less than 20% above or below the corresponding reference average value in individuals with high creativity, and
- a low level of creativity in the individual if at least one binocular motricity parameter calculated in step b3) is at least 20% above or below the corresponding reference average value in individuals with high creativity.

While a conclusion may be drawn based on direct comparison, one by one, of the value(s) of binocular motricity parameter(s) calculated in step b3) with corresponding reference values obtained when submitting one or more individual(s) individuals with high creativity to the same free exploration of one or several pictorial artwork(s) (preferably two-dimensional pictorial artwork(s) positioned in a vertical plane) or with reference values obtained when submitting one or more individual(s) individuals with low creativity to the same free exploration of one or several pictorial artwork(s) (preferably two-dimensional pictorial artwork(s) positioned in a vertical plane), as disclosed above, it is also possible to use an algorithm (also referred to as a classifier) that will classify the individual as having a high creativity or a low creativity based on the value(s) of binocular motricity parameter(s) calculated in step b3).

The classifier has been previously trained based on the value(s) of binocular motricity parameter(s) calculated in step b3) obtained from a cohort comprising both individuals with high creativity (for instance a population of dyslexic individuals) and individuals with low creativity (for instance a general population of healthy individuals).

Any suitable classifier may be used, including both linear and non-linear classifiers. Examples of classifiers that maybe used include logistic regression, Support Vector Machine (SVM), Ensembing, GaussianProcessClassifier, random-forest, KNeighborsClassifier, AdaBoost, BernoulliNB, Linear discriminant analysis, GaussianNB, Decision Tree, Quadratic discriminant analysis and Multilayer perceptron.

Preferred classifiers include logistic regression and Support Vector Machine (SVM).

### Individual

The method for assessing the level of creativity according to the invention may be used for any individual, either healthy or suffering or suspected to suffer from any disorder, in particular learning disorders and autism spectrum disorders (ASD).

The following examples merely intend to illustrate the present invention.

### EXAMPLES

**Example 1: Significantly different binocular motricity parameters between dyslexic and non-dyslexic individuals during free exploration of pictorial arts creating a visual illusion of movement in depth**

#### *Materials and Methods*

Participants

47 dyslexics (18 female, 29 male; aged 10 - 21; mean age 15.4) and 44 non dyslexics (22 female, 22 male; aged 8 - 20; mean age 14.8) were recruited from schools across Paris. The dyslexic adolescents were recruited from a school in Paris that specialized in dyslexic education and were admitted to the school on the basis of their dyslexia diagnosis. The dyslexic adolescents were given their diagnosis at specialized multidisciplinary centers, at which they conducted extensive testing at the time of diagnosis, including neuropsychologic and phonologic testing. From reviewing school records, 34.0% (16/47) identified their primary problem was visual/reading based, 4.3% (2/47) was auditory, 2.1% (1/47) was writing, and 59.6 (28/47) were mixed or unknown. Many of the dyslexic adolescents had co-morbid conditions: twelve were also diagnosed with dysorthographia, dyscalcula, and/or dyspraxia. 34 had been to an orthoptist or were currently enrolled in orthoptic rehabilitation. All participants had no neurologic or psychiatric abnormalities; non-dyslexic adolescents also had no difficulty in reading, vision, or visual impairment. The investigation adhered to the principles of the Declaration of Helsinki and was approved by our Institutional Human Experimentation Committee (CPP CNRS 18 011). Written, informed consent was obtained from the adolescents and/or their parents after they were given an explanation about the experimental procedure. The tests were conducted by two research assistants, who were trained together using the same material and conducted the experiment together for each measurement.

### Eye movement recording device

Eye movements were recorded binocularly with a head-mounted video-oculography device, Pupil Core, with recording at 200 Hz in binocular vision with an accuracy of 0.60° and precision of 0.02° (Pupil Labs, Berlin).

### Accelerometer

Posture was recorded for thirty seconds per condition using a body fixed sensor, or an accelerometer (Dynaport, MiniMod, McRoberts B.V. The Hague, The Netherlands). The device was placed at L5 on the participant's lower back using a belt. The MiniMod uses a triaxial seismic acceleration sensor (AXXL202, Analogue Devices, Norwood MA, USA). The sensor's full-scale range is +/- 2□. The sampling frequency was set to 100 Hz.

### Postural Parameters

The following parameters were analyzed: normalized area (NA in mm$^2$/s), root mean square of mediolateral body sway (side-to-side distance or RMS of M/L distance in mm), root mean square of anterior-posterior body sway (forward-backward distance or RMS of A/P distance in mm), root mean square of mediolateral velocity (side-to-side velocity or RMS of M/L velocity in mm/s), RMS of anterior-posterior velocity (forward-backward velocity or RMS of A/P velocity in mm/s), and mean power frequency (MPF in Hz). The first three measures describe the distance the body moved, while the last three describe the energy required to stabilize the body (Vernet M., M. A., Kapoula Z. "Postural and Emotional Impact of Carsten Höller's Artwork "Light Corner". In Exploring Transdisciplinarity in Art and Sciences., Kapoula Z., V. E., Renoult J., A. M. e. Eds.; Springer, Cham, 2018; pp 165-175). Body sway with feet placed side-by-side is modulated by two distinct muscle groups: at the ankle for anterior-posterior body sway and at the hip for mediolateral body sway (Gatev, P.; Thomas, S.; Kepple, T.; Hallett, M. Feedforward ankle strategy of balance during quiet stance in adults. J Physiol 1999, 514 ( Pt 3), 915-928; Winter, D. A.; Prince, F.; Frank, J. S.; Powell, C.; Zabjek, K. F. Unified theory regarding A/P and M/L balance in quiet stance. J Neurophysiol 1996, 75 (6), 2334-2343. DOI: 10.1152/jn.1996.75.6.2334 ; Winter, D. A.; Patla, A. E.; Ishac, M.; Gage, W. H. Motor mechanisms of balance during quiet standing. J Electromyogr Kinesiol 2003, 13 (1), 49-56; Day, B. L.; Steiger, M. J.; Thompson, P. D.; Marsden, C. D. Effect of vision and stance width on human body motion when standing: implications for afferent control of lateral sway. J Physiol 1993, 469, 479-499).

### Calibration of the Pupil Labs device

The standard Pupil Labs calibration (Pupil Capture) was applied using a target that was presented at viewing distance of 1m. The subject fixated on the center of the target and moved their head rightward, downward, leftward and upward at their own pace. They then repeated the sequence (Ward, L. M.; Kapoula, Z. Differential diagnosis of vergence and saccade disorders in dyslexia. Sci Rep 2020, 10 (1), 22116. DOI: 10.1038/s41598-020-79089-1).

### Procedure

Participants were asked to stand in front of a laptop that was positioned 40cm away from their eyes. The laptop was positioned so the image would appear in the middle of their vision with the center of the screen located at eye level. Each adolescent was instructed to try to keep their head still and not to move during the testing. Each participant was asked to fixate at a target on the computer screen at the bottom R corner of the screen prior to being shown each painting. Each participant was invited to explore the image for thirty seconds however they wished when the image appeared. They then were shown each painting on a black background sequentially for 30 seconds. The same instructions were given to each participant. Eye movements and posture were recorded during each 30 second session. In between each painting they were given 30 seconds to rest and reset for the next viewing session. They viewed three paintings in a row: Bridget Riley, Blaze 1, 1962; Bridget Riley, Movement in Squares, 1961; Bridget Riley, Cataract 3, 1967. (see Figures 1 A-1C).

### Paintings

Three paintings were chosen as each gave a different direction of implied movement. The first painting, Blaze 1, was chosen as it seemed to give an illusion of circular movement and movement forward in depth (see Figure 1A). The second painting, Movement in Squares, was chosen as it gave an illusion of movement forward in depth and movement from left to right (see Figure 1B). The third painting Cataract 3, was chosen as it gave a sensation of undulating, wave-like movement from left to right (see Figure 1B). It was also the only painting that incorporated color, which gives the viewer a flickering sensation due to the contrasting primary colors that are deliberately placed next to one another. All three paintings were chosen because they manipulated depth perception and caused an illusion of the painting moving either in the anterior or posterior plane.

Subjective evaluation of the paintings

After viewing the paintings, each child was given a brief questionnaire regarding each of the paintings. While they were filling out the survey, they were given the opportunity to review the paintings on the laptop. They were asked to give a brief written description of what they thought of the painting, how they would rate it on a scale of 1 to 10 (1 = you don't like it at all; 10 = you love it) and to rank how much they felt destabilized on a scale of 1 to 10 (1 = not at all; 10 = very destabilized). They were also asked which direction they felt they had moved as they were looking at each painting and were asked which direction they felt the painting had moved as they viewed it.

Data analysis

AIDEAL, a software developed in the IRIS laboratory (see WO2021/228724), was used to analyze data recorded with the Pupil Labs eye tracker. To analyze the eye movements, AIDEAL treated the conjugate signal, i.e. the L+R eye position/2. The saccade was defined as the time points where the peak velocity went above or below 10% of the peak velocity; this corresponded to values above or below 40°/s. AIDEAL defined the average velocity as the ratio of the total amplitude in degrees / time in seconds. The disconjugacy during saccades, or the evaluation of the binocular motricity, was measured as the difference in amplitude between the left and right eye signal. The Difference in drift amplitude in the first 80 or 160 ms of fixation was calculated as the disconjugate drift. Eye movements with blinks or artifacts were automatically discarded by AIDEAL.

Statistical Analysis

The Shapiro-Wilk test was performed for each comparison and all the data was found to be not normally distributed. As such, we performed the non-parametric Mann-Whitney U test for means comparison for eye movements between the dyslexic and non-dyslexic population as they looked at each painting. We then performed the non-parametric Mann-Whitney U test for means comparison for postural parameters between the dyslexic and non-dyslexic population as they looked at each painting. In terms of subjective ratings, we performed the non-parametric Mann-Whitney U test for means comparison of subjective ratings of destabilization, appreciation, and of movement between the dyslexic and non-dyslexic population as they looked at each painting.

In a second analysis, we calculated the Spearman's Rho correlation between each of the postural parameters in viewing each painting and the subjective ratings of appreciation, movement, and destabilization. We also calculated the Spearman's Rho correlation between each of the postural and eye movement parameters in viewing each painting.

For all analyses, the statistical significance was set at $p \leq 0.05$. We did not attempt to correct for multiple comparisons. All analyses were performed using SPSS version 25 (IBM Corp. Released 2017. IBM SPSS Statistics for Windows, Version 25.0. Armonk, NY: IBM Corp.).

***Results***

Eye Movement Differences between Groups by Painting

For all three paintings, dyslexics demonstrated a higher duration, a faster peak velocity, and a higher average velocity in saccades made to the left and right while viewing. (See **Tables 1A-1C**) This oculomotor profile is similar to that that was found in dyslexics while reading and while making saccades to audiovisual targets (Ward, L. M.; Kapoula, Z. Differential diagnosis of vergence and saccade disorders in dyslexia. Sci Rep 2020, 10 (1), 22116. DOI: 10.1038/s41598-020-79089-1; Ward, L. M.; Kapoula, Z. Dyslexics' Fragile Oculomotor Control Is Further Destabilized by Increased Text Difficulty. Brain Sci 2021, 11 (8). DOI: 10.3390/brainsci11080990; WO2022/074130).

While viewing Painting 1, dyslexics demonstrated a higher disconjugacy during rightward saccades (2.6° +/- 3.1° vs 1.4° +/- 0.8°; p = 0.004).

For all three paintings, dyslexics demonstrated a lower amplitude while making saccades to the left. While viewing paintings one and three, they also demonstrated a significantly smaller amplitude. (See **Tables 1A-1C**) When viewing painting two, which had an area of strong visual interest to the right, dyslexics and non-dyslexics did not demonstrate any difference in amplitude in saccades to the right (3.2° +/- SD 0.8° vs 3.3° +/- 0.1°; p = 0.240 respectively).

While viewing Painting 3, dyslexics demonstrated a higher fixation duration (307.1 ms +/- 90.4 ms vs 339.2 ms +/- 97.7 ms; p = 0.046) and a higher disconjugacy (2.9° +/-3.6° vs 1.5° +/- 0.7°; p = 0.014) during leftward saccades.

Please see Figure 2 for an example of one participant's eye movements during the thirty-second trial overlayed over Painting 1.

**Table 1A.** Eye Movements while viewing Painting 1.

|  | Dyslexic | | Non-Dyslexic | | P-Value |
|---|---|---|---|---|---|
|  | Median | SD | Median | SD |  |
| **Left Amplitude (deg)** | **3.2** | **0.9** | **4.1** | **1.0** | **<0.001** |
| **Right Amplitude (deg)** | **3.3** | **0.8** | **4.0** | **1.0** | **0.002** |
| **Left Duration (ms)** | **82.5** | **48.3** | **62.3** | **30.8** | **0.008** |
| **Right Duration (ms)** | **89.0** | **79.2** | **59.9** | **38.6** | **<0.001** |
| **Left Peak Velocity (deg/sec)** | **163.6** | **64.7** | **129.9** | **53.8** | **0.003** |
| **Right Peak Velocity (deg/sec)** | **170.7** | **110.0** | **119.6** | **49.4** | **<0.001** |
| **Left Average Velocity (deg/sec)** | **83.5** | **31.1** | **117.8** | **33.9** | **<0.001** |
| **Right Average Velocity (deg/sec)** | **85.8** | **30.0** | **116.7** | **34.6** | **<0.001** |
| Left Fixation Disconjugacy 80 msec after saccade (deg) | 0.6 | 0.2 | 0.7 | 0.3 | 0.561 |
| Right Fixation Disconjugacy 80 msec after saccade (deg) | 0.6 | 0.3 | 0.6 | 0.3 | 0.781 |
| Left Fixation Disconjugacy 160 msec after saccade (deg) | 1.0 | 0.4 | 0.9 | 0.4 | 0.465 |
| Right Fixation Disconjugacy 160 msec after saccade (deg) | 0.9 | 0.4 | 0.9 | 0.4 | 0.448 |
| Left Disconjugacy During Saccade (deg) | 2.4 | 3.0 | 1.5 | 0.7 | 0.086 |
| **Right Disconjugacy During Saccade (deg)** | **2.6** | **3.1** | **1.4** | **0.8** | **0.004** |
| Left Fixation Duration (msec) | 296.9 | 68.6 | 296.3 | 94.3 | 0.737 |
| Right Fixation duration (msec) | 314.1 | 90.1 | 316.2 | 79.1 | 0.234 |

**Table 1B.** Eye Movements while viewing Painting 2.

|  | Dyslexic | | Non-Dyslexic | | P-Value |
|---|---|---|---|---|---|
|  | Median | SD | Median | SD |  |
| **Left Amplitude (deg)** | **3.2** | **0.7** | **3.6** | **1.1** | **0.038** |
| **Right Amplitude (deg)** | 3.2 | 0.8 | 3.3 | 1.0 | 0.240 |
| **Left Duration (ms)** | **86.8** | **38.0** | **74.6** | **66.9** | **0.002** |
| **Right Duration (ms)** | **87.8** | **50.2** | **64.3** | **40.5** | **0.001** |
| **Left Peak Velocity (deg/sec)** | **186.9** | **86.5** | **130.1** | **59.6** | **<0.001** |
| **Right Peak Velocity (deg/sec)** | **176.4** | **80.0** | **130.2** | **78.1** | **<0.001** |
| **Left Average Velocity (deg/sec)** | **78.0** | **27.2** | **103.5** | **36.5** | **<0.001** |
| **Right Average Velocity (deg/sec)** | **77.2** | **30.0** | **103.1** | **38.8** | **0.001** |
| Left Fixation Disconjugacy 80 msec after saccade (deg) | 0.6 | 0.2 | 0.6 | 0.2 | 0.812 |
| Right Fixation Disconjugacy 80 msec after saccade (deg) | 0.6 | 0.3 | 0.6 | 0.2 | 0.266 |
| Left Fixation Disconjugacy 160 msec after saccade (deg) | 1.0 | 0.4 | 0.9 | 0.3 | 0.525 |
| Right Fixation Disconjugacy 160 msec after saccade (deg) | 1.0 | 0.6 | 0.8 | 0.3 | 0.104 |

(continued)

|  | Dyslexic | | Non-Dyslexic | | P-Value |
|---|---|---|---|---|---|
|  | Median | SD | Median | SD |  |
| Left Disconjugacy During Saccade (deg) | 3.1 | 3.8 | 1.6 | 1.1 | 0.063 |
| **Right Disconjugacy During Saccade (deg)** | 2.7 | 3.3 | 1.7 | 1.7 | 0.129 |
| Left Fixation Duration (msec) | 306.8 | 58.2 | 311.2 | 95.9 | 0.674 |
| Right Fixation duration (msec) | 331.5 | 83.2 | 338.2 | 89.6 | 0.504 |

**Table 1C.** Eye Movements while viewing Painting 3.

|  | Dyslexic | | Non-Dyslexic | | P-Value |
|---|---|---|---|---|---|
|  | Median | SD | Median | SD |  |
| **Left Amplitude (deg)** | **3.5** | **0.9** | **4.2** | **1.1** | **0.001** |
| **Right Amplitude (deg)** | **3.5** | **0.9** | **4.0** | **1.0** | **0.011** |
| **Left Duration (ms)** | **88.5** | **54.7** | **58.1** | **20.9** | **<0.001** |
| **Right Duration (ms)** | **90.3** | **70.0** | **58.5** | **21.3** | **<0.001** |
| **Left Peak Velocity (deg/sec)** | **185.6** | **88.1** | **139.4** | **58.5** | **<0.001** |
| **Right Peak Velocity (deg/sec)** | **188.6** | **80.8** | **135.0** | **56.1** | **<0.001** |
| **Left Average Velocity (deg/sec)** | **92.2** | **38.0** | **132.6** | **40.0** | **<0.001** |
| **Right Average Velocity (deg/sec)** | **91.6** | **33.9** | **128.4** | **37.4** | **<0.001** |
| Left Fixation Disconjugacy 80 msec after saccade (deg) | 0.7 | 0.3 | 0.7 | 0.3 | 0.657 |
| Right Fixation Disconjugacy 80 msec after saccade (deg) | 0.7 | 0.3 | 0.7 | 0.3 | 0.374 |
| Left Fixation Disconjugacy 160 msec after saccade (deg) | 1.1 | 0.5 | 0.9 | 0.3 | 0.520 |
| Right Fixation Disconjugacy 160 msec after saccade (deg) | 1.0 | 0.5 | 1.0 | 0.4 | 0.930 |
| Left Disconjugacy During Saccade (deg) | **2.9** | **3.6** | **1.5** | **0.7** | **0.014** |
| **Right Disconjugacy During Saccade (deg)** | 2.7 | 3.5 | 1.4 | 0.7 | 0.057 |
| Left Fixation Duration (msec) | **307.1** | **90.4** | **339.2** | **97.7** | **0.046** |
| Right Fixation duration (msec) | 312.4 | 79.9 | 343.6 | 91.3 | 0.091 |

Postural Differences between Groups by Painting

There were no significant differences in objective postural parameters measured with the accelerometer device between dyslexic and non-dyslexic children overall and for each individual painting. For all children, for all paintings, there was no difference in objective postural measurements between those who felt some movement and those who felt no movement at all. However, when examining the data for each of the three paintings, there is a trend towards greater velocity and distance moved in the anterior-posterior direction as compared to the mediolateral direction. (see **Figures 3A-3C**).

Subjective Reports of Destabilization and Movement

Dyslexic children reported feeling significantly more destabilized than non-dyslexic children overall when comparing reports of destabilization during all three recording sessions (4.7 +/- 3.1 vs 3.46 +/- 2.8, p <0.001). They also subjectively reported feeling more destabilized for Paintings 1 and 2 (Painting 1 : 4.8 +/- 2.9 vs 3.7 +/- 2.2, p = 0.026 ; Painting 2 : 3.7 +/- 2.9 vs 1.7 +/- 1.8, p = 0.001). There was no significant difference between dyslexic and non-dyslexic children in subjective feeling of destabilization for Painting 3, although dyslexic children tended to feel more destabilized (5.7 +/- 3.1 vs 5.0 +/- 3.2, p = 0.225).

For Painting 1, dyslexic children were more likely to subjectively report that they moved in no direction (14.9% vs 0.0%) as compared to non-dyslexics. Non-dyslexics were again more likely to report moving in multiple directions (53.2% vs 71.7%) p = 0.050.

For Painting 2, although not significant, dyslexic children trend towards subjectively reporting they moved more in the left direction (14.9% vs 4.3%) and in the forward direction (21.3% vs 8.7%), whereas non-dyslexics were more likely to report moving in multiple directions (34.0% vs 54.3%) p = 0.109.

For Painting 3, although not significant, dyslexic children trend towards subjectively reporting they moved more in the left direction (12.8% vs 0.0%) and in the forward direction (12.8% vs 8.7%), whereas non-dyslexics were more likely to report moving in multiple directions (48.9% vs 65.2%) p = 0.184.

For all paintings, there was no difference in the direction children reported the forms moving.

Appreciation by Group by Painting

No significant difference between dyslexic and non-dyslexic children was found in how much they liked the paintings overall or individually per painting, although non-dyslexics tended to report liking the paintings equally or more than dyslexic children (for all paintings and individually per painting). Please see **Figure 4** for a display of subjective appreciation and destabilization by group per painting.

Correlation between Subjective Destabilization and Appreciation by Group

For all children, the more destabilized they reported feeling, the more they liked the artwork. This was consistent for all three paintings combined (p = 0.005). When analyzed by group, the correlation was stronger for non-dyslexic children (p = 0.006) and was not correlated for dyslexics. Therefore, the more non-dyslexic children reported liking the painting, the more they felt destabilized.

Correlation of Objective Data

There was no correlation between postural parameters and eye movement parameters.

***Conclusions***

While viewing all three paintings, dyslexics demonstrated discoordinated eye movements that were similar to those they made while reading or while making saccades or vergence movements to audiovisual targets, indicating that their abnormal eye movements persist while viewing artwork.

However, specific to these paintings, dyslexics demonstrated significantly slower saccades, which were particularly disturbed in paintings that manipulated depth.

From this study, it seems that dyslexics visual system is disturbed when attempting to coordinate the eyes in depth, even while looking at a two-dimensional painting that is designed to create the illusion of depth. Their visual instability in regarding objects in depth may be translated to decreased attention while looking at the pieces. These Op Art paintings may have exacerbated dyslexic's already abnormal visual profile, causing them to feel more destabilized when viewing these pieces as compared to their peers.

In this way, their discoordinated vision is able to translate the message of the artist. Op Art exists as a generator of a perceptual response as opposed to an art object in and of itself; given that dyslexics felt more destabilized via their abnormal visual profile, we hypothesize that they may be more vulnerable to these types of works of art and more generally better receptors of the artistic experience.

Bridget Riley's work focuses on the manipulation of eye movements to produce this optical response. Indeed, the subjective response created by an objective, physiologic process is what makes Riley's paintings works of art. While research on dyslexia has focused on deficiencies and inadequacies, exercises such as this one that focuses on subjective interpretation facilitated by different physiology allows us to detect their discoordinated vision while focusing on dyslexics' strengths, such as their increased creativity. We suggest that the abnormalities in eye movements that persist while viewing artwork, while reading, and while viewing three-dimensional targets in everyday life may give dyslexics a different perspective that may make them more creative.

**Example 2: Creativity, eye-movement abnormalities, and aesthetic appreciation during free viewing of Magritte's paintings in dyslexic and non-dyslexic individuals**

*Materials and Methods*

Participants

In total, 47 dyslexics (18 female, 29 male; aged 10 - 21; mean age 15.4) and 44 non dyslexics (22 female, 22 male; aged 8 - 20; mean age 14.8) participated in the study. The dyslexic adolescents attended a school specialized for dyslexic students in Paris. They were accepted to the school on the basis of their dyslexia diagnosis, though they were given their diagnosis based on extensive neuropsychologic and phonologic testing at specialized multidisciplinary centers around France. 34.0% (16/47) identified their primary problem was visual/reading based, 4.3% (2/47) was auditory, 2.1% (1/47) was writing, and 59.6 (28/47) were mixed or unknown. As is common in dyslexia, many dyslexic adolescents also reported co-morbid conditions: twelve were concurrently diagnosed with dysorthographia, dyscalcula, and/or dyspraxia. As is exceedingly common in France, 34 participants had been to orthoptic rehabilitation or had seen an orthoptist. All participants had no neurologic or psychiatric abnormalities; non-dyslexics had no deficiencies in reading, writing, vision, or visual impairment. The investigation adhered to the principles of the Declaration of Helsinki and was approved by our Institutional Human Experimentation Committee (CPP CNRS 18 011). Written, informed consent was obtained from the adolescents and/or their parents after they were given an explanation about the experimental procedure. The tests were conducted by two research assistants, who were trained together using the same material and conducted the experiment together for each measurement.

Eye movement recording device

Eye movements were recorded binocularly with a head-mounted video-oculography device, Pupil Core, with recording at 200 Hz in binocular vision with an accuracy of 0.60° and precision of 0.02° (Pupil Labs, Berlin).

Calibration of the Pupil Labs device

The standard Pupil Labs calibration (Pupil Capture) was applied using a target that was presented at viewing distance of 1m. The subject fixated on the center of the target and moved their head rightward, downward, leftward and upward at their own pace. They then repeated the sequence.

Creativity Assessment

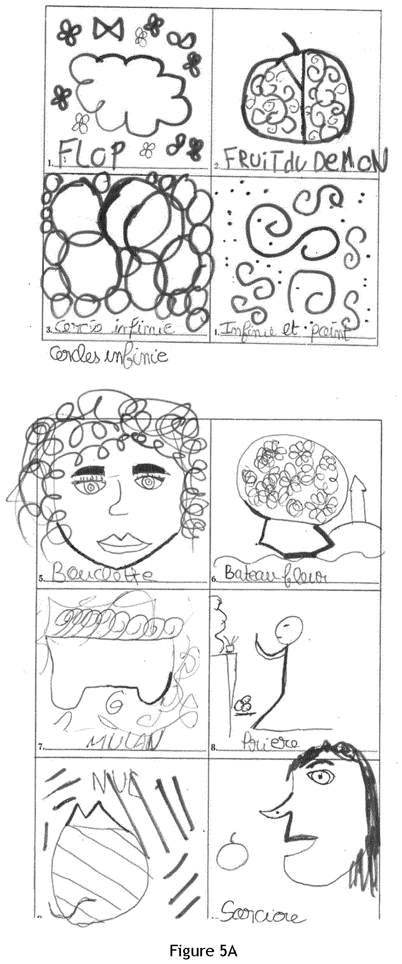

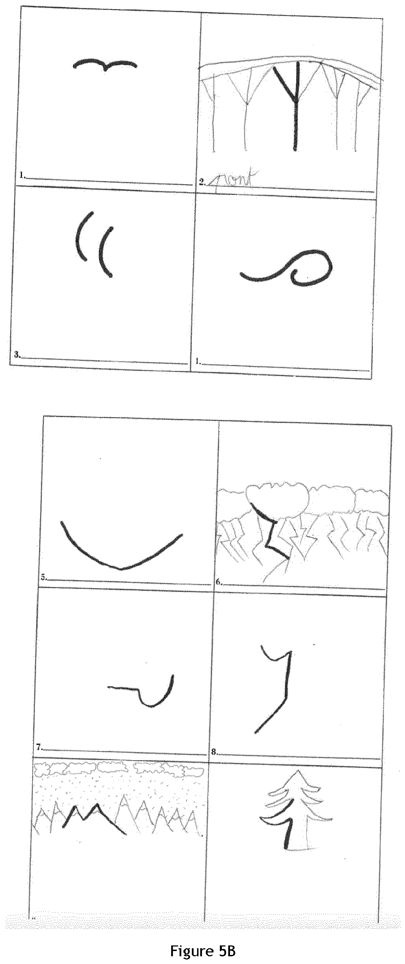

The TTCT-Figural form test is an age-normed test (up to 18 years old).[1] It is a thirty-minute test consisting of three parts of ten minutes each. Each component asks the participant to produce an unusual drawing from standardized shapes that were the same for each participant. (See **Figure 5A-5B**) The TTCT-Figural form is scored to assess four different components of creativity: originality (how uncommon each drawing is); fluency (the number of relevant drawings produced); elaboration (how enriched the drawings are); and flexibility (the number of different ideas created by the drawings). Each 10-minute component was scored, which was then converted into a standard score with a standardized TTCT chart. Each participant was administered the TTCT-Figural form in a quiet room. Each participant was instructed according to the TTCT protocol exactly as it is written. The test was conducted with paper and pencil. The results were scored by the investigators who were trained in the analysis of the Torrance test. The investigators were blinded to their classification as being dyslexic or non-dyslexic.

Procedure

The same instructions were given for each participant. Participants were asked to stand in front of a laptop that was positioned so that the center would be 40cm away from their eyes at eye-level. The image was positioned to be in the middle of their vision with the center of the screen at eye level. Each participant was instructed to keep their head still and to not move their body during testing. They were then asked to fixate on a target at the bottom right corner of the computer screen prior to being shown each painting. Each participant was then invited to explore the image as they wished for thirty seconds when the image appeared. They were then shown each image on a black background sequentially for 30 seconds. In between each painting they were given 30 seconds to rest prior to the next viewing session. They viewed three paintings in a row by René Magritte: La trahison des images (1929); La condition humaine (1933); and L'art de la conversation (1950).

Paintings

Three paintings by René Magritte were chosen due to their complex representation of meaning and physical space.

The first painting, La Trahison des Images, 1929, was chosen because, at first glance, it appears to demonstrates a direct contradiction between image and message **(Figure 1E)**. While the painting depicts a pipe, it also depicts a sentence underneath the pipe that reads "Ceci n'est pas une pipe [This is not a pipe]". The adolescents would therefore have to process the apparent contradiction between image and text and interpret the layers of meaning behind the painting.

The second painting, entitled La Condition Humaine, 1933, depicts an easel which displays a painting that directly matches the backyard background it is placed in front of **(Figure 1F).** It is difficult at first to interpret the painting as being separate from the room in which the easel is placed from the backyard the room looks out onto. The challenge for adolescents in viewing this painting was to interpret the easel as holding a painting, which is a two-dimensional surface separate from the three-dimensional space the two-dimensional painting is depicting.

Finally, the third painting, entitled L'Art de la Conversation, 1950, depicts two small figures standing in front of a rock formation **(Figure 1G)**. The rocks form the word *REVE* [dream]. Given their difficulty with reading, we were curious to see if dyslexic adolescents would have difficulty picking out the letters in the two-dimensional complicated space.

Subjective evaluation of the paintings

After viewing the paintings, each participant completed a questionnaire surveying their subjective responses to each of the paintings. While they were filling out the survey, they could review each painting on the laptop. They were asked how they would rate each painting on a scale from 1 to 10 (1 = you don't like it at all; 10 = you love it) and rate how bizarre they found each painting (1 = not at all; 10 = very bizarre).

Data analysis

AIDEAL, a software developed in the IRIS laboratory (see WO2021/228724), was used to analyze data recorded with the Pupil Labs eye tracker. To analyze the eye movements, AIDEAL treated the conjugate signal, i.e. the L+R eye position/2. The saccade was defined as the time points where the peak velocity went above or below 10% of the peak velocity; this corresponded to values above or below 40°/s. AIDEAL defined the average velocity as the ratio of the total amplitude in degrees / time in seconds. The disconjugacy during saccades, or the evaluation of the binocular motricity, was measured as the difference in amplitude between the left and right eye signal. The difference in drift amplitude in the first 80 or 160 ms of fixation was calculated as the disconjugate drift. Eye movements with blinks or artifacts were automatically discarded by AIDEAL.

Statistical Analysis

The Shapiro-Wilk test was performed for each comparison and all the data was found to be not normally distributed. As such, we performed the non-parametric Mann-Whitney U test for means comparison for creativity scores between the dyslexic and non-dyslexic populations.

In a second analysis, given that the data was not normally distributed, we performed the non-parametric Mann-Whitney U test for means comparison for eye movements between the dyslexic and non-dyslexic population as they looked at each painting. In terms of subjective ratings, again, as the data was not normally distributed, we performed the non-parametric Mann-Whitney U test for means comparison of subjective ratings of appreciation and bizarre-ness between the dyslexic and non-dyslexic population as they looked at each painting.

In a third analysis, we calculated the Spearman's Rho correlation between the subjective responses while viewing each painting and the creativity scores in the dyslexic population.

For all analyses, the statistical significance was set at p ≤ 0.05. We did not attempt to correct for multiple comparisons. All analyses were performed using SPSS version 25 (IBM Corp. Released 2017. IBM SPSS Statistics for Windows, Version 25.0. Armonk, NY: IBM Corp.).

***Results***

Creativity Scores

Dyslexics were found to have higher creativity scores in the fluidity (9.68 vs 8.75; p = 0.013) and flexibility (8.26 vs 7.30, p = 0.031) categories. They were not found to be statistically more creative in the other domains as compared to their non-dyslexic peers.

Eye Movement Differences between Groups by Painting

Please see **Figure 3A-3C** for an example of an individual dyslexic's eye movements during the thirty-second trial overlaid over each painting.

For all three paintings, dyslexics demonstrated a higher duration of saccades, a faster peak velocity, and a slower average velocity in saccades made to the left and right while viewing (see **Tables 2A-2C).** This oculomotor profile is similar to that found in dyslexics while reading and while making saccades to audiovisual targets (Ward, L. M.; Kapoula, Z. Differential diagnosis of vergence and saccade disorders in dyslexia. Sci Rep 2020, 10 (1), 22116. DOI: 10.1038/s41598-020-79089-1; Ward, L. M.; Kapoula, Z. Dyslexics' Fragile Oculomotor Control Is Further Destabilized by Increased Text Difficulty. Brain Sci 2021, 11 (8). DOI: 10.3390/brainsci11080990; WO2022/074130), as well as while viewing op-art artworks (see Example 1). They also demonstrated a higher disconjugacy during saccades to the right and left while viewing all paintings. Finally, dyslexics demonstrated a lower amplitude during right and left saccades while viewing all paintings, meaning they explore the painting with smaller saccades.

While viewing Painting 3, dyslexics demonstrated a longer fixation disconjugacy after the saccade as compared to non-dyslexics (0.88 +/- 0.46 vs 0.69 +/- 0.33; p = 0.033).

**Table 2A.** Eye Movements while viewing Painting 1 (see **Figure 1E**).

| | Dyslexic | | Non-Dyslexic | | P-Value |
|---|---|---|---|---|---|
| | Median | SD | Median | SD | |
| **Left Amplitude (deg)** | **3.56** | **0.99** | **4.22** | **0.74** | **<0.001** |
| **Right Amplitude (deg)** | **3.45** | **0.94** | **4.04** | **0.74** | **0.004** |
| **Left Duration (ms)** | **84.23** | **45.39** | **68.00** | **38.50** | **0.004** |
| **Right Duration (ms)** | **86.67** | **62.56** | **61.10** | **32.06** | **<0.001** |
| **Left Peak Velocity (deg/sec)** | **188.86** | **74.96** | **146.41** | **59.51** | **<0.001** |
| **Right Peak Velocity (deg/sec)** | **173.92** | **77.24** | **121.72** | **48.92** | **<0.001** |
| **Left Average Velocity (deg/sec)** | **87.10** | **36.93** | **124.39** | **35.01** | **<0.001** |
| **Right Average Velocity (deg/sec)** | **81.87** | **31.66** | **121.00** | **32.64** | **0.003** |
| Left Fixation Disconjugacy 80 msec after saccade (deg) | 0.62 | 0.24 | 0.64 | 0.22 | 0.93 |
| Right Fixation Disconjugacy 80 msec after saccade (deg) | 0.58 | 0.21 | 0.55 | 0.18 | 0.37 |
| Left Fixation Disconjugacy 160 msec after saccade (deg) | 0.95 | 0.41 | 0.91 | 0.43 | 0.50 |
| Right Fixation Disconjugacy 160 msec after saccade (deg) | 0.87 | 0.80 | 0.81 | 0.84 | 0.39 |
| **Left Disconjugacy During Saccade (deg)** | **2.76** | **3.20** | **1.61** | **0.84** | **0.037** |
| **Right Disconjugacy During Saccade (deg)** | **2.61** | **3.43** | **1.26** | **0.71** | **0.002** |
| Left Fixation Duration (msec) | 288.17 | 75.54 | 315.51 | 92.60 | 0.20 |
| Right Fixation duration (msec) | 286.45 | 72.00 | 307.69 | 79.29 | 0.19 |

**Table 2B.** Eye Movements while viewing Painting 2 (see **Figure 1F).**

| | Dyslexic | | Non-Dyslexic | | P-Value |
|---|---|---|---|---|---|
| | Median | SD | Median | SD | |
| **Left Amplitude (deg)** | **2.84** | **0.76** | **3.33** | **0.90** | **0.010** |
| **Right Amplitude (deg)** | **2.83** | **0.75** | **3.25** | **0.90** | **0.03** |
| **Left Duration (ms)** | **101.23** | **67.97** | **69.83** | **45.73** | **0.001** |
| **Right Duration (ms)** | **98.70** | **98.83** | **67.93** | **38.26** | **0.006** |
| **Left Peak Velocity (deg/sec)** | **172.90** | **108.43** | **119.17** | **56.25** | **<0.001** |
| **Right Peak Velocity (deg/sec)** | **165.01** | **77.30** | **124.71** | **82.54** | **<0.001** |

(continued)

|  | Dyslexic | | Non-Dyslexic | | P-Value |
|---|---|---|---|---|---|
|  | Median | SD | Median | SD |  |
| **Left Average Velocity (deg/sec)** | **64.93** | **33.41** | **93.85** | **34.93** | **<0.001** |
| **Right Average Velocity (deg/sec)** | **67.97** | **27.76** | **94.32** | **32.13** | **<0.001** |
| **Left Fixation Disconjugacy 80 msec after saccade (deg)** | **0.65** | **0.40** | **0.55** | **0.20** | **0.22** |
| **Right Fixation Disconjugacy 80 msec after saccade (deg)** | **0.64** | **0.47** | **0.57** | **0.27** | **0.39** |
| Left Fixation Disconjugacy 160 msec after saccade (deg) | 0.97 | 0.70 | 0.75 | 0.29 | 0.15 |
| Right Fixation Disconjugacy 160 msec after saccade (deg) | 1.03 | 0.93 | 0.79 | 0.35 | 0.11 |
| **Left Disconjugacy During Saccade (deg)** | **2.66** | **2.79** | **1.31** | **0.72** | **0.001** |
| **Right Disconjugacy During Saccade (deg)** | **2.44** | **2.40** | **1.41** | **1.03** | **0.003** |
| Left Fixation Duration (msec) | 320.76 | 89.34 | 289.87 | 90.04 | 0.11 |
| Right Fixation duration (msec) | 332.38 | 70.14 | 308.22 | 114.63 | 0.41 |

**Table 2C.** Eye Movements while viewing Painting 3 (See **Figure 1G).**

|  | Dyslexic | | Non-Dyslexic | | P-Value |
|---|---|---|---|---|---|
|  | Median | SD | Median | SD |  |
| **Left Amplitude (deg)** | **3.22** | **0.78** | **3.78** | **0.74** | **<0.001** |
| **Right Amplitude (deg)** | **3.10** | **0.77** | **3.78** | **0.70** | **<0.001** |
| **Left Duration (ms)** | **85.27** | **61.07** | **57.42** | **29.36** | **<0.001** |
| **Right Duration (ms)** | **75.98** | **33.07** | **56.03** | **31.07** | **<0.001** |
| **Left Peak Velocity (deg/sec)** | **159.41** | **75.45** | **114.94** | **49.11** | **<0.001** |
| **Right Peak Velocity (deg/sec)** | **147.49** | **72.37** | **107.43** | **50.60** | **<0.001** |
| **Left Average Velocity (deg/sec)** | **77.52** | **28.21** | **107.24** | **29.89** | **<0.001** |
| **Right Average Velocity (deg/sec)** | **75.58** | **27.16** | **103.35** | **28.99** | **<0.001** |
| Left Fixation Disconjugacy 80 msec after saccade (deg) | 0.62 | 0.31 | 0.55 | 0.18 | 0.65 |
| Right Fixation Disconjugacy 80 msec after saccade (deg) | 0.54 | 0.20 | 0.50 | 0.23 | 0.20 |
| Left Fixation Disconjugacy 160 msec after saccade (deg) | 0.93 | 0.55 | 0.77 | 0.29 | 0.34 |
| **Right Fixation Disconjugacy 160 msec after saccade (deg)** | **0.88** | **0.46** | **0.69** | **0.33** | **0.033** |
| **Left Disconjugacy During Saccade (deg)** | **2.51** | **2.93** | **1.32** | **0.83** | **0.005** |
| **Right Disconjugacy During Saccade (deg)** | **2.33** | **2.74** | **1.20** | **0.74** | **0.012** |
| Left Fixation Duration (msec) | 317.29 | 72.50 | 337.39 | 77.11 | 0.187 |
| Right Fixation duration (msec) | 336.97 | 61.90 | 345.70 | 65.93 | 0.634 |

Subjective Reports of Appreciation and the Perception of the Bizarre

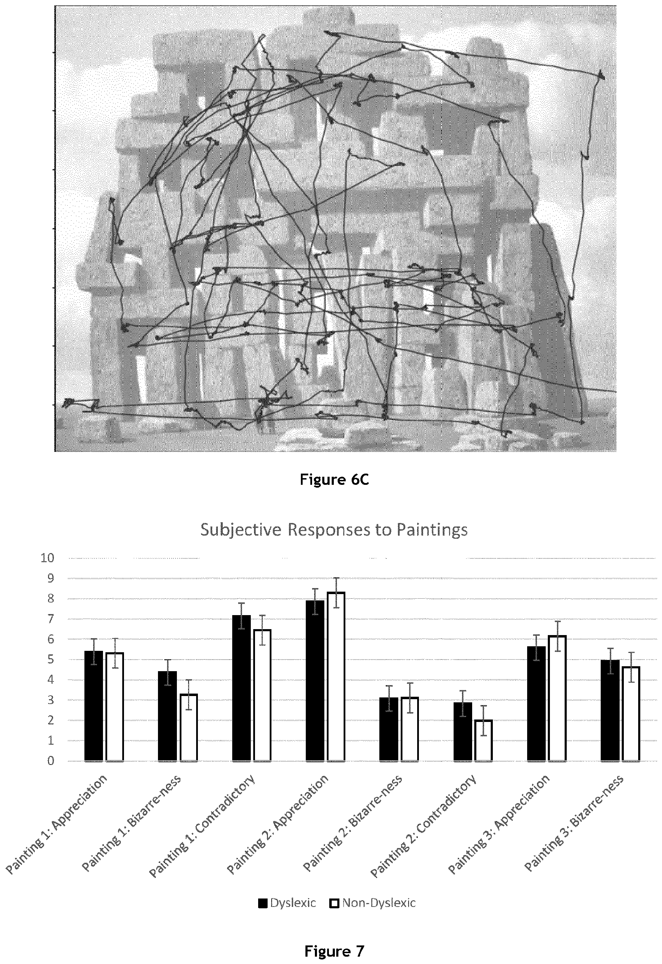

There were no significant differences in subjective ratings of appreciation, bizarre-ness, or how contradictory the paintings were between the two populations (See **Figure 7).**

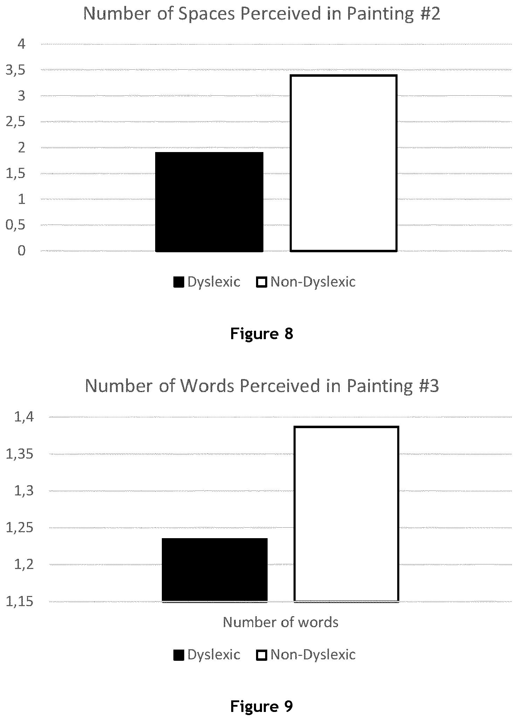

While viewing Painting 2, participants were asked how many spaces they perceived to be represented in the painting. Non-dyslexics reported perceiving significantly more spaces than dyslexics (1.96 +/- 1.33 vs 3.44 +/- 4.17; p = 0.003, see also **Figure 8).**

While viewing Painting 3, participants were asked how many words they recognized in the painting. Non-dyslexics recognized significantly more words as compared to dyslexic participants (1.23 +/- 1.13 vs 1.38 +/- 0.73; p = 0.043, see also **Figure 9).**

Correlation between Appreciation and the Perception of the Bizarre

For both populations, the more the adolescents found the paintings to be contradictory, the less they appreciated the artwork (dyslexic: Correlation co-efficient - 0.247, p < 0.17; non-dyslexic: Correlation co-efficient -0.410; p < 0.001).

*Discussion*

In terms of creativity, in our population, dyslexics were found to be more creative than non-dyslexics in terms of fluidity and flexibility. Fluidity is the measurement of the number of relevant ideas an adolescent produced in response to a single prompt. Flexibility is the ability to produce a wide range of ideas to a particular stimulus. Given that dyslexics have eye movements that are different from their peers, it is possible that this unusual way of moving their eyes across the image has allowed them to be more adaptable and therefore produce more, varied ideas to one particular stimulus, resulting in an increased creativity via fluidity and flexibility.

From an eye movement perspective, once again, dyslexics demonstrated an abnormal pattern of eye movement as they viewed all three paintings as compared to their non-dyslexic peers. This uncoordinated eye movement pattern, represented by a higher saccade disconjugacy, higher duration (slower saccades), a slower average velocity despite a faster velocity, was similar to those they produced while looking at Op Art (see Example 1), while viewing audiovisual targets, and while reading (see Ward, L. M.; Kapoula, Z. Differential diagnosis of vergence and saccade disorders in dyslexia. Sci Rep 2020, 10 (1), 22116. DOI: 10.1038/s41598-020-79089-1; Ward, L. M.; Kapoula, Z. Dyslexics' Fragile Oculomotor Control Is Further Destabilized by Increased Text Difficulty. Brain Sci 2021, 11 (8). DOI: 10.3390/brainsci11080990; WO2022/074130). These eye movements appear to be the same no matter what the task is. Even in these less regimented, playful conditions like regarding paintings, there are eye movement differences which persist despite the task, which could represent a physiologic oculomotor signature of dyslexia. This further consolidates evidence for fundamental differences in eye movements between dyslexics and non-dyslexics.

Despite their differences in creativity and in eye movement profile, dyslexics and non-dyslexics did not subjectively perceive the paintings differently in terms of appreciation, how bizarre and how contradictory the pieces were. Interestingly, however, there were some significant differences in perception of space and words. While viewing Painting 2, dyslexics reported perceiving significantly fewer spaces in the painting as compared to their peers. As discussed, it has been previously shown that dyslexics may have difficulty with depth perception, both while viewing targets in space, while reading, and while viewing two-dimensional representations of the illusion of depth in Op Art paintings. This difficulty with depth perception is again represented while viewing this trompe l'oeil image in which multiple spaces are depicted in depth. We postulate that dyslexics' difficulty to interpret this two-dimensional representation of a complex three-dimensional space may again be related to their difficulty coordinating their eyes in depth. This representation is slightly different from previous descriptions of dyslexics' interaction with space in that it requires the adolescent to recognize layers of conceptual space. Whereas our previous research has focused on examining eye movements while dyslexics view objects in true three-dimensional space or during the *illusion* of three-dimensional space, Magritte's paintings force the viewer to confront a conceptual space represented by a painting in a painting that is difficult to discern from the background of the painting. Conceptual space perception is different from the illusion of depth that is triggered by op art, which targets lower eye movement processing, in that it is perhaps a more complex, subtle test. It is interesting that this study demonstrates that dyslexic's abnormal oculomotor profile persists despite this difference in depth perception, suggesting a possible a physiologic oculomotor signature of dyslexia independent of the particular stimulus.

Dyslexics also reported they recognized significantly fewer words while viewing Painting 3. Painting 3 has one word represented "REVE", which means "dream" in French. Despite there only being one word, non-dyslexic adolescents reported recognizing more than one word more frequently than non-dyslexics; and dyslexics reported not recognizing any words more frequently than non-dyslexics. This could be due to dyslexic's well-established difficulty with reading.

*Conclusions*

Here, dyslexics adolescents were demonstrated to be more creative in terms of flexibility and fluidity than their non-dyslexic peers. This represents a difference from previous studies, which have found dyslexic children to be more creative in all domains. Educational upbringings and age may affect adolescent creativity scores.

Dyslexics also perceived the paintings differently from non-dyslexics. Indeed, we found that dyslexics perceived fewer conceptual spaces and fewer words than their non-dyslexic peers. This may be due to their difficulties with depth and space perception, as well as their well-documented difficulties in reading.

Dyslexics also demonstrated an abnormal oculomotor profile, in that they demonstrated difficulty with disconjugation and abnormalities in their velocity profile, which as sustained throughout all viewing conditions. Indeed, dyslexics demonstrated an abnormal pattern of eye movement as they viewed all three paintings as compared to their non-dyslexic peers. This uncoordinated eye movement pattern, represented by a higher saccade disconjugacy, higher duration (slower saccades), a slower average velocity despite a faster velocity, was similar to those they produced while looking at Op Art (see Example 1), while viewing audiovisual targets, and while reading (see Ward, L. M.; Kapoula, Z. Differential diagnosis of vergence and saccade disorders in dyslexia. Sci Rep 2020, 10 (1), 22116. DOI: 10.1038/s41598-020-79089-1; Ward, L. M.; Kapoula, Z. Dyslexics' Fragile Oculomotor Control Is Further Destabilized by Increased Text Difficulty. Brain Sci 2021, 11 (8). DOI: 10.3390/brainsci11080990; WO2022/074130). These eye movements appear to be the same no matter what the task is. Even in these less regimented, playful conditions like regarding paintings, there are eye movement differences which persist despite the task, which could represent a physiologic oculomotor signature of dyslexia. This further consolidates evidence for fundamental differences in eye movements between dyslexics and non-dyslexics.

Our analysis is restricted to the total viewing period. Further analysis which is currently ongoing is necessary to explore potential physiologic differences while dyslexics view more complex parts of the painting.

**Example 3: Predicting Dyslexia in Adolescents from Eye Movements during free painting viewing**

*Materials and Methods*

Participants

The population studied is described in Example 1.

In brief, eye movements were recorded during painting exploration in 46 dyslexic adolescents (18 female, 28 male; mean age 15.52, SD 2.45) and 41 non-dyslexic adolescents (20 female, 21 male; mean age 14.78 +/- 2.44) recruited from schools in Paris. Dyslexic adolescents were diagnosed in specialized medical centers and were admitted to their schools based on their dyslexia diagnosis.

Typically, diagnosis by these centers includes multiple testing which confers a diagnosis of visual, phonological dyslexia, mixed dyslexia, etc. We, therefore, did not have children self-identify their condition. Both dyslexic and non-dyslexic adolescents had no known neurologic or psychiatric abnormalities. Typical readers had no history of reading difficulty, no visual impairment, or difficulty with near vision. Of dyslexics, 34.0% (16/47) identified that their primary issue was visual/reading based, 4.3% (2/47) auditory, 2.1% (1/47) writing, and 59.6 (28/47) were mixed or unknown.

The investigation adhered to the principles of the Declaration of Helsinki and was approved by our Institutional Human Experimentation Committee (CPP CNRS 18 011). Written consent was obtained from the adolescents and/or their parents after they were explained the experimental procedure. The tests were conducted by two research assistants, who were trained together using the same material and conducted the experiment together for each measurement.

Eye Movement Recording Device

For each adolescent, eye movements were recorded binocularly with a head-mounted video-oculography device, Pupil Core [Pupila Capture Eye Tracker. Available online: https://pupil-labs.com/ (accessed on 01 12 2021)], enabling binocular recording at 200 Hz per eye (Pupil Labs, Berlin, Germany).

Subsequently, they were analyzed with AIDEAL, (see WO2021/228724). For saccade analysis, AIDEAL treated the conjugate signal, e.g., the L + R eye position/2. The onset and the offset of the saccade were defined as the time points where the peak velocity went above or below 10% of the peak velocity; practically, this corresponded to values above or below 40°/s (as the peak velocity of 20° saccades is typically above 40°/s). The total average velocity was defined as the ratio of total amplitude in degrees divided by time in seconds.

To evaluate binocular motricity of saccades, or the disconjugacy during saccadic movements, the difference in amplitude between the left and the right eye signal was calculated. The disconjugate drift, or the difference in drift amplitude during the first 80 or 160 ms of fixation, was calculated

Ocular Motor Tests

*Painting tests*

The data concern eye movements while viewing modern art painting selected because of their specific visual psychophysics attributes: three Op Art paintings from Bridget Riley, providing a strong illusion of visual motion-in-depth, and on the other hand, four paintings from Escher or Magritte with the high complexity of space construction, e.g. bistable spaces or image text contradictions. The reason for choosing such paintings was the hypothesis that dyslexics known to show more unstable fixations in-depth (diverging or converging post-saccadic drifts) would be perceptually more sensitive to the illusion of movement in depth as fixation instability could contribute to the genesis of such illusion. On the other hand, dyslexics would have a higher capacity in analyzing complex space compositions, or contradictory images Dyslexic and non-dyslexic teenagers viewed images of each painting for 30 seconds each in standing position in front of a screen, while their eye movements were recorded (with PupiLabs, core device).

Adolescents were asked to stand in front of a laptop that was positioned 40cm away from their eyes. The laptop was positioned so the image would appear in the middle of their vision. Each adolescent was instructed to try to keep their head still and not to move during the testing. Each participant was asked to fixate at a target on the computer screen at the bottom right corner of the screen before being shown each painting. They then were shown each painting on a black background sequentially for 30 seconds. Eye movements were recorded during each 30-second session. In between each painting they were given 30 seconds to rest and reset for the next viewing session. They viewed seven paintings in a row: Bridget Riley, Blaze 1, 1962 (Painting 1, see **Figure 1A);** Bridget Riley, Movement in Squares, 1961 (Painting 2, see **Figure 1B);** Bridget Riley, Cataract 3, 1967 (Painting 3, see **Figure 1C);** M. C. Escher, Relativité, 1953 (Painting 4, see **Figure 1D);** René Magritte, La condition humaine I, 1935 (Painting 5, see **Figure 1F);** René Magritte, The Art of Conversation-Color Variation, 1950 (Painting 6, see **Figure 1G);** and René Magritte, La trahison des images, 1973 (Painting 7, see **Figure 1E).**

### *Pre-processing and feature extraction*

Eye Movement Descriptors based on AIDEAL velocity threshold method

For each individual and each test, and each of the above-cited features, the AIDEAL software (see WO2021/228724) provides the mean value (based on 10 to 20 trials), the standard deviation, the coefficient of the variation (standard deviation/mean $\times$ 100), and the total number of trials used for each descriptor. The eye movement features computed are:

Amplitude: the amplitude of the movement (in degrees), i.e., the change in eye position between the onset and the offset of the eye movement; the onset and offset of eye movement being determined by velocity threshold criteria as described above using AIDEAL software.

Duration of the phasic component: the time of execution of the movement (in ms), i.e., the time between the onset of the movement and its offset.

P-Velocity: Peak velocity of the eye movement (in degrees per sec); the peak velocity is usually achieved at the first 3rd of the trajectory of the movement.

A-velocity: Average velocity, which is obtained via the ratio of the amplitude of the movement (in degrees) over its duration (in seconds).

Drift 1: is the amplitude (in degrees) of disconjugate drift of the eyes (i.e., subtraction amplitude of the drift of the right eye from that of the left eye) for the 80 ms period starting from the offset of the movement.

Drift 2: Is the amplitude of the disconjugate drift during the 160 ms period following the offset of the movement (in degrees).

Total Amplitude: The addition of the phasic amplitude and the vergence drift during the 160 msec.

Fixation duration: the period (in ms) between two successive fixations.

We considered that outlier values from AIDEAL do not occur randomly and thus convey information on the recorder gaze. For example, aberrant values could be due to blinks or artifacts of eye movement recording apparatus. We, therefore, chose to give these aberrances a value of 0 instead of the population average. Finally, we applied the procedure described in the next section to compute the disconjugacy index.

Disconjugacy global index - new analysis (DGI)

**Figure 10** illustrates the nature of the selected segment by our algorithm. The light grey curve represents the right eye movement coordinate over the disconjugacy axis, while the dark grey curve represents the left eye movement coordinate over the same axis.

The DGI score is a measure of the frequency of the occurrence of the disconjugate segments. A disconjugacy segment is defined as a time interval where two eyes are moving in opposite directions (converging or diverging, as is done when changing depth to look at near vs far objects). These segments are detected based on the velocity and position of eye movement signals of the two eyes continuously, i.e. the entire time series of signals as opposed to the AiDEAL approach that considers only the movement (saccade or vergence) and the periods immediately before and after the movement (a trial).

The segments extracted by our algorithm are visible in the position trace (subfigure (d,e)). our algorithm identifies the segment by searching in the signal of the product of the right and left eye velocity signals; the period during which the product is negative, for example, if the instantaneous velocity of the left eye and right eye is +10 degree/sec and -10 degree/sec respectively, then the product is -100 degree$^2$/sec$^2$ and this signifies a disjunctive period that we select, in contrast, if the instantaneous velocity is +10 degree/sec and +20degree/sec for the left and right eye, respectively, then the product is 200 degree$^2$/sec$^2$ and therefore this segment is not considered in our analysis.

To evaluate the DGI, we counted the total number of the disconjugacy segments, and we divided it by the total duration of the recording. To count the total number of the disconjugacy segments present in a signal, we retrieve the position of the left eye on the horizontal axis over time and the position of the right eye on the horizontal axis over time (1). Then, we computed the disconjugate signal, denoted by D defined as the difference of the left horizontal signal minus the right horizontal signal (2). Also, we computed the derivative of the left signal and the right signals and the product of the two derivatives, denoted by Dv (3). Then, we used the Dv signal to retrieve all the segments where the sign of this signal is negative (4). Those segments are the moments where the two eyes were moving in the opposite direction over the X, horizontal axis, in other words, these are the moments where the two eyes were moving oppositely converging or diverging.

For each Dv segment, we split it into sub-segments whenever the D corresponding block changes its sign (5). It is important to note that considering both the Disconjugate position signal and the product of the velocities of the two eyes enables to identify the periods during which the eyes moving in opposite directions; the D position signal alone does not give this information, as there might be instances where the eyes traveling in the same direction but by different amounts. Consequently, this new analysis is more focused on identifying continuously all the instantaneous segments during which the eyes are traveling in opposite directions (therefore we prefer to call this index a distinctiveness signal rather than disconjugate). Once each segment is identified as either micro-diverging or an elementary micro-converging, we retain all those segments that last for a period of at least 3 samples i.e., 12 msec of time. This decision was based on a prior analysis testing different segment minimal periods and seems physiologically plausible and not just a noise in the eye-tracking signals.

Finally, we counted the total number of the disconjugacy segments, and we divided by the total duration of the recording to make this new descriptor comparable across recordings of different durations (6).

The different steps to compute the DGI score are formally presented in the equation below.

$$X_l = [l_1, l_2, ..., l_n], \ X_r = [r_1, r_2, ..., l_n], \tag{1}$$

$$D = \left[ X_l^{(i)} - X_r^{(i)} \right]_{i=1}^n \tag{2}$$

$$DV = \left[ \frac{X_l^{(i+1)} - X_l^{(i)}}{T^{(i+1)} - T^{(i)}} \times \frac{X_r^{(i+1)} - X_r^{(i)}}{T^{(i+1)} - T^{(i)}} \right]_{i=1}^n \tag{3}$$

$$E = \left\{ (s, l) \in [2, n] \times [s+3, n-1] \mid DV^{(s-1)} > 0 \ \& \ DV^{(l+1)} > 0 \ \& \ \forall i \in [s, l], DV^{(i)} < 0 \right\} \tag{4}$$

$$Count = \sum_{(s,l)}^E card(\left\{ (a, b) \in [s, n] \times ]a, l] \mid D^{(a-1)} \neq D^{(a)} \ \& \ D^{(b+1)} \neq D^{(b)} \ \& \ \forall i \in [a, b], D^{(i+1)} = D^{(i)} \right\}) \tag{5}$$

$$DGI = \frac{Count}{T^{(n)} - T^{(i)}} \tag{6}$$

In addition to the DGI score, we've considered two other features: the mean amplitude and the mean duration over the different retrieved segments. Physiologically, it is important to know whether the amplitude and duration of such segments are different for the two populations; moreover, such additional features might increase the generalization ability.

*Model Fitting*

We've followed the same procedure presented in [El Hmimdi AE, Ward LM, Palpanas T, Kapoula Z. Predicting Dyslexia and Reading Speed in Adolescents from Eye Movements in Reading and Non-Reading Tasks: A Machine Learning Approach] to train a Support Vector Machine (SVM) with a radial basis function RBF kernel and a Logistic regression model to predict dyslexia based on eye movement descriptors extracted from the 7 paintings Dataset.

In the first analysis, we've considered the 7 datasets separately. In this analysis, for each dataset, all its samples were collected when each subject was observing the same painting. Hence, the different DGI scores were supposed to be easily comparable. On the other side the sample size was low (92 samples for each dataset).

In the second analysis, we've grouped the 7 paintings datasets into two groups. By aggregating the painting 1,2, and 3 datasets into group 1, and painting 4,5,6, and 7 into group 2. Hence, the sample size of each dataset has increased from 92 to 276 and 368 for the first and second dataset, respectively. On the other hand, the variance of the input space has increased.

The paintings datasets were grouped based on their visual psychophysics similarity of the painting table, hence the three datasets of the Op Art paintings from Bridget Riley were grouped into group1, whereas the other four paintings from Escher and Magritt were combined into group 2.

Finally, we've followed the same logic of analysis 2, by combining the seven datasets into one big dataset that contains 92*7 observations, to have a more reliable score of the generalization ability of our models. By merging the 7 datasets into one big dataset, the test size has increased from 20 samples to 140 samples. In this analysis. We've investigated the ability of each feature set (Aideal eye movement feature and the DGI features) to predict dyslexia without having the information of which painting each participant was looking at when eye movements were recorded.

During the three analyses, we used cross-validation (5-fold). At each fold, the model is fitted to the train set, and its generalization ability is evaluated on the test set. We report the average test performance on the five folds. At each fold, the train set, and test set were first normalized by subtracting the mean of the training set then dividing by the standard deviation of the training set. Since the size of the dataset did not allow for the creation of a representative validation set, we do not perform any hyperparameter tuning and use the default values of the different models in their scikit-learn implementation.

To evaluate our ML algorithms, we used 3 metrics: accuracy, sensitivity, and specificity. A more complete version of the procedure is presented in [El Hmimdi AE, Ward LM, Palpanas T, Kapoula Z. Predicting Dyslexia and Reading Speed in Adolescents from Eye Movements in Reading and Non-Reading Tasks: A Machine Learning Approach].

*Results*

Predicting dyslexia using Aideal based features of saccades and fixations

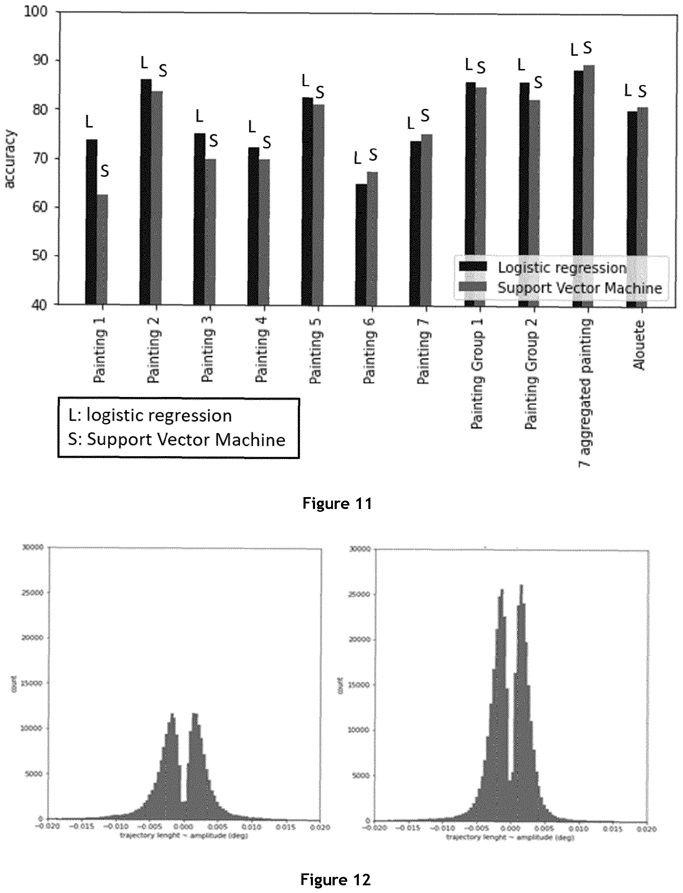

**Figure 11** presents a summary of the accuracy of the two models (Support vector machine and logistic regression) when using Aideal based features to predict dyslexia on the 10 datasets.

In addition, **Tables 3A to 3E** below present the different used metrics scores of the two models when trained on the Aideal Eye movement features on the analysis 1 (Tables 3A and 3B), the analysis 2 (Tables 3C and 3D), and the analysis 3 (Table 3E).

**Table 3A:** Support Vector Machine scores for Aideal based data sets.

| Dataset | accuracy | sensitivity | specificity |
|---|---|---|---|
| Painting 1 | 73.75% | 75% | 72.5% |
| Painting 2 | 86.25% | 82.5% | 90% |
| Painting 3 | 75% | 72.5% | 77.5% |
| Painting 4 | 72.5% | 67.5% | 77.5% |
| Painting 5 | 82.5% | 80% | 85% |
| Painting 6 | 65% | 67.5% | 62.5% |
| Painting 7 | 73.75% | 70% | 77.5% |
| Alouete | 80% | 82.5 | 77.5% |

**Table 3B:** Logistic regression scores for Aideal based data sets.

| Dataset | accuracy | sensitivity | specifici ty |
|---|---|---|---|
| Painting 1 | 62.5% | 65% | 60% |
| Painting 2 | 83.75% | 82.5% | 82.5% |
| Painting 3 | 70% | 85% | 55% |
| Painting 4 | 70% | 72.5% | 67.5% |
| Painting 5 | 81.25% | 77.5% | 85% |
| Painting 6 | 67.5% | 70% | 65% |
| Painting 7 | 75% | 77.5% | 72.5% |
| Alouete | 81% | 85% | 77.5% |

**Table 3C:** Support Vector Machine scores for the 2 grouped painting datasets.

| Dataset | accuracy | sensitivity | specificity |
|---|---|---|---|
| Painting Group 1 | 85.88% | 84.44% | 87.5% |
| Painting Group 2 | 85.88% | 84.44% | 87.5% |

**Table 3D:** Logistic regression scores for the 2 grouped painting datasets.

| Dataset | accuracy | sensitivity | specificity |
|---|---|---|---|
| Painting Group 1 | 84.71% | 84.44% | 85.00% |
| Painting Group 2 | 82.35% | 82.22% | 80.0% |

**Table 3E.** SVM and Logistic regression scores when trained on the 7 paintings dataset using Aideal eye movement features.

| Model | accuracy | sensitivity | specificity |
|---|---|---|---|
| Support Vector Machine | 88.24% | 88.89% | 87.5% |
| Logistic regression | 89.41% | 88.9% | 90.00% |

In the first analysis, when considering painting separately, for all 7 of the painting dataset, Aideal-based eye movement features were able to predict dyslexia. Except for the first dataset (painting 1), there was no significant difference between the performance of the linear model and the non-linear model.

The best performances were achieved when trained on the painting 2 dataset, with nonlinear SVM, with an accuracy of 86.25%, a sensitivity of 82.5%, and a specificity of 90%. On the other hand, the worst model in terms of the generalization ability was the logistic regression when trained on the painting 1 dataset.

In the second and the third analysis, where we've grouped multiple datasets to augment the sample size of each dataset. The accuracy has improved to 85.88% using the SVM model.

Finally, the best performance was achieved when merging all the 7 datasets, without considering the nature of the painting that was observed during the dataset acquisition process. This improvement can also be explained by the sample size that has been multiplied by 7 when considering combining all the 7 painting datasets.

Predicting dyslexia using DGI of the continuous eye movement signal

*Different DGI distributions between dyslexic and non-dyslexics population*

Before adding the DGI set of features to our ML experimental setup, we conducted some exploratory data analysis, to gain more intuition into the distributions of the three handcrafted features, namely, the DGI, the mean duration, and the mean amplitude and their ability to predict dyslexia. **Figure 12** presents the histogram of the retrieved segments from the 7 painting datasets combined for the dyslexic population and non-dyslexic population.

The left histogram corresponds to the non-dyslexic population, while the right histogram corresponds to the dyslexic population. The two histograms were computed using 100 bins, showing a statistical distribution with two main modes that correspond to the peak of micro convergence and micro divergence amplitude segments.

Most DGI segments have an amplitude less than 0.005°. For each of the 100 bins, its count for the dyslexic population is much higher than for the non-dyslexic population when considering the bins with an amplitude less than 0.005° and is approximately equal to the non-dyslexic population when considering the bins with an amplitude bigger than 0.005°.

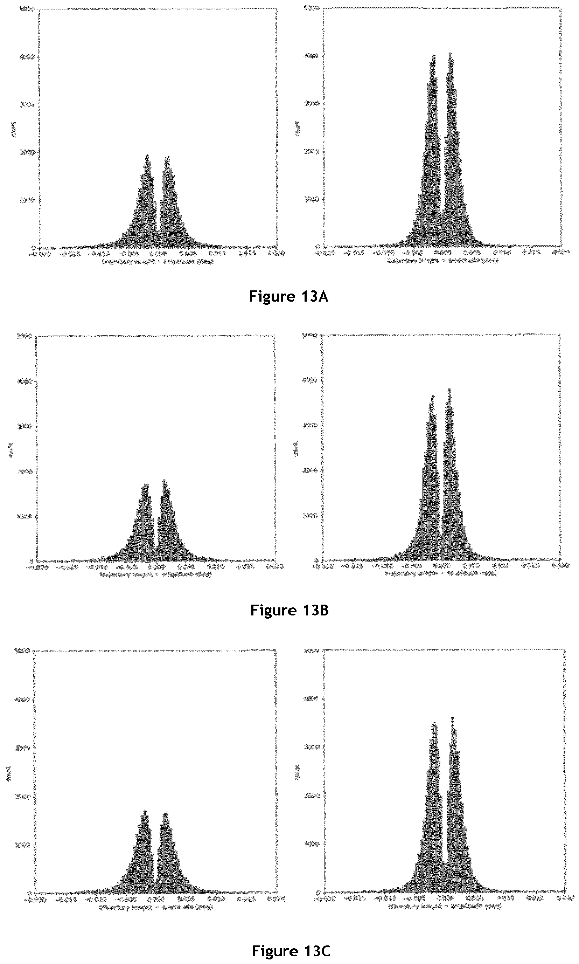

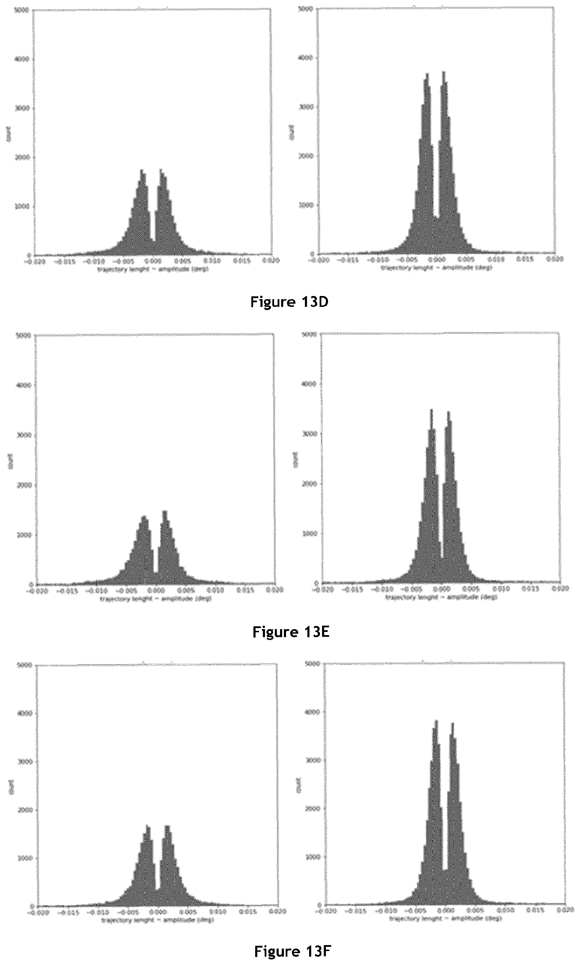

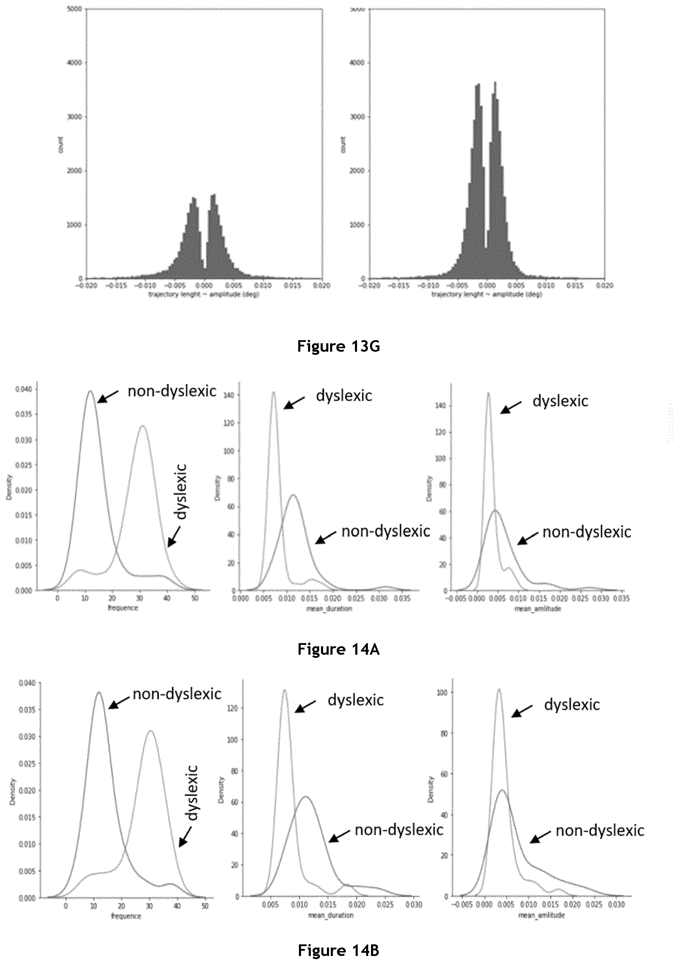

Also, **Figures 13A to 13G** present the histogram of the retrieved segments when considering the 7 paintings dataset separately. By analyzing the painting dataset separately, we've found the same pattern.

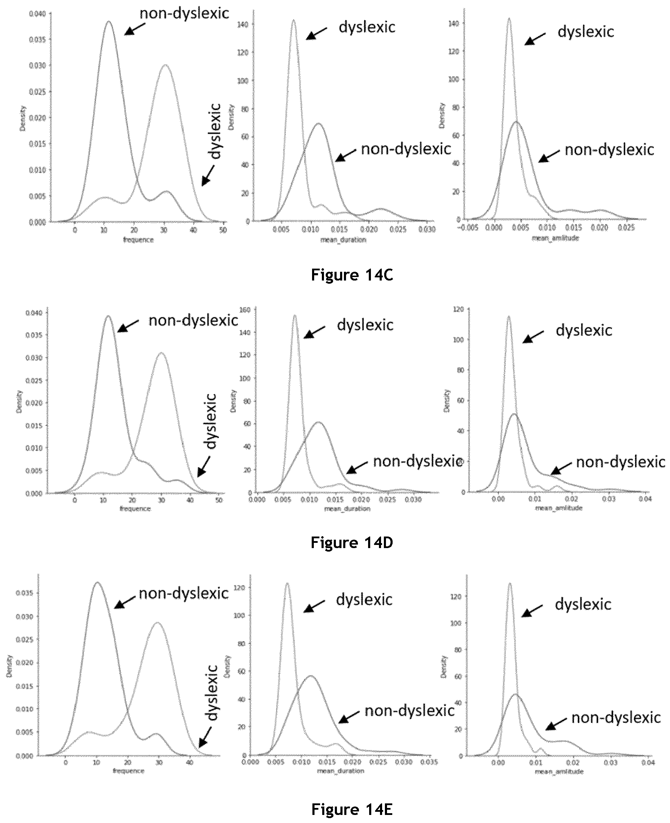

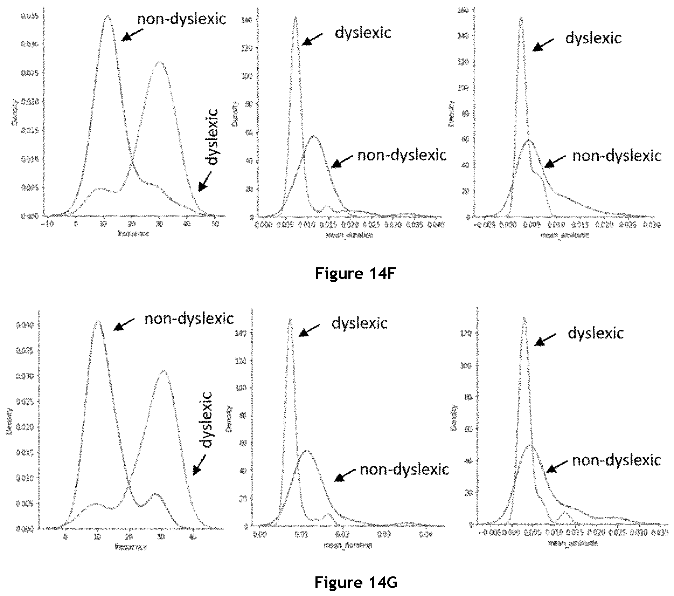

In addition, **Figures 14A to 14G** present the DGI (frequency), mean duration and mean amplitude of disconjugate segments during each painting test for the dyslexic and non-dyslexic populations.

**Table 4:** Mann-Whitney U-test for each of the three-parameter and the 7 paintings dataset

| Dataset | DGI | duration | amplitude |
|---|---|---|---|
| Painting 1 | -3.962 *** | -6.123 *** | -4.034 *** |
| Painting 2 | -4.034 *** | -5.892 *** | -2.811 *** |
| Painting 3 | -2.811 *** | -6.139 *** | -3.907 *** |
| Painting 4 | -3.907 *** | -5.622 *** | -4.352 *** |
| Painting 5 | -4.352 *** | -6.035 *** | -4.733 *** |
| Painting 6 | -4.733 *** | -5.734 *** | -4.280 *** |
| Painting 7 | -4.280 *** | -6.067 *** | -3.962 *** |

The Mann-Whitney U-test results shown in **Table 4** indicate statistically significant differences between dyslexic and non-dyslexic populations for all paintings.

*Stability of DGI values over paintings*

**Table 5** present the mean values of the disconjugacy velocity index for the dyslexic and the non-dyslexic population for each of the 7 painting tests. The mean of the DGI over the dyslexic and non-dyslexic population is quite stable over the 7 paintings dataset. Hence one can try to predict dyslexia without considering the 7 paintings separately.

**Table 5.** DGI mean values for the dyslexic and non-dyslexic populations for each of the 7 paintings datasets.

| | Mean DGI over non-dyslexic population | Mean DGI over dyslexic population |
|---|---|---|
| **Painting 1** | 0.34 | 0.60 |
| **Painting 2** | 0.33 | 0.58 |
| **Painting 3** | 0.33 | 0.59 |
| **Painting 4** | 0.34 | 0.58 |
| **Painting 5** | 0.30 | 0.56 |
| **Painting 6** | 0.33 | 0.58 |
| **Painting 7** | 0.31 | 0.58 |

For the 7 seven painting datasets, the mean of the computed DGI for the non-dyslexic population is centered at 0.325 with a standard deviation of 0.013. Also for the dyslexic population, the mean of the computed DGI is centered at 0.581 with a standard deviation of 0.012. meaning that the variability of the DGI means over this particular 7 painting is very low.

In addition, the variability of the DGI score over the 7 paintings dataset for each patient was analyzed. For the two populations, the variability of the DGI over this particular 7 painting is quite low (data not shown).

Hence, for the two populations, the variability of the DGI over this particular 7 painting is quite low, meaning that one can predict dyslexia using this score without taking into consideration the identifier of the painting table that was observed. In other words, we expect that the predictability won't depend on a particular painting.

*ML results: Predicting dyslexia using DGI*

**Tables 6A and 6B** present accuracy, sensitivity and specificity of classification using DGI (frequency), duration, amplitude, or DGI+Duration+Amplitude for each painting separately.

**Table 6A.** Support Vector Machine scores for the 7 DGI based data sets.

| Dataset | Features | accuracy | sensitivity | specificity |
|---|---|---|---|---|
| painting 1 | DGI | 88.75% | 90.0% | 87.5% |
| | Duration | 86.25% | 85.0% | 87.5% |
| | Amplitude | 70.0% | 70.0% | 70.0% |
| | DGI+Duration+Amplitude | 87.5% | 87.5% | 87.5% |
| painting 2 | DGI | 85.0% | 87.5% | 82.5% |
| | Duration | 82.5% | 80.0% | 85.0% |
| | Amplitude | 48.75% | 42.5% | 55.0% |
| | DGI+Duration+Amplitude | 86.25% | 90.0% | 82.5% |
| painting 3 | DGI | 87.5% | 90.0% | 85.0% |
| | Duration | 85.0% | 82.5% | 87.5% |
| | Amplitude | 61.25% | 75.0% | 47.5% |
| | DGI+Duration+Amplitude | 87.5% | 90.0% | 85.0% |
| painting 4 | DGI | 85.0% | 87.5% | 82.5% |
| | Duration | 83.75% | 82.5% | 85.0% |
| | Amplitude | 55.0% | 32.5% | 77.5% |
| | DGI+Duration+Amplitude | 85.0% | 85.0% | 85.0% |
| painting 5 | DGI | 86.25% | 92.5% | 80.0% |
| | Duration | 80.0% | 77.5% | 82.5% |
| | Amplitude | 63.75% | 55.0% | 72.5% |
| | DGI+Duration+Amplitude | 86.25% | 92.5% | 80.0% |
| painting 6 | DGI | 86.25% | 90.0% | 82.5% |
| | Duration | 85.0% | 82.5% | 84.34% |
| | Amplitude | 68.75% | 60.0% | 77.5% |
| | DGI+Duration+Amplitude | 86.25% | 87.5% | 85.0% |
| painting 7 | DGI | 88.75% | 90.0% | 87.5% |
| | Duration | 83.75% | 80.0% | 87.5% |
| | Amplitude | 52.5% | 40.0% | 65.0% |
| | DGI+Duration+Amplitude | 88.75% | 90.0% | 87.5% |

**Table 6B.** Logistic regression scores for the 7 DGI based datasets.

| Dataset | Feature | accuracy | sensitivit y | specificit y |
|---|---|---|---|---|
| Painting 1 | DGI | 88.75% | 90.0% | 87.5% |
| | Duration | 86.25% | 85.0% | 87.5% |
| | Amplitude | 71.25% | 65.0% | 77.5% |
| | DGI+Duration+Amplitude | 86.25% | 87.5% | 85.0% |
| Painting 2 | DGI | 85.0% | 87.5% | 82.5% |
| | Duration | 82.5% | 80.0% | 85.0% |
| | Amplitude | 63.75% | 45.0% | 82.5% |
| | DGI+Duration+Amplitude | 83.75% | 85.0% | 82.5% |
| Painting 3 | DGI | 87.5% | 90.0% | 85.0% |
| | Duration | 85.0% | 82.5% | 87.5% |

(continued)

| Dataset | Feature | accuracy | sensitivit y | specificit y |
|---------|---------|----------|--------------|--------------|
| | Amplitude | 66.25% | 60.0% | 72.5% |
| | DGI+Duration+Amplitude | 86.25% | 87.5% | 85.0% |
| Painting 4 | DGI | 85.0% | 87.5% | 82.5% |
| | Duration | 83.75% | 82.5% | 85.0% |
| | Amplitude | 62.5% | 50.0% | 75.0% |
| | DGI+Duration+Amplitude | 85.0% | 85.0% | 85.0% |
| Painting 5 | DGI | 86.25% | 92.5% | 80.0% |
| | Duration | 80.0% | 77.5% | 82.5% |
| | Amplitude | 63.75% | 55.0% | 72.5% |
| | DGI+Duration+Amplitude | 83.75% | 87.5% | 80.0% |
| Painting 6 | DGI | 87.5% | 90.0% | 85.0% |
| | Duration | 85.0% | 82.5% | 87.5% |
| | Amplitude | 71.25% | 60.0% | 82.5% |
| | Combinaison des 3 | 85.0% | 87.5% | 82.5% |
| Painting 7 | DGI | 88.75% | 90.0% | 87.5% |
| | Duration | 83.75% | 80.0% | 87.5% |
| | Amplitude | 66.25% | 50.0% | 82.5% |
| | DGI+Duration+Amplitude | 85.0% | 82.5% | 87.5% |

For each of the 7 painting datasets, and each of the logistic regression and RBF-SVM model, the feature importance of the duration was similar to the DGI, whereas the feature importance of the amplitude was very low compared to DGI and the duration. In addition, the accuracy score didn't improve when considering the duration and the amplitude, in addition to the DGI to predict dyslexia. This is due to the high correlation of The DGI with the amplitude and the duration. We've considered only the DGI feature for further analysis (analysis 2 and 3).

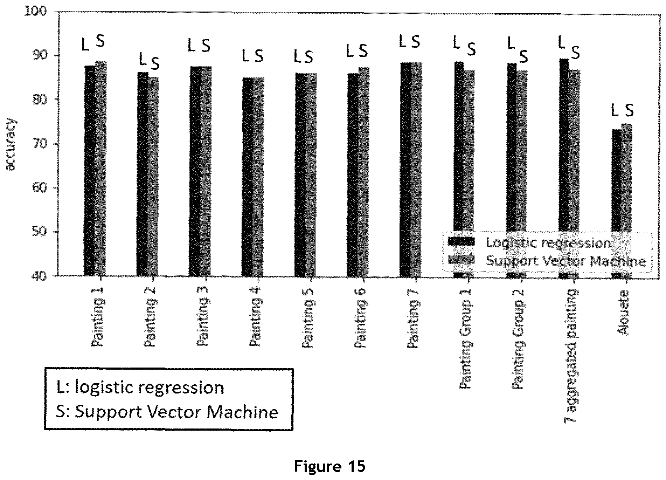

**Figure 15** presents the accuracy of the two models (Support vector machine and logistic regression). For the three analyses (for each of the 10 painting datasets), the score on the test set was overall the same. In other words, the generalization ability of each of the linear and nonlinear models has slightly improved when considering augmenting the sample size of the used dataset.

In addition, **Tables 6C to 6E** present accuracy, sensitivity and specificity of classification using DGI (frequency), duration, amplitude, or DGI+Duration+Amplitude for analysis 2 and analysis 3.

**Table 6C.** Support Vector Machine scores for the 2 grouped painting datasets.

| Dataset | accuracy | sensitivity | specificity |
|---------|----------|-------------|-------------|
| Painting Group 1 | 88.99% | 89.75% | 87.69% |
| Painting Group 2 | 87.06% | 87.43% | 87.12% |

**Table 6D.** Logistic regression scores for the 2 grouped painting datasets.

| Dataset | accuracy | sensitivity | specificity |
|---------|----------|-------------|-------------|
| Painting Group 1 | 88.53% | 88.82% | 87.69% |
| Painting Group 2 | 87.06% | 87.43% | 87.12% |

**Table 6E.** SVM and Logistic regression scores when trained on the 7 paintings dataset using DGI features.

| Model | accuracy | sensitivity | specificity |
|-------|----------|-------------|-------------|
| Support Vector Machine | 89.71% | 92.34% | 86.99% |
| Logistic regression | 87.32% | 87.00% | 87.49% |

The best accuracy was achieved when training in analysis 3, on the combined 7 painting dataset, with an accuracy of 89.71%, a sensitivity of 92.34%, and specificity of 86.99%.

Further investigation and analysis

*Sample size importance for the AIDEAL:*

To compare the generalization ability of each model when trained on Aideal deterministic based eye movement features, during each of the three analyses, for each model and each analysis, we aggregated the accuracy of the test set of each dataset by weighted means. The different scores are presented in **Table 7:**

Table 7. Logistic regression and Support Vector Machine global accuracy scores for the 3 analyses.

| Model | analysis 1 | analysis 2 | analysis 3 |
|---|---|---|---|
| Logistic regression | 75.53 % | 85.88 % | 88.24 % |
| Support Vector Machine | 72.85 % | 83.53 % | 89.41 % |

One can expect that the accuracy of each model would decrease when predicting dyslexia from the Aideal eye movement feature without taking into consideration the image at which each subject was looking since the eye movement feature depends on the trajectory of the eye, which depends on the observed image. Hence, ignoring the observed will increase the complexity of the classification problem.

However, the generalization ability of both, the linear and nonlinear model, has increased when considering merging the data of the different painting datasets, from 75.53% and 72.85% in analysis 1 to 85.88 % and 83.53 % in analysis 2, and finally to 88.24% and 89.41% in the last analysis, respectively. This can be explained by the sample size since the size of the dataset used in analysis 3 was 7 times bigger than the one used in analysis 1.

If such dependence is not shown for DGI it is due to the very low feature space dimension.

*Investigating the complementarity of Aideal and DGI based features*

To investigate the complementarity of the two feature sets in predicting dyslexia on the dataset of analysis 3, we retrained the two models to predict dyslexia using the DGI feature in addition to the Aideal eye movement features. In **Table 8,** we present the scores of the linear and nonlinear models when combining Aideal and DGI. When combining Aideal eye movement features and DGI features, we achieve an accuracy of 90.0% and 87.5%, a sensitivity of 90.0%, and a specificity of 90.0% and 85.5%.

Table 8. Support Vector Machine and Logistic regression scores when trained on the 7 paintings dataset using Aideal eye movement and DGI features together.

| Model | accuracy | sensitivity | specificity |
|---|---|---|---|
| Support Vector Machine | 90.0% | 90.0% | 90.0% |
| Logistic regression | 87.5% | 90.0% | 85.5% |

Summary of the results

When considering augmenting the dataset size by merging the 7-painting dataset (the third analysis), we've found that both the deterministic approach with the Aideal eye movement features, and DGI base approach, can successfully predict dyslexia using both the linear and nonlinear models. Also, their predictability has increased when considering merging multiple datasets and ignoring the painting index feature.

In addition, when combining the Aideal eye movement feature and DGI feature, we achieve a better accuracy using the RBF-SVM.

**Complementarity and need of both types of analysis (DGI and AIDEAL)**

The DGI feature applies to the whole time series of the eye movement data but it is more fragmented as it considers only very few portions of the signals, that is the moments when the eyes are traveling in opposite directions. It is also important to note that these instances mostly occur during resting periods between saccades and therefore do not concern active eye movement programming and execution phase.

Therefore, we can conclude on the complementarity and need of both types of analysis for more successful classification and physiologically plausible. The DGI feature associated with the specific paintings used here can be an approximate rough tool to evaluate both qualities of binocular conjugate control of the eyes in free painting exploration perhaps related to the sensitivity of the observers to movement illusion and /or complexity of space composition of the images

The Aideal based methods provided more neurologically grounded categorization on properties of eye movement control binocular eye movement stability It remains to be seen whether the DGI feature is powerful in any eye movement condition including oculomotor tests such as saccade and vergence tests, or including free exploration of narrative paintings or banaler everyday images.

To investigate how the DGI feature is powerful in the saccade and vergence tests, we've applied the same method described in analysis 1, but on the saccade and vergence dataset present in the previous analysis [see Example 2]. We've found that DGI can predict dyslexia from the saccade dataset with an accuracy of 75%, and from the vergence dataset with an accuracy of 80%.

Hence, the very high predictability of the DGI is lost, which confirms that the power of the paintings chosen, is responsible for the relevance of the DGI; we predict that paintings without depth illusion or complexity would provide less predictive power of the DGI. A current study with classic paintings e.g. Le tricheur by Georges de La Tour seems to confirm such a prediction.

**Table 9.** Support vector Machine scores when using the DGI feature on the saccade and vergence dataset

| dataset | accuracy | sensitivity | specificity |
|---|---|---|---|
| vergence | 75.0% | 83.33% | 63.33% |
| saccade | 80.0% | 86.67% | 73.33% |

**Table 10.** Logistic regression scores when using DGI feature on the saccade and vergence dataset

| dataset | accuracy | sensitivity | specificity |
|---|---|---|---|
| vergence | 75.0% | 80.0% | 66.67% |
| saccade | 80.0% | 86.67% | 73.33% |

In contrast to literature that focuses on the detection of binocular or conjugate micromovements, we focus here on micro-movements in the opposite direction for the two eyes. Also, in the microsaccade literature data cited above are mostly coming from a fixation task during which the subject has to fixate a target for several seconds typically 15 seconds.

Moreover, we do not limit to a particular waveform such as saccade type, the movement can be rapid or drifting. Also, the analysis is done on traces of the eyes continuously during a free exploration task self-triggered. In a way this analysis is less deterministic. Yet, the only hypothesis would be that the visual features of the paintings (perspective, overlapping, motion, contrast etc.) would somehow stimulate continuously disconjugate or micro-vergence eye movements; this is in line with prior studies see.

### Conclusions

We've developed a new algorithm to compute a new descriptor that we've called the disconjugacy global index (DGI), then we've investigated the complementarity of such features with Aideal based features to predict dyslexia. We evaluated the generalization ability when the models were trained on three different conditions, where we had a different sample size and different variance for each feature.

In the first analysis, each model was trained on the 7 paintings dataset separately and on the Alouette reading test. Also, in the second analysis, each model was trained on two groups of painting. Finally, in the last analysis, each model has been trained on the entire 7 paintings datasets.

For each for the three analyses, we've used Aideal based features and the DGI feature computed from the eye movement to predict dyslexia, in the third analysis we've also considered the complementarity between the two sets of descriptors, and we've found that we achieved the best score in term of accuracy, sensitivity, and specificity. We found that when combining the 7 paintings dataset, we can predict dyslexia with an accuracy of 89.71%, a sensitivity of 92.34%, and a specificity of 86.99% when using the DGI feature. Similarly, we found that we can predict dyslexia with an accuracy of 89.41%, a sensitivity of 88.89%, and a better specificity of 90% when using the Aideal based parameters.

Finally, we found that by using the Aideal eye movement features with DGI, we can predict dyslexia with an accuracy of 91.21%, a sensitivity of 90.0%, and a specificity of 92%.

**Claims**

1. A method for measuring binocular motricity parameters in an individual, comprising:

    a1) inviting the individual to free exploration of one or several pictorial artwork(s);
    b1) recording the eye movements of the left eye and the right eye of the individual during the free exploration of the one or more pictorial artwork(s); and
    c1) calculating, using a digital processing device, the value of one or more binocular motricity parameter(s) from the records obtained in step b1);

    wherein when several pictorial artworks are explored, they are explored one by one, and
    wherein each of the one or more pictorial artwork(s) is selected from pictorial artworks creating an illusion of visual motion in depth and pictorial artworks with spatial or sense ambiguity.

2. The method according to claim 1, wherein the one or more pictorial artwork(s) are two-dimensional pictorial artwork(s) positioned in a vertical plane.

3. The method according to claim 1 or claim 2, wherein:

    a) pictorial artworks creating an illusion of visual motion in depth are selected from Bridget Riley Op-art paintings, preferably from *Blaze 1* (1962), *Movements in square* (1961), and *Cataract 3* (1967);
    b) pictorial artworks with spatial or sense ambiguity are selected from Escher and Magritte pictorial artworks, preferably from:

        • Escher, *Relativité,* 1953,
        • René Magritte, *La trahison des images,* 1973,
        • René Magritte, *La condition humaine I*, 1935, and
        • René Magritte, *The Art of Conversation-Color Variation,* 1950.

4. The method according to anyone of claims 1 to 3, wherein the individual is submitted to free exploration of several pictorial artwork(s) in step a1), preferably the individual is submitted to free exploration of:

    i) several pictorial artworks creating an illusion of visual motion in depth,
    ii) several pictorial artworks with spatial or sense ambiguity, or
    iii) one or more pictorial artwork(s) creating an illusion of visual motion in depth and one or more pictorial artwork(s) with spatial or sense ambiguity.

5. The method according to anyone of claims 1 to 4, wherein one or more binocular motricity parameter(s) calculated in step c1) is(are) selected from binocular motricity parameters of horizontal and/or vertical components of saccades, preferably from binocular motricity parameters of horizontal components of saccades.

6. The method according to anyone of claims 1 to 5, wherein one or more binocular motricity parameter(s) calculated in step c1) is(are) selected from:

    • velocity parameters of horizontal and/or vertical components of saccades, in particular peak velocity or average velocity of horizontal and/or vertical components of saccades,
    • amplitude parameters of horizontal and/or vertical components of saccades,
    • duration parameters of horizontal and/or vertical components of saccades, and
    • horizontal and/or vertical disconjugacy during saccades,

preferably one or more binocular motricity parameter(s) calculated in step c1) is(are) selected from:

    • velocity parameters of the horizontal component of saccades, in particular peak velocity or average velocity of the horizontal component of saccades, and
    • horizontal disconjugacy during saccades.

7. The method according to anyone of claims 1 to 6, wherein one or more binocular motricity parameter(s) calculated in step c1) is(are) selected from binocular motricity parameters of disconjugacy segments during the whole exploration of each pictorial artwork, preferably from frequency, mean duration and mean amplitude of disconjugacy segments during the whole exploration of each pictorial artwork, more preferably one or more binocular motricity parameter(s) calculated in step c1) is(are) selected from frequency and mean duration of disconjugacy segments during whole while exploration of each pictorial artwork.

8. The method according to anyone of claims 1 to 7, wherein one binocular motricity parameter(s) calculated in step c1) is the frequency of disconjugacy segments during the whole exploration of each pictorial artwork (DGI index).

9. The method according to anyone of claims 1 to 8, wherein binocular motricity parameter(s) calculated in step c1) comprise:

   a) binocular motricity parameters of horizontal and/or vertical components of saccades, and
   b) the frequency of disconjugacy segments during the whole exploration of each pictorial artwork (DGI index).

10. The method according to anyone of claims 1 to 9, further comprising:

   d1) submitting the individual to one or more ocular motor test(s) selected from a reading test, a vergence test and a saccade test;
   e1) recording the eye movements of the left eye and the right eye of the individual during each of the ocular motor tests; and
   f1) calculating, using a digital processing device, the value of at least one binocular motricity parameter from the records obtained in step e1);

      wherein the vergence test and the saccade test are conducted using a binocular motor stimulation device configured to specifically and physically stimulate vergences and saccades, comprising visual or audiovisual targets placed in real space at eccentricities and depths of a surface;
      wherein the vergence test comprises 30 to 50 trials of:

         • visually and/or audiovisually stimulating the individual at a point located at the eyes level, in the central axis between the left eye and the right eye, at a first distance, and
         • visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and

      wherein the saccade test comprises 30 to 50 trials of:

         • visually and/or audiovisually stimulating the individual at a point located at the eyes level, in the central axis between the left eye and the right eye, and
         • visually and/or audiovisually stimulating the individual at another point located at the eyes level, on the left or on the right of the previous point, half of the trials being on the left, the other half on the right, the trials on the left and on the right being interleaved.

11. The method according to anyone of claims 1 to 10, wherein the individual:

   i) is a healthy individual,
   ii) suffers or is suspected to suffer from a learning disorder, in particular dyslexia or
   iii) suffers or is suspected to suffer from an autism spectrum disorder (ASD), in particular autism.

12. A method for detecting features of a learning disorder (in particular dyslexia) or an autistic spectrum disorder (in particular autism) in an individual, comprising:

   a2) providing previously obtained recordings of the eye movements of the left eye and the right eye of the individual during free exploration of one or several pictorial artwork(s);
   b2) calculating, using a digital processing device, the value(s) of at least one binocular motricity parameter from the records provided in step a2);

c2) comparing the value(s) calculated in step b2) to one or more reference value(s) obtained in one or more reference individual(s); and

d2) concluding to the presence or absence of features of a learning disorder (in particular dyslexia) or an autistic spectrum disorder (in particular autism) in the individual based on said comparison.

13. The according to claim 12, which further comprises:

e2) providing previously obtained recordings of the eye movements of the left eye and the right eye of the individual during one or more other ocular motor test(s), preferably selected from a reading test, a vergence test and a saccade test;

f2) calculating, using a digital processing device, the value(s) of at least one binocular motricity parameter from the records provided in step e2); and

g2) comparing the value(s) calculated in step f2) to one or more reference value(s) obtained in one or more reference individual(s);

wherein steps e2) to g2) are performed before step d2), and step d2) consists in concluding to the presence or absence of features of a learning disorder (in particular dyslexia) or an autistic spectrum disorder (in particular autism) in the individual based on the comparisons of steps c2) and g2).

14. A method for assessing the level of creativity of an individual, comprising:

a3) providing previously obtained recordings of the eye movements of the left eye and the right eye of the individual during free exploration of one or several pictorial artwork(s);

b3) calculating, using a digital processing device, the value(s) of at least one binocular motricity parameter from the records provided in step a3);

c3) comparing the value(s) calculated in step b3) to one or more reference value(s) obtained in one or more reference individual(s); and

d3) concluding to the level of creativity of the individual based on said comparison.

15. The method according to anyone of claims 12 to 14, wherein the recordings provided in step a2) or a3) have been obtained as in step a1) of the method according to any one of claims 1 to 9.

Figure 1A

Figure 1B

Figure 1C

Figure 1D

Figure 1E

Figure 1F

Figure 1G

Figure 2

## Posture Parameters while viewing Painting 1

**Figure 3A**

## Posture Parameters while viewing Painting 2

**Figure 3B**

## Posture Parameters while viewing Painting 3

**Figure 3C**

## Subjective Response to Paintings

**Figure 4**

Figure 5A

Figure 5B

Figure 6A

Figure 6B

Figure 6C

Figure 7

## Number of Spaces Perceived in Painting #2

■ Dyslexic  □ Non-Dyslexic

**Figure 8**

## Number of Words Perceived in Painting #3

Number of words

■ Dyslexic  □ Non-Dyslexic

**Figure 9**

Figure 10

Figure 11

Figure 12

Figure 13A

Figure 13B

Figure 13C

Figure 13D

Figure 13E

Figure 13F

Figure 13G

Figure 14A

Figure 14B

**Figure 14C**

**Figure 14D**

**Figure 14E**

**Figure 14F**

**Figure 14G**

Figure 15

**Figure 16**

Figure 17

Europäisches
Patentamt

European
Patent Office

Office européen
des brevets

# EUROPEAN SEARCH REPORT

**Application Number**

EP 22 30 5903

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KAPOULA ZOÃ ET AL: "Eye movement instructions modulate motion illusion and body sway with Op Art", FRONTIERS IN HUMAN NEUROSCIENCE, vol. 9, 26 March 2015 (2015-03-26), XP093003385, CH ISSN: 1662-5161, DOI: 10.3389/fnhum.2015.00121 | 1-11 | INV. A61B5/113 A61B5/16 |
| A | * sections "Stimuli and apparatus" and "Procedure"; figures 4A-4D * | 12-15 | |
| X | KAPOULA ZOÏ ET AL: "Free exploration of painting uncovers particularly loose yoking of saccades in dyslexics", DYSLEXIA, vol. 15, no. 3, 1 August 2009 (2009-08-01), pages 243-259, XP093003360, GB ISSN: 1076-9242, DOI: 10.1002/dys.377 | 12-15 | |
| A | * sections "Free exploration of artworks", "Calculation of measures", "Statistical analyses", "Discussion" * | 1-11 | TECHNICAL FIELDS SEARCHED (IPC) A61B |

-/--

The present search report has been drawn up for all claims

| Place of search | Date of completion of the search | Examiner |
|---|---|---|
| The Hague | 30 November 2022 | Knüpling, Moritz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 30 5903

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Stephanie Jainta ET AL: "Dyslexic Children Are Confronted with Unstable Binocular Fixation while Reading", PLoS ONE, 6 April 2011 (2011-04-06), pages 1-9, XP055022200, DOI: 10.1371/journal.pone.0018694 Retrieved from the Internet: URL:www.plosone.org/article/fetchObjectAtt achment.action?uri=info%3Adoi%2F10.1371%2F journal.pone.0018694&representation=PDF [retrieved on 2012-03-19] | 12-15 | |
| A | * section "Fixation disparity and its standard deviation during free exploration of a painting" * ----- | 1-11 | |
| A | JOTHI PRABHA A ET AL: "Predictive Model for Dyslexia from Fixations and Saccadic Eye Movement Events", COMPUTER METHODS AND PROGRAMS IN BIOMEDICINE., vol. 195, 1 October 2020 (2020-10-01), page 105538, XP093003674, NL ISSN: 0169-2607, DOI: 10.1016/j.cmpb.2020.105538 Retrieved from the Internet: URL:https://www.sciencedirect.com/science/ article/pii/S0169260720300377/pdfft?md5=13 f86fb64b3b09c5fde18c43723b1e09&pid=1-s2.0- S0169260720300377-main.pdf> * abstract * ----- | 12-15 | TECHNICAL FIELDS SEARCHED (IPC) |
| | -/-- | | |

The present search report has been drawn up for all claims

| Place of search | Date of completion of the search | Examiner |
|---|---|---|
| The Hague | 30 November 2022 | Knüpling, Moritz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

# EUROPEAN SEARCH REPORT

Application Number

EP 22 30 5903

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CANCER ALICE ET AL: "The alleged link between creativity and dyslexia: Identifying the specific process in which dyslexic students excel", COGENT PSYCHOLOGY, vol. 3, no. 1, 8 June 2016 (2016-06-08), page 1190309, XP093003688, DOI: 10.1080/23311908.2016.1190309 Retrieved from the Internet: URL:https://www.cogentoa.com/article/10.10 80/23311908.2016.1190309.pdf> * abstract * ----- | 14,15 | |

**TECHNICAL FIELDS SEARCHED (IPC)**

The present search report has been drawn up for all claims

| Place of search | Date of completion of the search | Examiner |
|---|---|---|
| The Hague | 30 November 2022 | Knüpling, Moritz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022074130 A1
- WO 2021228724 A
- WO 2022074130 A
- US 8851669 B
- WO 2011073288 A1
- EP 22305061

**Non-patent literature cited in the description**

- **TORRANCE, E. P. ; VERBAL TESTS, FORMS A AND B. FIGURAL TESTS, FORMS A AND B.** Torrance tests of creative thinking. Norms-technical manual. Research edition. Personnel Press, 1966
- **KAPOULA, Z. ; RUIZ, S. ; SPECTOR, L. ; MOCOROVI, M. ; GAERTNER, C. ; QUILICI, C. ; VERNET, M.** Education Influences Creativity in Dyslexic and Non-Dyslexic Children and Teenagers. *PLoS One,* 2016, vol. 11 (3), e0150421
- **EVERATT, J. ; STEFFERT, B. ; SMYTHE, I.** An eye for the unusual: Creative thinking in dyslexics. *Dyslexia,* 1999, vol. 5 (1), 28-46
- **RACK, L.** Developmental dyslexia and literary creativity: creativity in the area of deficit. *J Learn Disabil,* 1981, vol. 14 (5), 262-263
- **CANCER, A. ; MANZOLI, S. ; ANTONIETTI, A.** The alleged link between creativity and dyslexia: Identifying the specific process in which dyslexic students excel. *Cogent Psychology,* 2016, vol. 3 (1), 1190309
- **TAFTI, M. A. ; HAMEEDY, M. A. ; BAGHAL, N. M.** Dyslexia, a deficit or a difference: comparing the creativity and memory skills of dyslexic and nondyslexic students in Iran. *Social Behavior and Personality: An International Journal,* 2009, vol. 37, 1009
- **WOLFF, U. ; LUNDBERG, I.** The prevalence of dyslexia among art students. *Dyslexia,* 2002, vol. 8 (1), 34-42
- **WARD, L. M. ; KAPOULA, Z.** Differential diagnosis of vergence and saccade disorders in dyslexia. *Sci Rep,* 2020, vol. 10 (1), 22116
- **WARD, L. M. ; KAPOULA, Z.** Dyslexics' Fragile Oculomotor Control Is Further Destabilized by Increased Text Difficulty. *Brain Sci,* 2021, vol. 11 (8
- **EL HMIMDI AE ; WARD LM ; PALPANAS T ; KAPOULA Z.** *Predicting Dyslexia and Reading Speed in Adolescents from Eye Movements in Reading and Non-Reading Tasks: A Machine Learning Approach*
- **PENNISI P ; GIALLONGO L ; MILINTENDA G ; CANNAROZZO M.** Autism, autistic traits and creativity: a systematic review and meta-analysis. *Cogn Process,* February 2021, vol. 22 (1), 1-36
- *J Integr Neurosci.,* 30 December 2021, vol. 20 (4), 1095-1104
- **KASIRER A ; ADI-JAPHA E ; MASHAL N.** Verbal and Figural Creativity in Children With Autism Spectrum Disorder and Typical Development. *Front Psychol.,* 27 October 2020, vol. 11, 559238
- **COULTER RA ; BADE A ; JENEWEIN EC ; TEA YC ; MITCHELL GL.** Near-point Findings in Children with Autism Spectrum Disorder and in Typical Peers. *Optom Vis Sci.,* 01 April 2021, vol. 98 (4), 384-393
- **SVEND ERIK MOURIDSEN ; BENTE RICH ; TORBEN ISAGER.** Eye Disorders among Adult People Diagnosed with Infantile Autism in Childhood: A Longitudinal Case Control Study. *Ophthalmic Epidemiology,* 2017, vol. 24 (5), 332-335
- **JOLIE R. KEEMINK et al.** Eye Movements and Behavioural Responses to Gaze-Contingent Expressive Faces in Typically Developing Infants and Infant Siblings. *Autism Res,* 2021, vol. 14, 973-983
- **BRIDGET RILEY.** *Blaze,* 1962
- **BRIDGET RILEY.** *Movements in square,* 1961
- **BRIDGET RILEY.** *Cataract,* 1967
- **M. C. ESCHER.** *Relativité,* 1953
- **RENÉ MAGRITTE.** *La trahison des images,* 1973

- **RENÉ MAGRITTE.** *La condition humaine I,* 1935
- **RENÉ MAGRITTE.** *The Art of Conversation-Color Variation,* 1950
- **BRIDGET RILEY.** *Movement in Squares,* 1961
- **ESCHER.** *Relativité,* 1953
- **BRIDGET RILEY.** *Blaze 1,* 1962
- **BRIDGET RILEY.** *Cataract 3,* 1967
- *Blaze 1,* 1962
- *Movements in square,* 1961
- *Cataract 3,* 1967
- *La condition humaine I,* 1935
- *The Art of Conversation-Color Variation,* 1950
- **KAPOULA Z et al.** HSOA Journal of Clinical Studies and Medical Case Reports. *J Clin Stud Med Case Rep,* 2019, vol. 6, 74, https://www.medi-cus.ua/eng/product/ophthalmic-equipment/plusoptiX-R09-PowerRef-3/manufacturer
- **ANNA GAVALDA ; BAYARD JEUNESSE.** 35 Kilos D'Espoir
- **SOTIROPOULOS MG ; ANAGNOSTOULI M.** Genes, brain dynamics and art: the genetic underpinnings of creativity in dancing, musicality and visual arts. *J Integr Neurosci.,* 30 December 2021, vol. 20 (4), 1095-1104
- **PENNISI P ; GIALLONGO L ; MILINTENDA G ; CANNAROZZO M.** Autism, autistic traits and creativity: a systematic review and meta-analysis. *Cogn Process.,* February 2021, vol. 22 (1), 1-36
- Postural and Emotional Impact of Carsten Höller's Artwork "Light Corner. **VERNET M. ; M. A., KAPOU-LA Z.** Exploring Transdisciplinarity in Art and Scienc-es. Springer, 2018, 165-175
- **GATEV, P. ; THOMAS, S. ; KEPPLE, T. ; HALLETT, M.** Feedforward ankle strategy of balance during qui-et stance in adults. *J Physiol,* 1999, vol. 514, 915-928
- **WINTER, D. A. ; PRINCE, F. ; FRANK, J. S. ; POW-ELL, C. ; ZABJEK, K. F.** Unified theory regarding A/P and M/L balance in quiet stance. *J Neurophysiol,* 1996, vol. 75 (6), 2334-2343
- **WINTER, D. A. ; PATLA, A. E. ; ISHAC, M. ; GAGE, W. H.** Motor mechanisms of balance during quiet standing. *J Electromyogr Kinesiol,* 2003, vol. 13 (1), 49-56
- **DAY, B. L. ; STEIGER, M. J. ; THOMPSON, P. D. ; MARSDEN, C. D.** Effect of vision and stance width on human body motion when standing: implications for afferent control of lateral sway. *J Physiol,* 1993, vol. 469, 479-499
- **WARD, L. M. ; KAPOULA, Z.** Dyslexics' Fragile Oc-ulomotor Control Is Further Destabilized by Increased Text Difficulty. *Brain Sci,* 2021, vol. 11, 8
- **RENÉ MAGRITTE.** *La trahison des images,* 1929
- **RENÉ MAGRITTE.** *La condition humaine,* 1933
- **RENÉ MAGRITTE.** *L'art de la conversation,* 1950
- *La Trahison des Images,* 1929
- *La Condition Humaine,* 1933
- *L'Art de la Conversation,* 1950
- **WARD, L. M ; KAPOULA, Z.** Differential diagnosis of vergence and saccade disorders in dyslexia. *Sci Rep,* 2020, vol. 10 (1), 22116